# EUROPEAN PATENT APPLICATION

(11) **EP 3 889 183 A1**
(43) Date of publication of application: **06.10.2021**
(21) Application number: 20315096.6
(22) Date of filing: 01.04.2020
(51) Int. Cl.: C07K 16/30, C07K 16/28, C07K 16/00, A61P 35/00, C07K 14/54, C07K 14/715

(54) **A PROTEIN COMPLEX COMPRISING AN IMMUNOCYTOKINE**

(71) Applicant: PIERRE FABRE MEDICAMENT, 92100 Boulogne-Billancourt (FR)
(72) Inventor: LOWE, Peter, 01300 Belley (FR); MALISSARD, Martine, 01200 Valserhône (FR); AKLA, Barbara, 74350 Cruseilles (FR); TREPREAU, Juliette, 74160 Saint Julien en Genevois (FR)
(74) Representative: Regimbeau

(57) **Abstract**

The present invention relates to new protein complexes which are useful for the treatment of cancer. These fusion proteins comprise (i) an immunocytokine comprising an antibody or antigen-binding fragment thereof fused to a cleavable peptide linker, and a cytokine, preferably IL-15, or a functional fragment thereof; and (ii) a cofactor which is preferably IL-15Rα or an IL-15-binding fragment thereof. Methods of treatment using these protein complexes are also disclosed.

SEQUENCE LISTING

<110>  PIERRE FABRE MEDICAMENT

<120>  A PROTEIN COMPLEX COMPRISING AN IMMUNOCYTOKINE

<130>  B75906EPD40404

<160>  94

<170>  PatentIn version 3.5

<210>  1
<211>  107
<212>  PRT
<213>  Homo sapiens

<400>  1

<210>  2
<211>  106
<212>  PRT
<213>  Homo sapiens

<400>  2

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105

<210> 3
<211> 321
<212> DNA
<213> Homo sapiens

<400> 3
cgtacggtgg ccgctcccag cgtgttcatc ttccccccaa gcgacgagca gctgaagagc 60

ggcaccgcca gcgtggtgtg tctgctgaac aacttctacc ccagggaggc caaggtgcag 120

tggaaggtgg acaacgccct gcagagcggc aacagccagg agagcgtcac cgagcaggac 180

agcaaggact ccacctacag cctgagcagc accctgaccc tgagcaaggc cgactacgag 240

aagcacaagg tgtacgcctg tgaggtgacc caccagggcc tgtccagccc cgtgaccaag 300

agcttcaaca ggggcgagtg c 321

<210> 4
<211> 321
<212> DNA
<213> Homo sapiens

<400> 4
ggtcaaccca aggccgctcc cagcgtgacc ctgttccccc cctcgagcga ggagctgcag 60

gccaacaagg ccaccctggt gtgtctgatc agcgacttct acccaggcgc cgtgaccgtg 120

gcctggaagg ccgacagcag ccccgtgaag gccggcgtgg agaccaccac ccccagcaag 180

cagagcaaca acaagtacgc cgccagcagc tacctgagcc tgaccccaga gcagtggaag 240

agccacagga gctacagctg ccaggtcacc cacgagggca gcaccgtgga aaagaccgtg 300

gccccaaccg agtgctcctg a 321

<210> 5
<211> 112
<212> PRT
<213> Artificial

<220>
<223> Ganitumab Light Chain Variable Region

<400> 5

<210> 6
<211> 112
<212> PRT
<213> Artificial

<220>
<223> m9G4 Light Chain Variable Region

<400> 6

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Thr Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro His Thr Phe Gly Gly Gly Thr Lys Leu Glu Ile Lys
            100                 105                 110

<210>  7
<211>  108
<212>  PRT
<213>  Artificial

<220>
<223>  hNHS76 Light Chain Variable Region

<400>  7

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Asn Ser Arg Asp Ser Ser Gly Asn His
                85                  90                  95

Val Val Phe Gly Gly Gly Thr Lys Leu Thr Val Leu
            100                 105

<210>  8
<211>  336
<212>  DNA
<213>  Artificial

<220>
<223>  Ganitumab Light Chain Variable Region

<400>  8
gatgttgtga tgactcagtc tccactctcc ctgcccgtca cccctggaga gccggcctcc      60

atctcctgca ggtctagtca gagcctcctg catagtaatg gatacaacta tttggattgg     120

tacctgcaga agccagggca gtctccacag ctcctgatct atttgggttc taatcgggcc     180

tccggggtcc ctgacaggtt cagtggcagt ggatcaggca cagattttac actgaaaatc     240

agcagggtgg aggctgagga tgttggggtt tattactgca tgcaaggtac acactggcct     300

ctgacgttcg gccaagggac caaggtggag atcaaa                               336

<210>  9
<211>  336
<212>  DNA
<213>  Artificial

<220>
<223>  m9G4 Light Chain Variable Region

<400>  9
cgtacggtga tgactcaaac ccctctgacc ctcagcgtga cgatcgggca gcccgctagt      60

attagctgta agagtagcca gtctctcctg gattctgatg gcaagactta tctgaactgg     120

ctgttgcagc gacctggtca gtcaccgaca cggctcatat atctggtatc caaactcgac     180

tccggcgtgc cagacagatt caccggatct ggctcaggca cagactttac actgaaaatc     240

tccagggtcg aggcagagga ccttggggtt tactactgct ggcagggaac ccactttcca     300

catacattcg gtggcggaac caaactggaa atcaag                               336

<210>  10
<211>  324

<212>  DNA
<213>  Artificial

<220>
<223>  hNHS76 Light Chain Variable Region

<400>  10
tcctctgagc tgactcagga ccctgctgtg tctgtggcct tgggacagac agtcaggatc      60

acatgccaag gagacagcct cagaagctat tatgcaagct ggtaccagca gaagccagga      120

caggcccctg tacttgtcat ctatggtaaa aacaaccggc cctcagggat tccagaccga      180

ttctctggct ccagctcagg aaacacagct tccttgacca tcactggggc tcaggcggaa      240

gatgaggctg actattactg taactcccgg gacagcagtg gtaaccatgt ggtattcggc      300

ggagggacca agctgaccgt ccta                                              324


<210>  11
<211>  219
<212>  PRT
<213>  Artificial

<220>
<223>  Ganitumab Complete Light Chain

<400>  11

Asp Val Val Met Thr Gln Ser Pro Leu Ser Leu Pro Val Thr Pro Gly
1               5                   10                  15


Glu Pro Ala Ser Ile Ser Cys Arg Ser Ser Gln Ser Leu Leu His Ser
            20                  25                  30


Asn Gly Tyr Asn Tyr Leu Asp Trp Tyr Leu Gln Lys Pro Gly Gln Ser
        35                  40                  45


Pro Gln Leu Leu Ile Tyr Leu Gly Ser Asn Arg Ala Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Met Gln Gly
                85                  90                  95


Thr His Trp Pro Leu Thr Phe Gly Gln Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  12
<211>  219
<212>  PRT
<213>  Artificial

<220>
<223>  m9G4 Complete Light Chain

<400>  12

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Gln Arg Pro Gly Gln Ser
        35                  40                  45

Pro Thr Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Trp Gln Gly
                85                  90                  95

Thr His Phe Pro His Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  13
<211>  214
<212>  PRT
<213>  Artificial

<220>
<223>  hNHS76 Complete Light Chain

<400>  13

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Asn Ser Arg Asp Ser Ser Gly Asn His
                85                  90                  95

Val Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  14
<211>  657
<212>  DNA
<213>  Artificial

<220>

<223>  Ganitumab Complete Light Chain

<400>  14
gatgttgtga tgactcagtc tccactctcc ctgcccgtca cccctggaga gccggcctcc      60

atctcctgca ggtctagtca gagcctcctg catagtaatg gatacaacta tttggattgg     120

tacctgcaga agccagggca gtctccacag ctcctgatct atttgggttc taatcgggcc     180

tccggggtcc ctgacaggtt cagtggcagt ggatcaggca cagattttac actgaaaatc     240

agcagggtgg aggctgagga tgttggggtt tattactgca tgcaaggtac acactggcct     300

ctgacgttcg gccaagggac caaggtggag atcaaacgta cggtggccgc tcccagcgtg     360

ttcatcttcc ccccaagcga cgagcagctg aagagcggca ccgccagcgt ggtgtgtctg     420

ctgaacaact tctaccccag ggaggccaag gtgcagtgga aggtggacaa cgccctgcag     480

agcggcaaca gccaggagag cgtcaccgag caggacagca aggactccac ctacagcctg     540

agcagcaccc tgaccctgag caaggccgac tacgagaagc acaaggtgta cgcctgtgag     600

gtgacccacc agggcctgtc cagccccgtg accaagagct tcaacagggg cgagtgc        657

<210>  15
<211>  657
<212>  DNA
<213>  Artificial

<220>
<223>  m9G4 Complete Light Chain

<400>  15
cgtacggtga tgactcaaac ccctctgacc ctcagcgtga cgatcgggca gcccgctagt      60

attagctgta agagtagcca gtctctcctg gattctgatg gcaagactta tctgaactgg     120

ctgttgcagc gacctggtca gtcaccgaca cggctcatat atctggtatc caaactcgac     180

tccggcgtgc cagacagatt caccggatct ggctcaggca cagactttac actgaaaatc     240

tccagggtcg aggcagagga ccttggggtt tactactgct ggcagggaac ccactttcca     300

catacattcg gtggcggaac caaactggaa atcaagcgta cggtggccgc tcccagcgtg     360

ttcatcttcc ccccaagcga cgagcagctg aagagcggca ccgccagcgt ggtgtgtctg     420

ctgaacaact tctaccccag ggaggccaag gtgcagtgga aggtggacaa cgccctgcag     480

agcggcaaca gccaggagag cgtcaccgag caggacagca aggactccac ctacagcctg     540

agcagcaccc tgaccctgag caaggccgac tacgagaagc acaaggtgta cgcctgtgag     600

gtgacccacc agggcctgtc cagccccgtg accaagagct tcaacagggg cgagtgc        657

<210>  16
<211>  642
<212>  DNA
<213>  Artificial

<220>
<223>  hNHS76 Complete Light Chain

<400>  16
tcctctgagc tgactcagga ccctgctgtg tctgtggcct tgggacagac agtcaggatc      60

acatgccaag gagacagcct cagaagctat tatgcaagct ggtaccagca gaagccagga     120

caggcccctg tacttgtcat ctatggtaaa aacaaccggc cctcagggat tccagaccga     180

ttctctggct ccagctcagg aaacacagct tccttgacca tcactggggc tcaggcggaa     240

gatgaggctg actattactg taactcccgg gacagcagtg gtaaccatgt ggtattcggc     300

ggagggacca agctgaccgt cctaggtcaa cccaaggccg ctcccagcgt gaccctgttc     360

ccccctcga gcgaggagct gcaggccaac aaggccaccc tggtgtgtct gatcagcgac     420

ttctacccag gcgccgtgac cgtggcctgg aaggccgaca gcagccccgt gaaggccggc     480

gtggagacca ccacccccag caagcagagc aacaacaagt acgccgccag cagctacctg     540

agcctgaccc cagagcagtg gaagagccac aggagctaca gctgccaggt cacccacgag     600

ggcagcaccg tggaaaagac cgtggcccca accgagtgct cc                        642


<210>  17
<211>  329
<212>  PRT
<213>  Homo sapiens

<400>  17

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly
                325

<210> 18
<211> 326
<212> PRT
<213> Homo sapiens

<400> 18

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly
                325

<210>  19
<211>  987
<212>  DNA
<213>  Homo sapiens

<400>  19
gcgtccacaa agggcccaag cgtgttcccg ctagccccca gcagcaagag caccagcggc        60

ggcacagccg ccctgggctg cctggtgaag gactacttcc ccgagcccgt gaccgtgtcc 120

tggaacagcg gagccctgac ctccggcgtg cacaccttcc ccgccgtgct gcagagcagc 180

ggcctgtaca gcctgagcag cgtggtgacc gtgcccagca gcagcctggg cacccagacc 240

tacatctgta acgtgaacca caagcccagc aacaccaagg tggacaagag agtggagccc 300

aagagctgtg acaagaccca cacctgtccc ccctgcccag cccccgagct gctgggcgga 360

cccagcgtgt tcctgttccc ccccaagccc aaggacaccc tgatgatcag cagaaccccc 420

gaggtgacct gtgtggtggt ggacgtgtcc cacgaggacc cagaggtgaa gttcaactgg 480

tacgtggacg gcgtggaggt gcacaacgcc aagaccaagc ccagagagga gcagtacaac 540

agcacctaca gggtggtgtc cgtgctgacc gtgctgcacc aggactggct gaacggcaag 600

gagtacaagt gtaaggtgtc caacaaggcc ctgccagccc caatcgaaaa gaccatcagc 660

aaggccaagg gccagccaag agagccccag gtgtacaccc tgccacccag cagggaggag 720

atgaccaaga accaggtgtc cctgacctgt ctggtgaagg gcttctaccc aagcgacatc 780

gccgtggagt gggagagcaa cggccagccc gagaacaact acaagaccac cccccccagtg 840

ctggacagcg acggcagctt cttcctgtac agcaagctga ccgtggacaa gagcagatgg 900

cagcagggca acgtgttcag ctgctccgtg atgcacgagg ccctgcacaa ccactacacc 960

cagaagagcc tgagcctgtc cccaggc 987

<210> 20
<211> 978
<212> DNA
<213> Homo sapiens

<400> 20
gcgtccacaa agggccccag cgtgttccct ctggccccct gcagcagaag caccagcgag 60

tctacagccg ccctgggatg cctggtcaag gactacttcc ccgagcccgt gaccgtgtcc 120

tggaactctg gcgccctgac cagcggcgtg cacacctttc cagccgtgct gcagagcagc 180

ggcctgtaca gcctgagcag cgtcgtgacc gtgcctagca gcagcctggg caccaagacc 240

tacacctgta acgtggacca caagcccagc aacaccaagg tggacaagcg ggtggaatct 300

aagtacggcc ctccctgccc cccctgccca gcccctgaat ttctgggcgg accctccgtg 360

ttcctgttcc ccccaaagcc caaggacacc ctgatgatca gccggacccc cgaagtgacc 420

tgcgtggtgg tggacgtgtc ccaggaagat cccgaggtcc agttcaactg gtacgtggac 480

ggcgtggaag tgcacaacgc caagaccaag cccagagagg aacagttcaa cagcacctac 540

cgggtggtgt ccgtgctgac cgtgctgcac caggactggc tgaacggcaa agagtacaag 600

tgcaaggtct ccaacaaggg cctgcccagc tccatcgaga aaaccatcag caaggccaag 660

ggccagcccc gcgagcccca ggtgtacaca ctgcccccca gccaggaaga gatgaccaag 720

aaccaggtgt ccctgacctg cctcgtgaag ggcttctacc ccagcgatat cgccgtggaa 780

tgggagagca acggccagcc cgagaacaac tacaagacca ccccccctgt gctggacagc 840

gacggcagct tcttcctgta ctcccggctg accgtcgaca agagccggtg gcaggaaggc 900

aacgtcttca gctgcagcgt gatgcacgag gccctgcaca accactacac ccagaagtcc 960

ctgagcctga gcctggga 978

<210> 21
<211> 119
<212> PRT
<213> Artificial

<220>
<223> Ganitumab Heavy Chain Variable Region

<400> 21

<210>  22
<211>  113
<212>  PRT
<213>  Artificial

<220>
<223>  m9G4 Heavy Chain Variable Region

<400>  22

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala

<210>  23
<211>  117
<212>  PRT
<213>  Artificial

<220>
<223>  hNHS76 Heavy Chain Variable Region

<400>  23

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser
        115

<210>  24
<211>  357
<212>  DNA
<213>  Artificial

<220>
<223>  Ganitumab Heavy Chain Variable Region

<400>  24
caggtgcagc tgcaggagtc gggaccagga ctggtgaagc cttcggggac cctgtccctc      60

acctgcgctg tctctggtgg ctccatcagc agtagtaact ggtggagttg ggtccgccag     120

cccccaggga aggggctgga gtggattggg gaaatctatc atagtgggag caccaactac     180

aacccgtccc tcaagagtcg agtcaccata tcagtagaca agtccaagaa ccagttctcc     240

ctgaagctga gctctgtgac cgccgcggac acggccgtgt attactgtgc gagatggacc     300

gggcgtactg atgcttttga tatctggggc caagggacaa tggtcaccgt ctcaagc        357

<210>  25
<211>  338

<212>  DNA
<213>  Artificial

<220>
<223>  m9G4 Heavy Chain Variable Region

<400>  25
aagtgatgtt ggtagaaagc ggtggtgggc tggtgaaacc tggcgggtcc ctgaaactct      60

cctgtactgc tagtggattc acattcagca cttacgacat gtcatgggtc cggcagaccc     120

cagagaagag actggagtgg gtcagcacta tctcctcttc tcggacctat acgtattaca     180

gtgccagcgt caagggacgc tttaccatta gcagggataa tgccagaaac atactgtacc     240

tgcagatgtc aagcctccga agtgaggaca ccgccatgta ttactgcgct aggcaatttt     300

cacactgggg ccaggggaca ttggttaccg tgtctgca                             338


<210>  26
<211>  351
<212>  DNA
<213>  Artificial

<220>
<223>  hNHS76 Heavy Chain Variable Region

<400>  26
caggtgcagc tgcaggagtc cggcccagga ctggtgaagc cttcggagac cctgtccctc      60

acctgcgctg tctctggtta ctccatcagc agtggttact actggggctg gattcggcag     120

cccccaggga aggggctgga gtggattggg agtatctatc atagtgggag cacctactac     180

aacccgtccc tcaagagtcg agtcaccata tcagtagaca cgtccaagaa ccagttctcc     240

ctgaagctga gctctgtgac cgccgcagac acggccgtgt attactgtgc aagagggaag     300

tggtcgaagt ttgactattg gggccaaggc accctggtca ccgtctcttc a              351


<210>  27
<211>  448
<212>  PRT
<213>  Artificial

<220>
<223>  Ganitumab_IgG1 Complete Heavy Chain

<400>  27

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

<210>  28
<211>  445
<212>  PRT
<213>  Artificial

<220>
<223>  Ganitumab_IgG4 Complete Heavy Chain

<400>  28

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly
        435                 440                 445

<210>  29
<211>  442
<212>  PRT
<213>  Artificial

<220>
<223>  m9G4_IgG1 Complete Heavy Chain

<400>  29

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Arg Val Glu Pro Lys Ser Cys Asp Lys Thr His Thr Cys Pro Pro
    210                 215                 220

Cys Pro Ala Pro Glu Leu Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly
        435                 440

<210>  30
<211>  439
<212>  PRT
<213>  Artificial

<220>
<223>  m9G4_IgG4 Complete Heavy Chain

<400>  30

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly
        435

<210>  31
<211>  446
<212>  PRT
<213>  Artificial

<220>
<223>  hNHS76_IgG1 Complete Heavy Chain

<400>  31

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly
        435                 440                 445

<210>  32
<211>  442
<212>  PRT
<213>  Artificial

<220>
<223>  hNHS76_IgG4 Complete Heavy Chain

<400>  32

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Arg Val Glu Pro Lys Ser Cys Asp Lys Thr His Thr Cys Pro Pro
    210                 215                 220

Cys Pro Ala Pro Glu Leu Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly
        435                 440

<210>  33
<211>  1344
<212>  DNA
<213>  Artificial

<220>
<223>  Ganitumab_IgG1 Complete Heavy Chain

<400>  33
caggtgcagc tgcaggagtc gggaccagga ctggtgaagc cttcggggac cctgtccctc        60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggc 1344

<210> 34
<211> 1335
<212> DNA
<213> Artificial

<220>
<223> Ganitumab_IgG4 Complete Heavy Chain

<400> 34
caggtgcagc tgcaggagtc gggaccagga ctggtgaagc cttcggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gccccagcgt gttccctctg gccccctgca gcagaagcac cagcgagtct 420

acagccgccc tgggatgcct ggtcaaggac tacttccccg agcccgtgac cgtgtcctgg 480

aactctggcg ccctgaccag cggcgtgcac acctttccag ccgtgctgca gagcagcggc 540

ctgtacagcc tgagcagcgt cgtgaccgtg cctagcagca gcctgggcac caagacctac 600

acctgtaacg tggaccacaa gcccagcaac accaaggtgg acaagcgggt ggaatctaag 660

tacggccctc cctgcccccc ctgcccagcc cctgaatttc tgggcggacc ctccgtgttc 720

ctgttccccc caaagcccaa ggacaccctg atgatcagcc ggacccccga agtgacctgc 780

gtggtggtgg acgtgtccca ggaagatccc gaggtccagt tcaactggta cgtggacggc 840

gtggaagtgc acaacgccaa gaccaagccc agagaggaac agttcaacag cacctaccgg 900

gtggtgtccg tgctgaccgt gctgcaccag gactggctga acggcaaaga gtacaagtgc 960

aaggtctcca acaagggcct gcccagctcc atcgagaaaa ccatcagcaa ggccaagggc 1020

cagccccgcg agccccaggt gtacacactg ccccccagcc aggaagagat gaccaagaac 1080

caggtgtccc tgacctgcct cgtgaagggc ttctacccca gcgatatcgc cgtggaatgg 1140

gagagcaacg gccagcccga gaacaactac aagaccaccc cccctgtgct ggacagcgac 1200

ggcagcttct tcctgtactc ccggctgacc gtcgacaaga gccggtggca ggaaggcaac 1260

gtcttcagct gcagcgtgat gcacgaggcc ctgcacaacc actacaccca gaagtccctg 1320

agcctgagcc tggga 1335

<210> 35
<211> 1326
<212> DNA
<213> Artificial

<220>
<223> m9G4_IgG1 Complete Heavy Chain

<400> 35
gaagtgatgt tggtagaaag cggtggtggg ctggtgaaac ctggcgggtc cctgaaactc 60

tcctgtactg ctagtggatt cacattcagc acttacgaca tgtcatgggt ccggcagacc 120

ccagagaaga gactggagtg ggtcagcact atctcctctt ctcggaccta tacgtattac 180

agtgccagcg tcaagggacg ctttaccatt agcagggata atgccagaaa catactgtac 240

ctgcagatgt caagcctccg aagtgaggac accgccatgt attactgcgc taggcaattt 300

tcacactggg gccaggggac attggttacc gtgtctgcag cgtccacaaa gggcccaagc 360

gtgttcccgc tagcccccag cagcaagagc accagcggcg gcacagccgc cctgggctgc 420

ctggtgaagg actacttccc cgagcccgtg accgtgtcct ggaacagcgg agccctgacc 480

tccggcgtgc acaccttccc cgccgtgctg cagagcagcg gcctgtacag cctgagcagc 540

gtggtgaccg tgcccagcag cagcctgggc acccagacct acatctgtaa cgtgaaccac 600

aagcccagca acaccaaggt ggacaagaga gtggagccca agagctgtga caagacccac 660

acctgtcccc cctgcccagc ccccgagctg ctgggcggac ccagcgtgtt cctgttcccc 720

cccaagccca aggacaccct gatgatcagc agaacccccg aggtgacctg tgtggtggtg 780

gacgtgtccc acgaggaccc agaggtgaag ttcaactggt acgtggacgg cgtggaggtg 840

cacaacgcca agaccaagcc cagagaggag cagtacaaca gcacctacag ggtggtgtcc 900

gtgctgaccg tgctgcacca ggactggctg aacggcaagg agtacaagtg taaggtgtcc 960

aacaaggccc tgccagcccc aatcgaaaag accatcagca aggccaaggg ccagccaaga 1020

gagccccagg tgtacaccct gccacccagc agggaggaga tgaccaagaa ccaggtgtcc 1080

ctgacctgtc tggtgaaggg cttctaccca agcgacatcg ccgtggagtg ggagagcaac 1140

ggccagcccg agaacaacta caagaccacc cccccagtgc tggacagcga cggcagcttc 1200

ttcctgtaca gcaagctgac cgtggacaag agcagatggc agcagggcaa cgtgttcagc 1260

tgctccgtga tgcacgaggc cctgcacaac cactacaccc agaagagcct gagcctgtcc 1320

ccaggc 1326

<210> 36
<211> 1317
<212> DNA
<213> Artificial

<220>
<223> m9G4_IgG4 Complete Heavy Chain

<400> 36
gaagtgatgt tggtagaaag cggtggtggg ctggtgaaac ctggcgggtc cctgaaactc 60

tcctgtactg ctagtggatt cacattcagc acttacgaca tgtcatgggt ccggcagacc 120

ccagagaaga gactggagtg ggtcagcact atctcctctt ctcggaccta tacgtattac 180

agtgccagcg tcaagggacg ctttaccatt agcagggata atgccagaaa catactgtac 240

ctgcagatgt caagcctccg aagtgaggac accgccatgt attactgcgc taggcaattt 300

tcacactggg gccaggggac attggttacc gtgtctgcag cgtccacaaa gggccccagc 360

gtgttccctc tggccccctg cagcagaagc accagcgagt ctacagccgc cctgggatgc 420

ctggtcaagg actacttccc cgagcccgtg accgtgtcct ggaactctgg cgccctgacc 480

agcggcgtgc acacctttcc agccgtgctg cagagcagcg gcctgtacag cctgagcagc 540

gtcgtgaccg tgcctagcag cagcctgggc accaagacct acacctgtaa cgtggaccac 600

aagcccagca acaccaaggt ggacaagcgg gtggaatcta agtacggccc tccctgcccc 660

ccctgcccag cccctgaatt tctgggcgga ccctccgtgt tcctgttccc cccaaagccc 720

aaggacaccc tgatgatcag ccggaccccc gaagtgacct gcgtggtggt ggacgtgtcc 780

caggaagatc ccgaggtcca gttcaactgg tacgtggacg gcgtggaagt gcacaacgcc 840

aagaccaagc ccagagagga acagttcaac agcacctacc gggtggtgtc cgtgctgacc 900

gtgctgcacc aggactggct gaacggcaaa gagtacaagt gcaaggtctc caacaagggc 960

ctgcccagct ccatcgagaa aaccatcagc aaggccaagg gccagccccg cgagccccag 1020

gtgtacacac tgccccccag ccaggaagag atgaccaaga accaggtgtc cctgacctgc 1080

ctcgtgaagg gcttctaccc cagcgatatc gccgtggaat gggagagcaa cggccagccc 1140

gagaacaact acaagaccac cccccctgtg ctggacagcg acggcagctt cttcctgtac 1200

tcccggctga ccgtcgacaa gagccggtgg caggaaggca acgtcttcag ctgcagcgtg 1260

atgcacgagg ccctgcacaa ccactacacc cagaagtccc tgagcctgag cctggga 1317

<210> 37
<211> 1338
<212> DNA
<213> Artificial

<220>
<223> hNHS76_IgG1 Complete Heavy Chain

<400> 37
caggtgcagc tgcaggagtc cggcccagga ctggtgaagc cttcggagac cctgtccctc 60

acctgcgctg tctctggtta ctccatcagc agtggttact actggggctg gattcggcag 120

cccccaggga aggggctgga gtggattggg agtatctatc atagtgggag cacctactac 180

aacccgtccc tcaagagtcg agtcaccata tcagtagaca cgtccaagaa ccagttctcc 240

ctgaagctga gctctgtgac cgccgcagac acggccgtgt attactgtgc aagagggaag 300

tggtcgaagt ttgactattg gggccaaggc accctggtca ccgtctcttc agcgtccaca 360

aagggcccaa gcgtgttccc gctagccccc agcagcaaga gcaccagcgg cggcacagcc 420

gccctgggct gcctggtgaa ggactacttc cccgagcccg tgaccgtgtc ctggaacagc 480

ggagccctga cctccggcgt gcacaccttc cccgccgtgc tgcagagcag cggcctgtac 540

agcctgagca gcgtggtgac cgtgcccagc agcagcctgg gcacccagac ctacatctgt 600

aacgtgaacc acaagcccag caacaccaag gtggacaaga gagtggagcc caagagctgt 660

gacaagaccc acacctgtcc cccctgccca gcccccgagc tgctgggcgg acccagcgtg 720

ttcctgttcc cccccaagcc caaggacacc ctgatgatca gcagaacccc cgaggtgacc 780

tgtgtggtgg tggacgtgtc ccacgaggac ccagaggtga agttcaactg gtacgtggac 840

ggcgtggagg tgcacaacgc caagaccaag cccagagagg agcagtacaa cagcacctac 900

agggtggtgt ccgtgctgac cgtgctgcac caggactggc tgaacggcaa ggagtacaag 960

tgtaaggtgt ccaacaaggc cctgccagcc ccaatcgaaa agaccatcag caaggccaag 1020

ggccagccaa gagagcccca ggtgtacacc ctgccaccca gcagggagga gatgaccaag 1080

aaccaggtgt ccctgacctg tctggtgaag ggcttctacc caagcgacat cgccgtggag 1140

tgggagagca acggccagcc cgagaacaac tacaagacca cccccccagt gctggacagc 1200

gacggcagct tcttcctgta cagcaagctg accgtggaca agagcagatg gcagcagggc 1260

aacgtgttca gctgctccgt gatgcacgag gccctgcaca accactacac ccagaagagc 1320

ctgagcctgt ccccaggc 1338

<210> 38
<211> 1211
<212> DNA
<213> Artificial

<220>
<223> hNHS76_IgG4 Complete Heavy Chain

<400> 38
caggtgcagc tgcaggagtc cggcccagga ctggtgaagc cttcggagac cctgtccctc 60

acctgcgctg tctctggtta ctccatcagc agtggttact actggggctg gattcggcag 120

cccccaggga aggggctgga gtggattggg agtatctatc atagtgggag cacctactac 180

aacccgtccc tcaagagtcg agtcaccata tcagtagaca cgtccaagaa ccagttctcc 240

ctgaagctga gctctgtgac cgccgcagac acggccgtgt attactgtgc aagagggaag 300

tggtcgaagt ttgactattg gggccaaggc accctggtca ccgtctcttc agcgtccaca 360

aagggcccca gcgtgttccc tctggccccc tgcagcagaa gcaccagcga gtctacagcc 420

gccctgggat gcctggtcaa ggactacttc cccgagcccg tgaccgtgtc ctggaactct 480

ggcgccctga ccagcggcgt gcacaccttt ccagccgtgc tgcagagcag cggcctgtac 540

agcctgagca gcgtcgtgac cgtgcctagc agcagcctgg gcaccaagac ctacacctgt 600

aacgtggacc acaagcccag caacaccaag gtggacaagc gggtggaatc taagtacggc 660

cctccctgcc ccccctgccc agcccctgaa tttctgggcg gaccctccgt gttcctgttc 720

cccccaaagc ccaaggacac cctgatgatc agccggaccc ccgaagtgac ctgcgtggtg 780

gtggacgtgt cccaggaaga tcccgaggtc cagttcaact ggtacgtgga cggcgtggaa 840

gtgcacaacg ccaagaccaa gcccagagag gaacagttca acagcaccta ccgggtggtg 900

tccgtgctga ccgtgctgca ccaggactgg ctgaacggca aagagtacaa gtgcaaggtc 960

tccaacaagg gcctgcccag ctccatcgag aaaaccatca gcaaggccaa gggccagccc 1020

cgcgagcccc aggtgtacac actgcccccc agccaggaag agatgaccaa gaaccaggtg 1080

tccctgacct gcctcgtgaa gggcttctac cccagcgata tcgccgtgga atgggagagc 1140

aacggccagc ccgagaacaa ctacaagacc accccccctg tgctggacag cgacggcagc 1200

ttcttcctgt a 1211

<210> 39
<211> 6
<212> PRT
<213> Artificial

<220>
<223> linker

<400> 39

Pro Val Gly Leu Ile Gly
1               5

<210> 40
<211> 12

<212>  PRT
<213>  Artificial

<220>
<223>  linker

<400>  40

Pro Val Gly Leu Ile Gly Pro Val Gly Leu Ile Gly
1               5                   10


<210>  41
<211>  30
<212>  PRT
<213>  Artificial

<220>
<223>  linker

<400>  41

Gly Gly Gly Gly Ser Gly Gly Gly Gly Ser Gly Gly Gly Gly Ser Gly
1               5                   10                  15


Gly Gly Gly Ser Gly Gly Gly Gly Ser Gly Gly Gly Ala Ser
            20                  25                  30


<210>  42
<211>  4
<212>  PRT
<213>  Artificial

<220>
<223>  linker

<400>  42

Ser Gly Arg Ser
1


<210>  43
<211>  5
<212>  PRT
<213>  Artificial

<220>
<223>  linker

<400>  43

Ser Gly Arg Ser Ala
1               5

<210>  44
<211>  18
<212>  DNA
<213>  Artificial

<220>
<223>  linker

<400>  44
ccggtgggcc tgattggc                                                    18


<210>  45
<211>  36
<212>  DNA
<213>  Artificial

<220>
<223>  linker

<400>  45
cctgtcggac tcatcggacc ggtgggcctg attggc                                36


<210>  46
<211>  90
<212>  DNA
<213>  Artificial

<220>
<223>  linker

<400>  46
ggaggcggag gatctggtgg tggtggatct ggcggcggag gaagtggcgg aggcggaagc      60

ggtggtggcg gttcaggtgg cggagctagc                                       90


<210>  47
<211>  12
<212>  DNA
<213>  Artificial

<220>
<223>  linker

<400>  47
tcaggcagat ct                                                          12


<210>  48
<211>  15
<212>  DNA
<213>  Artificial

<220>
<223>  linker

<400>  48
tcaggcagat ctgcc                                                        15

<210>  49
<211>  114
<212>  PRT
<213>  Homo sapiens

<400>  49

Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asn Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

<210>  50
<211>  342
<212>  DNA
<213>  Homo sapiens

<400>  50
aactgggtga atgtaataag tgatttgaaa aaaattgaag atcttattca atctatgcat       60

attgatgcta ctttatatac ggaaagtgat gttcacccca gttgcaaagt aacagcaatg      120

aagtgctttc tcttggagtt acaagttatt tcacttgagt ccggagatgc aagtattcat 180

gatacagtag aaaatctgat catcctagca aacaacagtt tgtcttctaa tgggaatgta 240

acagaatctg gatgcaaaga atgtgaggaa ctggaggaaa aaaatattaa agaatttttg 300

cagagttttg tacatattgt ccaaatgttc atcaacactt ct 342

<210> 51
<211> 65
<212> PRT
<213> Homo sapiens

<400> 51

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg
65

<210> 52
<211> 77
<212> PRT
<213> Homo sapiens

<400> 52

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Asp Pro Ala Leu Val His Gln Arg Pro Ala Pro Pro
65                  70                  75

<210>  53
<211>  175
<212>  PRT
<213>  Homo sapiens

<400>  53

Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly
            20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Asp Pro Ala Leu Val His Gln Arg Pro Ala Pro Pro Ser Thr Val
65                  70                  75                  80

Thr Thr Ala Gly Val Thr Pro Gln Pro Glu Ser Leu Ser Pro Ser Gly
                85                  90                  95

Lys Glu Pro Ala Ala Ser Ser Pro Ser Ser Asn Asn Thr Ala Ala Thr
            100                 105                 110

Thr Ala Ala Ile Val Pro Gly Ser Gln Leu Met Pro Ser Lys Ser Pro
        115                 120                 125

Ser Thr Gly Thr Thr Glu Ile Ser Ser His Glu Ser Ser His Gly Thr
    130                 135                 140

Pro Ser Gln Thr Thr Ala Lys Asn Trp Glu Leu Thr Ala Ser Ala Ser
145                 150                 155                 160

His Gln Pro Pro Gly Val Tyr Pro Gln Gly His Ser Asp Thr Thr

165                 170                 175

<210>  54
<211>  195
<212>  DNA
<213>  Homo sapiens

<400>  54
atcacctgtc ctccacctat gagcgtggaa cacgccgaca tctgggtcaa gagctacagc      60

ctgtacagca gagagcggta catctgcaac agcggcttca agagaaaggc cggcacaagc     120

agcctgaccg agtgtgtgct gaacaaggcc acaaacgtgg cccactggac cacacctagc     180

ctgaagtgca tcaga                                                      195

<210>  55
<211>  231
<212>  DNA
<213>  Homo sapiens

<400>  55
atcacctgtc ctccacctat gagcgtggaa cacgccgaca tctgggtcaa gagctacagc      60

ctgtacagca gagagcggta catctgcaac agcggcttca agagaaaggc cggcacaagc     120

agcctgaccg agtgtgtgct gaacaaggcc acaaacgtgg cccactggac cacacctagc     180

ctgaagtgca tcagagaccc cgccctggtg caccagcggc ccgccccccc c              231

<210>  56
<211>  525
<212>  DNA
<213>  Homo sapiens

<400>  56
atcacctgtc ctccacctat gagcgtggaa cacgccgaca tctgggtcaa gagctacagc      60

ctgtacagca gagagcggta catctgcaac agcggcttca agagaaaggc cggcacaagc     120

agcctgaccg agtgtgtgct gaacaaggcc acaaacgtgg cccactggac cacacctagc     180

ctgaagtgca tcagagatcc cgctctggtc catcagaggc ctgctcctcc atctacagtg     240

acaacagctg gcgtgacccc tcagcctgag tctctgtctc catctggaaa agagcctgcc     300

gccagctctc ccagctctaa caatactgct gccaccacag ccgccatcgt gcctggatct     360

cagctgatgc ctagcaagag ccctagcacc ggcacaacag agatcagctc tcacgagtct     420

agccacggca ccccatctca gaccacagcc aagaattggg aactgaccgc ctctgcctct     480

catcagccac ctggtgttta ccctcaaggc cactctgata ccacc                     525

<210>  57
<211>  568
<212>  PRT
<213>  Artificial

<220>
<223>  Ganitumab_IgG1_Heavy Chain_PVGLIG_IL15

<400>  57

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Pro Val Gly Leu Ile Gly Asn Trp Val Asn Val Ile Ser Asp Leu Lys
    450                 455                 460

Lys Ile Glu Asp Leu Ile Gln Ser Met His Ile Asp Ala Thr Leu Tyr
465                 470                 475                 480

Thr Glu Ser Asp Val His Pro Ser Cys Lys Val Thr Ala Met Lys Cys
                485                 490                 495

Phe Leu Leu Glu Leu Gln Val Ile Ser Leu Glu Ser Gly Asp Ala Ser
            500                 505                 510

Ile His Asp Thr Val Glu Asn Leu Ile Ile Leu Ala Asn Asn Ser Leu
        515                 520                 525

Ser Ser Asn Gly Asn Val Thr Glu Ser Gly Cys Lys Glu Cys Glu Glu
    530                 535                 540

Leu Glu Glu Lys Asn Ile Lys Glu Phe Leu Gln Ser Phe Val His Ile
545                 550                 555                 560

Val Gln Met Phe Ile Asn Thr Ser
                565

<210> 58
<211> 565
<212> PRT
<213> Artificial

<220>
<223> Ganitumab_IgG4_Heavy Chain_PVGLIG_IL15

<400> 58

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Pro Val Gly
        435                 440                 445

Leu Ile Gly Asn Trp Val Asn Val Ile Ser Asp Leu Lys Lys Ile Glu
    450                 455                 460

Asp Leu Ile Gln Ser Met His Ile Asp Ala Thr Leu Tyr Thr Glu Ser
465                 470                 475                 480

Asp Val His Pro Ser Cys Lys Val Thr Ala Met Lys Cys Phe Leu Leu
                485                 490                 495

Glu Leu Gln Val Ile Ser Leu Glu Ser Gly Asp Ala Ser Ile His Asp
            500                 505                 510

Thr Val Glu Asn Leu Ile Ile Leu Ala Asn Asn Ser Leu Ser Ser Asn
        515                 520                 525

Gly Asn Val Thr Glu Ser Gly Cys Lys Glu Cys Glu Glu Leu Glu Glu
    530                 535                 540

Lys Asn Ile Lys Glu Phe Leu Gln Ser Phe Val His Ile Val Gln Met
545                 550                 555                 560

Phe Ile Asn Thr Ser
                565

<210>  59
<211>  562
<212>  PRT
<213>  Artificial

<220>
<223>  m9G4_IgG1_Heavy Chain_PVGLIG_IL15

<400>  59

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Arg Val Glu Pro Lys Ser Cys Asp Lys Thr His Thr Cys Pro Pro
    210                 215                 220

Cys Pro Ala Pro Glu Leu Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Pro Val Gly Leu Ile Gly
        435                 440                 445

Asn Trp Val Asn Val Ile Ser Asp Leu Lys Lys Ile Glu Asp Leu Ile
    450                 455                 460

Gln Ser Met His Ile Asp Ala Thr Leu Tyr Thr Glu Ser Asp Val His
465                 470                 475                 480

Pro Ser Cys Lys Val Thr Ala Met Lys Cys Phe Leu Leu Glu Leu Gln
                485                 490                 495

Val Ile Ser Leu Glu Ser Gly Asp Ala Ser Ile His Asp Thr Val Glu
            500                 505                 510

Asn Leu Ile Ile Leu Ala Asn Asn Ser Leu Ser Ser Asn Gly Asn Val
        515                 520                 525

Thr Glu Ser Gly Cys Lys Glu Cys Glu Glu Leu Glu Glu Lys Asn Ile
    530                 535                 540

Lys Glu Phe Leu Gln Ser Phe Val His Ile Val Gln Met Phe Ile Asn
545                 550                 555                 560

Thr Ser

<210>  60
<211>  559
<212>  PRT
<213>  Artificial

<220>
<223>  m9G4_IgG4_Heavy Chain_PVGLIG_IL15

<400>  60

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Pro Val Gly Leu Ile Gly Asn Trp Val
        435                 440                 445

Asn Val Ile Ser Asp Leu Lys Lys Ile Glu Asp Leu Ile Gln Ser Met
    450                 455                 460

His Ile Asp Ala Thr Leu Tyr Thr Glu Ser Asp Val His Pro Ser Cys
465                 470                 475                 480

Lys Val Thr Ala Met Lys Cys Phe Leu Leu Glu Leu Gln Val Ile Ser
                485                 490                 495

Leu Glu Ser Gly Asp Ala Ser Ile His Asp Thr Val Glu Asn Leu Ile
            500                 505                 510

Ile Leu Ala Asn Asn Ser Leu Ser Ser Asn Gly Asn Val Thr Glu Ser
        515                 520                 525

Gly Cys Lys Glu Cys Glu Glu Leu Glu Glu Lys Asn Ile Lys Glu Phe
    530                 535                 540

Leu Gln Ser Phe Val His Ile Val Gln Met Phe Ile Asn Thr Ser
545                 550                 555

<210>  61
<211>  566

<212>  PRT
<213>  Artificial

<220>
<223>  NHS76_IgG1_Heavy Chain_PVGLIG_IL15

<400>  61

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Pro Val
        435                 440                 445

Gly Leu Ile Gly Asn Trp Val Asn Val Ile Ser Asp Leu Lys Lys Ile
    450                 455                 460

Glu Asp Leu Ile Gln Ser Met His Ile Asp Ala Thr Leu Tyr Thr Glu
465                 470                 475                 480

Ser Asp Val His Pro Ser Cys Lys Val Thr Ala Met Lys Cys Phe Leu
                485                 490                 495

Leu Glu Leu Gln Val Ile Ser Leu Glu Ser Gly Asp Ala Ser Ile His
            500                 505                 510

Asp Thr Val Glu Asn Leu Ile Ile Leu Ala Asn Asn Ser Leu Ser Ser
        515                 520                 525

Asn Gly Asn Val Thr Glu Ser Gly Cys Lys Glu Cys Glu Glu Leu Glu
    530                 535                 540

Glu Lys Asn Ile Lys Glu Phe Leu Gln Ser Phe Val His Ile Val Gln
545                 550                 555                 560

Met Phe Ile Asn Thr Ser
                565

<210> 62
<211> 563
<212> PRT
<213> Artificial

<220>
<223> NHS76_IgG4_Heavy Chain_PVGLIG_IL15

<400> 62

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Pro Val Gly Leu Ile
        435                 440                 445

Gly Asn Trp Val Asn Val Ile Ser Asp Leu Lys Lys Ile Glu Asp Leu
    450                 455                 460

Ile Gln Ser Met His Ile Asp Ala Thr Leu Tyr Thr Glu Ser Asp Val
465                 470                 475                 480

His Pro Ser Cys Lys Val Thr Ala Met Lys Cys Phe Leu Leu Glu Leu
                485                 490                 495

Gln Val Ile Ser Leu Glu Ser Gly Asp Ala Ser Ile His Asp Thr Val
            500                 505                 510

Glu Asn Leu Ile Ile Leu Ala Asn Asn Ser Leu Ser Ser Asn Gly Asn
        515                 520                 525

Val Thr Glu Ser Gly Cys Lys Glu Cys Glu Glu Leu Glu Glu Lys Asn
    530                 535                 540

Ile Lys Glu Phe Leu Gln Ser Phe Val His Ile Val Gln Met Phe Ile
545                 550                 555                 560

Asn Thr Ser

<210>  63
<211>  567
<212>  PRT
<213>  Artificial

<220>
<223>  Ganitumab_IgG1_Heavy Chain_SGRSA_IL15

<400>  63

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Ser Gly Arg Ser Ala Asn Trp Val Asn Val Ile Ser Asp Leu Lys Lys
    450                 455                 460

Ile Glu Asp Leu Ile Gln Ser Met His Ile Asp Ala Thr Leu Tyr Thr
465                 470                 475                 480

Glu Ser Asp Val His Pro Ser Cys Lys Val Thr Ala Met Lys Cys Phe
                485                 490                 495

Leu Leu Glu Leu Gln Val Ile Ser Leu Glu Ser Gly Asp Ala Ser Ile
            500                 505                 510

His Asp Thr Val Glu Asn Leu Ile Ile Leu Ala Asn Asn Ser Leu Ser
        515                 520                 525

Ser Asn Gly Asn Val Thr Glu Ser Gly Cys Lys Glu Cys Glu Glu Leu
    530                 535                 540

Glu Glu Lys Asn Ile Lys Glu Phe Leu Gln Ser Phe Val His Ile Val
545                 550                 555                 560

Gln Met Phe Ile Asn Thr Ser
                565

<210>  64
<211>  1704
<212>  DNA
<213>  Artificial

<220>
<223>  Ganitumab_IgG1_Heavy Chain_PVGLIG_IL15

<400>  64
caggtgcagc tgcaggagtc gggaccagga ctggtgaagc cttcggggac cctgtccctc      60

acctgcgctg tctctggtgg ctccatcagc agtagtaact ggtggagttg ggtccgccag     120

cccccaggga aggggctgga gtggattggg gaaatctatc atagtgggag caccaactac     180

aacccgtccc tcaagagtcg agtcaccata tcagtagaca agtccaagaa ccagttctcc     240

ctgaagctga gctctgtgac cgccgcggac acggccgtgt attactgtgc gagatggacc     300

gggcgtactg atgcttttga tatctggggc caagggacaa tggtcaccgt ctcaagcgcg     360

tccacaaagg gcccaagcgt gttcccgcta gcccccagca gcaagagcac cagcggcggc     420

acagccgccc tgggctgcct ggtgaaggac tacttccccg agcccgtgac cgtgtcctgg     480

aacagcggag ccctgacctc cggcgtgcac accttccccg ccgtgctgca gagcagcggc     540

ctgtacagcc tgagcagcgt ggtgaccgtg cccagcagca gcctgggcac ccagacctac     600

atctgtaacg tgaaccacaa gcccagcaac accaaggtgg acaagagagt ggagcccaag     660

agctgtgaca agacccacac ctgtcccccc tgcccagccc ccgagctgct gggcggaccc     720

agcgtgttcc tgttcccccc caagcccaag gacaccctga tgatcagcag aacccccgag     780

gtgacctgtg tggtggtgga cgtgtcccac gaggacccag aggtgaagtt caactggtac     840

gtggacggcg tggaggtgca caacgccaag accaagccca gagaggagca gtacaacagc     900

acctacaggg tggtgtccgt gctgaccgtg ctgcaccagg actggctgaa cggcaaggag     960

tacaagtgta aggtgtccaa caaggccctg ccagccccaa tcgaaaagac catcagcaag    1020

gccaagggcc agccaagaga gccccaggtg tacaccctgc cacccagcag ggaggagatg    1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggcccggtg ggcctgattg gcaactgggt gaatgtaata 1380

agtgatttga aaaaaattga agatcttatt caatctatgc atattgatgc tactttatat 1440

acggaaagtg atgttcaccc cagttgcaaa gtaacagcaa tgaagtgctt tctcttggag 1500

ttacaagtta tttcacttga gtccggagat gcaagtattc atgatacagt agaaaatctg 1560

atcatcctag caaacaacag tttgtcttct aatgggaatg taacagaatc tggatgcaaa 1620

gaatgtgagg aactggagga aaaaaatatt aaagaatttt tgcagagttt tgtacatatt 1680

gtccaaatgt tcatcaacac ttct 1704

<210> 65
<211> 1695
<212> DNA
<213> Artificial

<220>
<223> Ganitumab_IgG4_Heavy Chain_PVGLIG_IL15

<400> 65
   caggtgcagc tgcaggagtc gggaccagga ctggtgaagc cttcggggac cctgtccctc 60
   acctgcgctg tctctggtgg ctccatcagc agtagtaact ggtggagttg ggtccgccag 120
   cccccaggga aggggctgga gtggattggg gaaatctatc atagtgggag caccaactac 180
   aacccgtccc tcaagagtcg agtcaccata tcagtagaca agtccaagaa ccagttctcc 240
   ctgaagctga gctctgtgac cgccgcggac acggccgtgt attactgtgc gagatggacc 300
   gggcgtactg atgcttttga tatctggggc caagggacaa tggtcaccgt ctcaagcgcg 360
   tccacaaagg gccccagcgt gttccctctg gccccctgca gcagaagcac cagcgagtct 420
   acagccgccc tgggatgcct ggtcaaggac tacttccccg agcccgtgac cgtgtcctgg 480
   aactctggcg ccctgaccag cggcgtgcac acctttccag ccgtgctgca gagcagcggc 540
   ctgtacagcc tgagcagcgt cgtgaccgtg cctagcagca gcctgggcac caagacctac 600
   acctgtaacg tggaccacaa gcccagcaac accaaggtgg acaagcgggt ggaatctaag 660
   tacggccctc cctgcccccc ctgcccagcc cctgaatttc tgggcggacc ctccgtgttc 720
   ctgttccccc caaagcccaa ggacaccctg atgatcagcc ggacccccga agtgacctgc 780
   gtggtggtgg acgtgtccca ggaagatccc gaggtccagt tcaactggta cgtggacggc 840
   gtggaagtgc acaacgccaa gaccaagccc agagaggaac agttcaacag cacctaccgg 900
   gtggtgtccg tgctgaccgt gctgcaccag gactggctga acggcaaaga gtacaagtgc 960
   aaggtctcca acaagggcct gcccagctcc atcgagaaaa ccatcagcaa ggccaagggc 1020
   cagccccgcg agccccaggt gtacacactg ccccccagcc aggaagagat gaccaagaac 1080
   caggtgtccc tgacctgcct cgtgaagggc ttctacccca gcgatatcgc cgtggaatgg 1140
   gagagcaacg gccagcccga gaacaactac aagaccaccc cccctgtgct ggacagcgac 1200
   ggcagcttct tcctgtactc ccggctgacc gtcgacaaga gccggtggca ggaaggcaac 1260
   gtcttcagct gcagcgtgat gcacgaggcc ctgcacaacc actacaccca gaagtccctg 1320
   agcctgagcc tgggaccggt gggcctgatt ggcaactggg tgaatgtaat aagtgatttg 1380
   aaaaaaattg aagatcttat tcaatctatg catattgatg ctactttata tacggaaagt 1440
   gatgttcacc ccagttgcaa agtaacagca atgaagtgct ttctcttgga gttacaagtt 1500
   atttcacttg agtccggaga tgcaagtatt catgatacag tagaaaatct gatcatccta 1560
   gcaaacaaca gtttgtcttc taatgggaat gtaacagaat ctggatgcaa agaatgtgag 1620
   gaactggagg aaaaaaatat taaagaattt ttgcagagtt ttgtacatat tgtccaaatg 1680
   ttcatcaaca cttct 1695

<210> 66
<211> 1698
<212> DNA
<213> Artificial

<220>
<223> NHS76_IgG1_Heavy Chain_PVGLIG_IL15

<400> 66
caggtgcagc tgcaggagtc cggcccagga ctggtgaagc cttcggagac cctgtccctc 60

acctgcgctg tctctggtta ctccatcagc agtggttact actggggctg gattcggcag 120

cccccaggga aggggctgga gtggattggg agtatctatc atagtgggag cacctactac 180

aacccgtccc tcaagagtcg agtcaccata tcagtagaca cgtccaagaa ccagttctcc 240

ctgaagctga gctctgtgac cgccgcagac acggccgtgt attactgtgc aagagggaag 300

tggtcgaagt ttgactattg gggccaaggc accctggtca ccgtctcttc agcgtccaca 360

aagggcccaa gcgtgttccc gctagccccc agcagcaaga gcaccagcgg cggcacagcc 420

gccctgggct gcctggtgaa ggactacttc cccgagcccg tgaccgtgtc ctggaacagc 480

ggagccctga cctccggcgt gcacaccttc cccgccgtgc tgcagagcag cggcctgtac 540

agcctgagca gcgtggtgac cgtgcccagc agcagcctgg gcacccagac ctacatctgt 600

aacgtgaacc acaagcccag caacaccaag gtggacaaga gagtggagcc caagagctgt 660

gacaagaccc acacctgtcc cccctgccca gcccccgagc tgctgggcgg acccagcgtg 720

ttcctgttcc cccccaagcc caaggacacc ctgatgatca gcagaacccc cgaggtgacc 780

tgtgtggtgg tggacgtgtc ccacgaggac ccagaggtga agttcaactg gtacgtggac 840

ggcgtggagg tgcacaacgc caagaccaag cccagagagg agcagtacaa cagcacctac 900

agggtggtgt ccgtgctgac cgtgctgcac caggactggc tgaacggcaa ggagtacaag 960

tgtaaggtgt ccaacaaggc cctgccagcc ccaatcgaaa agaccatcag caaggccaag 1020

ggccagccaa gagagcccca ggtgtacacc ctgccaccca gcagggagga gatgaccaag 1080

aaccaggtgt ccctgacctg tctggtgaag ggcttctacc caagcgacat cgccgtggag 1140

tgggagagca acggccagcc cgagaacaac tacaagacca cccccccagt gctggacagc 1200

gacggcagct tcttcctgta cagcaagctg accgtggaca agagcagatg gcagcagggc 1260

aacgtgttca gctgctccgt gatgcacgag gccctgcaca accactacac ccagaagagc 1320

ctgagcctgt ccccaggccc ggtgggcctg attggcaact gggtgaatgt aataagtgat 1380

ttgaaaaaaa ttgaagatct tattcaatct atgcatattg atgctacttt atatacggaa 1440

agtgatgttc accccagttg caaagtaaca gcaatgaagt gctttctctt ggagttacaa 1500

gttatttcac ttgagtccgg agatgcaagt attcatgata cagtagaaaa tctgatcatc 1560

ctagcaaaca acagtttgtc ttctaatggg aatgtaacag aatctggatg caaagaatgt 1620

gaggaactgg aggaaaaaaa tattaaagaa tttttgcaga gttttgtaca tattgtccaa 1680

atgttcatca acacttct 1698

<210> 67
<211> 1689
<212> DNA
<213> Artificial

<220>
<223> NHS76_IgG4_Heavy Chain_PVGLIG_IL15

<400> 67
caggtgcagc tgcaggagtc cggcccagga ctggtgaagc cttcggagac cctgtccctc 60
acctgcgctg tctctggtta ctccatcagc agtggttact actggggctg gattcggcag 120
cccccaggga aggggctgga gtggattggg agtatctatc atagtgggag cacctactac 180
aacccgtccc tcaagagtcg agtcaccata tcagtagaca cgtccaagaa ccagttctcc 240
ctgaagctga gctctgtgac cgccgcagac acggccgtgt attactgtgc aagagggaag 300
tggtcgaagt ttgactattg gggccaaggc accctggtca ccgtctcttc agcgtccaca 360
aagggcccca gcgtgttccc tctggccccc tgcagcagaa gcaccagcga gtctacagcc 420
gccctgggat gcctggtcaa ggactacttc cccgagcccg tgaccgtgtc ctggaactct 480
ggcgccctga ccagcggcgt gcacaccttt ccagccgtgc tgcagagcag cggcctgtac 540
agcctgagca gcgtcgtgac cgtgcctagc agcagcctgg gcaccaagac ctacacctgt 600
aacgtggacc acaagcccag caacaccaag gtggacaagc gggtggaatc taagtacggc 660
cctccctgcc ccccctgccc agcccctgaa tttctgggcg gaccctccgt gttcctgttc 720
cccccaaagc ccaaggacac cctgatgatc agccggaccc ccgaagtgac ctgcgtggtg 780
gtggacgtgt cccaggaaga tcccgaggtc cagttcaact ggtacgtgga cggcgtggaa 840
gtgcacaacg ccaagaccaa gcccagagag gaacagttca acagcaccta ccgggtggtg 900
tccgtgctga ccgtgctgca ccaggactgg ctgaacggca aagagtacaa gtgcaaggtc 960
tccaacaagg gcctgcccag ctccatcgag aaaaccatca gcaaggccaa gggccagccc 1020
cgcgagcccc aggtgtacac actgcccccc agccaggaag agatgaccaa gaaccaggtg 1080
tccctgacct gcctcgtgaa gggcttctac cccagcgata tcgccgtgga atgggagagc 1140
aacggccagc ccgagaacaa ctacaagacc accccccctg tgctggacag cgacggcagc 1200
ttcttcctgt actcccggct gaccgtcgac aagagccggt ggcaggaagg caacgtcttc 1260
agctgcagcg tgatgcacga ggccctgcac aaccactaca cccagaagtc cctgagcctg 1320
agcctgggac cggtgggcct gattggcaac tgggtgaatg taataagtga tttgaaaaaa 1380
attgaagatc ttattcaatc tatgcatatt gatgctactt tatatacgga aagtgatgtt 1440
caccccagtt gcaaagtaac agcaatgaag tgctttctct tggagttaca agttatttca 1500
cttgagtccg gagatgcaag tattcatgat acagtagaaa atctgatcat cctagcaaac 1560
aacagtttgt cttctaatgg gaatgtaaca gaatctggat gcaaagaatg tgaggaactg 1620
gaggaaaaaa atattaaaga atttttgcag agttttgtac atattgtcca aatgttcatc 1680

aacacttct                                                          1689

<210>  68
<211>  1686
<212>  DNA
<213>  Artificial

<220>
<223>  m9G4_IgG1_Heavy Chain_PVGLIG_IL15

<400>  68
gaagtgatgt tggtagaaag cggtggtggg ctggtgaaac ctggcgggtc cctgaaactc      60

tcctgtactg ctagtggatt cacattcagc acttacgaca tgtcatgggt ccggcagacc     120

ccagagaaga gactggagtg ggtcagcact atctcctctt ctcggaccta tacgtattac     180

agtgccagcg tcaagggacg ctttaccatt agcagggata atgccagaaa catactgtac     240

ctgcagatgt caagcctccg aagtgaggac accgccatgt attactgcgc taggcaattt     300

tcacactggg gccaggggac attggttacc gtgtctgcag cgtccacaaa gggcccaagc     360

gtgttcccgc tagcccccag cagcaagagc accagcggcg gcacagccgc cctgggctgc     420

ctggtgaagg actacttccc cgagcccgtg accgtgtcct ggaacagcgg agccctgacc     480

tccggcgtgc acaccttccc cgccgtgctg cagagcagcg gcctgtacag cctgagcagc     540

gtggtgaccg tgcccagcag cagcctgggc acccagacct acatctgtaa cgtgaaccac     600

aagcccagca acaccaaggt ggacaagaga gtggagccca agagctgtga caagacccac     660

acctgtcccc cctgcccagc ccccgagctg ctgggcggac ccagcgtgtt cctgttcccc      720

cccaagccca aggacaccct gatgatcagc agaacccccg aggtgacctg tgtggtggtg     780

gacgtgtccc acgaggaccc agaggtgaag ttcaactggt acgtggacgg cgtggaggtg     840

cacaacgcca agaccaagcc cagagaggag cagtacaaca gcacctacag ggtggtgtcc     900

gtgctgaccg tgctgcacca ggactggctg aacggcaagg agtacaagtg taaggtgtcc     960

aacaaggccc tgccagcccc aatcgaaaag accatcagca aggccaaggg ccagccaaga    1020

gagccccagg tgtacaccct gccacccagc agggaggaga tgaccaagaa ccaggtgtcc    1080

ctgacctgtc tggtgaaggg cttctaccca agcgacatcg ccgtggagtg ggagagcaac    1140

ggccagcccg agaacaacta caagaccacc cccccagtgc tggacagcga cggcagcttc    1200

ttcctgtaca gcaagctgac cgtggacaag agcagatggc agcagggcaa cgtgttcagc    1260

tgctccgtga tgcacgaggc cctgcacaac cactacaccc agaagagcct gagcctgtcc    1320

ccaggcccgg tgggcctgat tggcaactgg gtgaatgtaa taagtgattt gaaaaaaatt 1380

gaagatctta ttcaatctat gcatattgat gctactttat atacggaaag tgatgttcac 1440

cccagttgca aagtaacagc aatgaagtgc tttctcttgg agttacaagt tatttcactt 1500

gagtccggag atgcaagtat tcatgataca gtagaaaatc tgatcatcct agcaaacaac 1560

agtttgtctt ctaatgggaa tgtaacagaa tctggatgca aagaatgtga ggaactggag 1620

gaaaaaaata ttaaagaatt tttgcagagt tttgtacata ttgtccaaat gttcatcaac 1680

acttct 1686

<210> 69
<211> 1677
<212> DNA
<213> Artificial

<220>
<223> m9G4_IgG4_Heavy Chain_PVGLIG_IL15

<400> 69
gaagtgatgt tggtagaaag cggtggtggg ctggtgaaac ctggcgggtc cctgaaactc 60

tcctgtactg ctagtggatt cacattcagc acttacgaca tgtcatgggt ccggcagacc 120

ccagagaaga gactggagtg ggtcagcact atctcctctt ctcggaccta tacgtattac 180

agtgccagcg tcaagggacg ctttaccatt agcagggata atgccagaaa catactgtac 240

ctgcagatgt caagcctccg aagtgaggac accgccatgt attactgcgc taggcaattt 300

tcacactggg gccaggggac attggttacc gtgtctgcag cgtccacaaa gggccccagc 360

gtgttccctc tggccccctg cagcagaagc accagcgagt ctacagccgc cctgggatgc 420

ctggtcaagg actacttccc cgagcccgtg accgtgtcct ggaactctgg cgccctgacc 480

agcggcgtgc acacctttcc agccgtgctg cagagcagcg gcctgtacag cctgagcagc 540

gtcgtgaccg tgcctagcag cagcctgggc accaagacct acacctgtaa cgtggaccac 600

aagcccagca acaccaaggt ggacaagcgg gtggaatcta agtacggccc tccctgcccc 660

ccctgcccag cccctgaatt tctgggcgga ccctccgtgt tcctgttccc cccaaagccc 720

aaggacaccc tgatgatcag ccggaccccc gaagtgacct gcgtggtggt ggacgtgtcc 780

caggaagatc ccgaggtcca gttcaactgg tacgtggacg gcgtggaagt gcacaacgcc 840

aagaccaagc ccagagagga acagttcaac agcacctacc gggtggtgtc cgtgctgacc 900

gtgctgcacc aggactggct gaacggcaaa gagtacaagt gcaaggtctc caacaagggc 960

ctgcccagct ccatcgagaa aaccatcagc aaggccaagg gccagccccg cgagccccag 1020

gtgtacacac tgccccccag ccaggaagag atgaccaaga accaggtgtc cctgacctgc 1080

ctcgtgaagg gcttctaccc cagcgatatc gccgtggaat gggagagcaa cggccagccc 1140

gagaacaact acaagaccac cccccctgtg ctggacagcg acggcagctt cttcctgtac 1200

tcccggctga ccgtcgacaa gagccggtgg caggaaggca acgtcttcag ctgcagcgtg 1260

atgcacgagg ccctgcacaa ccactacacc cagaagtccc tgagcctgag cctgggaccg 1320

gtgggcctga ttggcaactg ggtgaatgta ataagtgatt tgaaaaaaat tgaagatctt 1380

attcaatcta tgcatattga tgctacttta tatacggaaa gtgatgttca ccccagttgc 1440

aaagtaacag caatgaagtg ctttctcttg gagttacaag ttatttcact tgagtccgga 1500

gatgcaagta ttcatgatac agtagaaaat ctgatcatcc tagcaaacaa cagtttgtct 1560

tctaatggga atgtaacaga atctggatgc aaagaatgtg aggaactgga ggaaaaaaat 1620

attaaagaat ttttgcagag ttttgtacat attgtccaaa tgttcatcaa cacttct 1677

<210> 70
<211> 1701
<212> DNA
<213> Artificial

<220>
<223> Ganitumab_IgG1_Heavy Chain_SGRSA_IL15

<400> 70
caggtgcagc tgcaggagtc gggaccagga ctggtgaagc cttcggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggctcaggc agatctgcca actgggtgaa tgtaataagt 1380

gatttgaaaa aaattgaaga tcttattcaa tctatgcata ttgatgctac tttatatacg 1440

gaaagtgatg ttcaccccag ttgcaaagta acagcaatga agtgctttct cttggagtta 1500

caagttattt cacttgagtc cggagatgca agtattcatg atacagtaga aaatctgatc 1560

atcctagcaa acaacagttt gtcttctaat gggaatgtaa cagaatctgg atgcaaagaa 1620

tgtgaggaac tggaggaaaa aaatattaaa gaatttttgc agagttttgt acatattgtc 1680

caaatgttca tcaacacttc t 1701

<210> 71
<211> 574
<212> PRT
<213> Artificial

<220>
<223> Ganitumab_IgG1_Heavy Chain_PVGLIG_IPVGLIG_L15

<400> 71

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Pro Val Gly Leu Ile Gly Pro Val Gly Leu Ile Gly Asn Trp Val Asn
    450                 455                 460

Val Ile Ser Asp Leu Lys Lys Ile Glu Asp Leu Ile Gln Ser Met His
465                 470                 475                 480

Ile Asp Ala Thr Leu Tyr Thr Glu Ser Asp Val His Pro Ser Cys Lys
                485                 490                 495

Val Thr Ala Met Lys Cys Phe Leu Leu Glu Leu Gln Val Ile Ser Leu
            500                 505                 510

Glu Ser Gly Asp Ala Ser Ile His Asp Thr Val Glu Asn Leu Ile Ile
        515                 520                 525

Leu Ala Asn Asn Ser Leu Ser Ser Asn Gly Asn Val Thr Glu Ser Gly
    530                 535                 540

Cys Lys Glu Cys Glu Glu Leu Glu Glu Lys Asn Ile Lys Glu Phe Leu
545                 550                 555                 560

Gln Ser Phe Val His Ile Val Gln Met Phe Ile Asn Thr Ser
                565                 570

<210> 72
<211> 571
<212> PRT
<213> Artificial

<220>
<223> Ganitumab_IgG4_Heavy Chain_PVGLIG_IPVGLIG_L15

<400> 72

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Pro Val Gly
        435                 440                 445

Leu Ile Gly Pro Val Gly Leu Ile Gly Asn Trp Val Asn Val Ile Ser
    450                 455                 460

Asp Leu Lys Lys Ile Glu Asp Leu Ile Gln Ser Met His Ile Asp Ala
465                 470                 475                 480

Thr Leu Tyr Thr Glu Ser Asp Val His Pro Ser Cys Lys Val Thr Ala
                485                 490                 495

Met Lys Cys Phe Leu Leu Glu Leu Gln Val Ile Ser Leu Glu Ser Gly
            500                 505                 510

Asp Ala Ser Ile His Asp Thr Val Glu Asn Leu Ile Ile Leu Ala Asn
        515                 520                 525

Asn Ser Leu Ser Ser Asn Gly Asn Val Thr Glu Ser Gly Cys Lys Glu
    530                 535                 540

Cys Glu Glu Leu Glu Glu Lys Asn Ile Lys Glu Phe Leu Gln Ser Phe
545                 550                 555                 560

Val His Ile Val Gln Met Phe Ile Asn Thr Ser
                565                 570

<210>  73
<211>  568
<212>  PRT
<213>  Artificial

<220>
<223>  m9G4_IgG1_Heavy Chain_PVGLIG_IPVGLIG_L15

<400>  73

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Ser Ser
        115                 120                 125

Lys Ser Thr Ser Gly Gly Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Gln
            180                 185                 190

Thr Tyr Ile Cys Asn Val Asn His Lys Pro Ser Asn Thr Lys Val Asp
        195                 200                 205

Lys Arg Val Glu Pro Lys Ser Cys Asp Lys Thr His Thr Cys Pro Pro
    210                 215                 220

Cys Pro Ala Pro Glu Leu Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Lys Phe Asn
            260                 265                 270

Trp Tyr Val Asp Gly Val Glu Val His Asn Ala Lys Thr Lys Pro Arg
        275                 280                 285

Glu Glu Gln Tyr Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Ala Leu Pro Ala Pro Ile Glu Lys Thr Ile Ser Lys Ala Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Pro Val Gly Leu Ile Gly
        435                 440                 445

Pro Val Gly Leu Ile Gly Asn Trp Val Asn Val Ile Ser Asp Leu Lys
    450                 455                 460

Lys Ile Glu Asp Leu Ile Gln Ser Met His Ile Asp Ala Thr Leu Tyr
465                 470                 475                 480

Thr Glu Ser Asp Val His Pro Ser Cys Lys Val Thr Ala Met Lys Cys
                485                 490                 495

Phe Leu Leu Glu Leu Gln Val Ile Ser Leu Glu Ser Gly Asp Ala Ser
            500                 505                 510

Ile His Asp Thr Val Glu Asn Leu Ile Ile Leu Ala Asn Asn Ser Leu
        515                 520                 525

Ser Ser Asn Gly Asn Val Thr Glu Ser Gly Cys Lys Glu Cys Glu Glu
    530                 535                 540

Leu Glu Glu Lys Asn Ile Lys Glu Phe Leu Gln Ser Phe Val His Ile
545                 550                 555                 560

Val Gln Met Phe Ile Asn Thr Ser
                565

<210>  74
<211>  565
<212>  PRT
<213>  Artificial

<220>
<223>  m9G4_IgG4_Heavy Chain_PVGLIG_IPVGLIG_L15

<400>  74

Glu Val Met Leu Val Glu Ser Gly Gly Gly Leu Val Lys Pro Gly Gly
1               5                   10                  15

Ser Leu Lys Leu Ser Cys Thr Ala Ser Gly Phe Thr Phe Ser Thr Tyr
            20                  25                  30

Asp Met Ser Trp Val Arg Gln Thr Pro Glu Lys Arg Leu Glu Trp Val
        35                  40                  45

Ser Thr Ile Ser Ser Ser Arg Thr Tyr Thr Tyr Tyr Ser Ala Ser Val
    50                  55                  60

Lys Gly Arg Phe Thr Ile Ser Arg Asp Asn Ala Arg Asn Ile Leu Tyr
65                  70                  75                  80

Leu Gln Met Ser Ser Leu Arg Ser Glu Asp Thr Ala Met Tyr Tyr Cys
                85                  90                  95

Ala Arg Gln Phe Ser His Trp Gly Gln Gly Thr Leu Val Thr Val Ser
            100                 105                 110

Ala Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr
                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Pro Val Gly Leu Ile Gly Pro Val Gly
        435                 440                 445

Leu Ile Gly Asn Trp Val Asn Val Ile Ser Asp Leu Lys Lys Ile Glu
    450                 455                 460

Asp Leu Ile Gln Ser Met His Ile Asp Ala Thr Leu Tyr Thr Glu Ser
465                 470                 475                 480

Asp Val His Pro Ser Cys Lys Val Thr Ala Met Lys Cys Phe Leu Leu
                485                 490                 495

Glu Leu Gln Val Ile Ser Leu Glu Ser Gly Asp Ala Ser Ile His Asp
            500                 505                 510

Thr Val Glu Asn Leu Ile Ile Leu Ala Asn Asn Ser Leu Ser Ser Asn
        515                 520                 525

Gly Asn Val Thr Glu Ser Gly Cys Lys Glu Cys Glu Glu Leu Glu Glu
    530                 535                 540

Lys Asn Ile Lys Glu Phe Leu Gln Ser Phe Val His Ile Val Gln Met
545                 550                 555                 560

Phe Ile Asn Thr Ser
                565

<210>  75
<211>  474
<212>  PRT
<213>  Artificial

<220>
<223>  NHS76_IgG1_Heavy Chain_PVGLIG_IPVGLIG_L15

<400>  75

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Pro Val
        435                 440                 445

Gly Leu Ile Gly Pro Val Gly Leu Ile Gly Asn Trp Val Asn Val Ile
    450                 455                 460

Ser Asp Leu Lys Lys Ile Glu Asp Leu Ile
465                 470

<210> 76
<211> 569
<212> PRT
<213> Artificial

<220>
<223> NHS76_IgG4_Heavy Chain_PVGLIG_IPVGLIG_L15

<400> 76

Gln Val Gln Leu Gln Glu Ser Gly Pro Gly Leu Val Lys Pro Ser Glu
1               5                   10                  15

Thr Leu Ser Leu Thr Cys Ala Val Ser Gly Tyr Ser Ile Ser Ser Gly
            20                  25                  30

Tyr Tyr Trp Gly Trp Ile Arg Gln Pro Pro Gly Lys Gly Leu Glu Trp
        35                  40                  45

Ile Gly Ser Ile Tyr His Ser Gly Ser Thr Tyr Tyr Asn Pro Ser Leu
    50                  55                  60

Lys Ser Arg Val Thr Ile Ser Val Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Gly Lys Trp Ser Lys Phe Asp Tyr Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Pro Val Gly Leu Ile
        435                 440                 445

Gly Pro Val Gly Leu Ile Gly Asn Trp Val Asn Val Ile Ser Asp Leu
    450                 455                 460

Lys Lys Ile Glu Asp Leu Ile Gln Ser Met His Ile Asp Ala Thr Leu
465                 470                 475                 480

Tyr Thr Glu Ser Asp Val His Pro Ser Cys Lys Val Thr Ala Met Lys
                485                 490                 495

Cys Phe Leu Leu Glu Leu Gln Val Ile Ser Leu Glu Ser Gly Asp Ala
            500                 505                 510

Ser Ile His Asp Thr Val Glu Asn Leu Ile Ile Leu Ala Asn Asn Ser
        515                 520                 525

Leu Ser Ser Asn Gly Asn Val Thr Glu Ser Gly Cys Lys Glu Cys Glu
    530                 535                 540

Glu Leu Glu Glu Lys Asn Ile Lys Glu Phe Leu Gln Ser Phe Val His
545                 550                 555                 560

Ile Val Gln Met Phe Ile Asn Thr Ser
                565

<210> 77
<211> 1722
<212> DNA
<213> Artificial

<220>
<223> Ganitumab_IgG1_Heavy Chain_PVGLIG_IPVGLIG_L15

<400> 77
caggtgcagc tgcaggagtc gggaccagga ctggtgaagc cttcggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggccctgtc ggactcatcg gaccggtggg cctgattggc 1380

aactgggtga atgtaataag tgatttgaaa aaaattgaag atcttattca atctatgcat 1440

attgatgcta ctttatatac ggaaagtgat gttcacccca gttgcaaagt aacagcaatg 1500

aagtgctttc tcttggagtt acaagttatt tcacttgagt ccggagatgc aagtattcat 1560

gatacagtag aaaatctgat catcctagca aacaacagtt tgtcttctaa tgggaatgta 1620

acagaatctg gatgcaaaga atgtgaggaa ctggaggaaa aaaatattaa agaatttttg 1680

cagagttttg tacatattgt ccaaatgttc atcaacactt ct 1722

<210> 78
<211> 1713

<212>  DNA
<213>  Artificial

<220>
<223>  Ganitumab_IgG4_Heavy Chain_PVGLIG_IPVGLIG_L15

<400>  78
caggtgcagc tgcaggagtc gggaccagga ctggtgaagc cttcggggac cctgtccctc      60

acctgcgctg tctctggtgg ctccatcagc agtagtaact ggtggagttg ggtccgccag     120

cccccaggga aggggctgga gtggattggg gaaatctatc atagtgggag caccaactac     180

aacccgtccc tcaagagtcg agtcaccata tcagtagaca agtccaagaa ccagttctcc     240

ctgaagctga gctctgtgac cgccgcggac acggccgtgt attactgtgc gagatggacc     300

gggcgtactg atgcttttga tatctggggc caagggacaa tggtcaccgt ctcaagcgcg     360

tccacaaagg gccccagcgt gttccctctg gccccctgca gcagaagcac cagcgagtct     420

acagccgccc tgggatgcct ggtcaaggac tacttccccg agcccgtgac cgtgtcctgg     480

aactctggcg ccctgaccag cggcgtgcac acctttccag ccgtgctgca gagcagcggc     540

ctgtacagcc tgagcagcgt cgtgaccgtg cctagcagca gcctgggcac caagacctac     600

acctgtaacg tggaccacaa gcccagcaac accaaggtgg acaagcgggt ggaatctaag     660

tacggccctc cctgcccccc ctgcccagcc cctgaatttc tgggcggacc ctccgtgttc     720

ctgttccccc caaagcccaa ggacaccctg atgatcagcc ggacccccga agtgacctgc     780

gtggtggtgg acgtgtccca ggaagatccc gaggtccagt tcaactggta cgtggacggc     840

gtggaagtgc acaacgccaa gaccaagccc agagaggaac agttcaacag cacctaccgg     900

gtggtgtccg tgctgaccgt gctgcaccag gactggctga acggcaaaga gtacaagtgc     960

aaggtctcca acaagggcct gcccagctcc atcgagaaaa ccatcagcaa ggccaagggc    1020

cagccccgcg agccccaggt gtacacactg ccccccagcc aggaagagat gaccaagaac    1080

caggtgtccc tgacctgcct cgtgaagggc ttctacccca gcgatatcgc cgtggaatgg    1140

gagagcaacg gccagcccga gaacaactac aagaccaccc cccctgtgct ggacagcgac    1200

ggcagcttct tcctgtactc ccggctgacc gtcgacaaga gccggtggca ggaaggcaac    1260

gtcttcagct gcagcgtgat gcacgaggcc ctgcacaacc actacaccca gaagtccctg    1320

agcctgagcc tgggacctgt cggactcatc ggaccggtgg gcctgattgg caactgggtg    1380

aatgtaataa gtgatttgaa aaaaattgaa gatcttattc aatctatgca tattgatgct    1440

actttatata cggaaagtga tgttcacccc agttgcaaag taacagcaat gaagtgcttt    1500

ctcttggagt tacaagttat ttcacttgag tccggagatg caagtattca tgatacagta 1560

gaaaatctga tcatcctagc aaacaacagt ttgtcttcta atgggaatgt aacagaatct 1620

ggatgcaaag aatgtgagga actggaggaa aaaaatatta aagaattttt gcagagtttt 1680

gtacatattg tccaaatgtt catcaacact tct 1713

<210> 79
<211> 1704
<212> DNA
<213> Artificial

<220>
<223> m9G4_IgG1_Heavy Chain_PVGLIG_IPVGLIG_L15

<400> 79
gaagtgatgt tggtagaaag cggtggtggg ctggtgaaac ctggcgggtc cctgaaactc 60

tcctgtactg ctagtggatt cacattcagc acttacgaca tgtcatgggt ccggcagacc 120

ccagagaaga gactggagtg ggtcagcact atctcctctt ctcggaccta tacgtattac 180

agtgccagcg tcaagggacg ctttaccatt agcagggata atgccagaaa catactgtac 240

ctgcagatgt caagcctccg aagtgaggac accgccatgt attactgcgc taggcaattt 300

tcacactggg gccaggggac attggttacc gtgtctgcag cgtccacaaa gggcccaagc 360

gtgttcccgc tagcccccag cagcaagagc accagcggcg gcacagccgc cctgggctgc 420

ctggtgaagg actacttccc cgagcccgtg accgtgtcct ggaacagcgg agccctgacc 480

tccggcgtgc acaccttccc cgccgtgctg cagagcagcg gcctgtacag cctgagcagc 540

gtggtgaccg tgcccagcag cagcctgggc acccagacct acatctgtaa cgtgaaccac 600

aagcccagca acaccaaggt ggacaagaga gtggagccca agagctgtga caagacccac 660

acctgtcccc cctgcccagc ccccgagctg ctgggcggac ccagcgtgtt cctgttcccc 720

cccaagccca aggacaccct gatgatcagc agaacccccg aggtgacctg tgtggtggtg 780

gacgtgtccc acgaggaccc agaggtgaag ttcaactggt acgtggacgg cgtggaggtg 840

cacaacgcca agaccaagcc cagagaggag cagtacaaca gcacctacag ggtggtgtcc 900

gtgctgaccg tgctgcacca ggactggctg aacggcaagg agtacaagtg taaggtgtcc 960

aacaaggccc tgccagcccc aatcgaaaag accatcagca aggccaaggg ccagccaaga 1020

gagccccagg tgtacaccct gccacccagc agggaggaga tgaccaagaa ccaggtgtcc 1080

ctgacctgtc tggtgaaggg cttctaccca agcgacatcg ccgtggagtg ggagagcaac 1140

ggccagcccg agaacaacta caagaccacc cccccagtgc tggacagcga cggcagcttc 1200

ttcctgtaca gcaagctgac cgtggacaag agcagatggc agcagggcaa cgtgttcagc 1260

tgctccgtga tgcacgaggc cctgcacaac cactacaccc agaagagcct gagcctgtcc 1320

ccaggccctg tcggactcat cggaccggtg ggcctgattg gcaactgggt gaatgtaata 1380

agtgatttga aaaaaattga agatcttatt caatctatgc atattgatgc tactttatat 1440

acggaaagtg atgttcaccc cagttgcaaa gtaacagcaa tgaagtgctt tctcttggag 1500

ttacaagtta tttcacttga gtccggagat gcaagtattc atgatacagt agaaaatctg 1560

atcatcctag caaacaacag tttgtcttct aatgggaatg taacagaatc tggatgcaaa 1620

gaatgtgagg aactggagga aaaaaatatt aaagaatttt tgcagagttt tgtacatatt 1680

gtccaaatgt tcatcaacac ttct 1704

<210> 80
<211> 1695
<212> DNA
<213> Artificial

<220>
<223> m9G4_IgG4_Heavy Chain_PVGLIG_IPVGLIG_L15

<400> 80
gaagtgatgt tggtagaaag cggtggtggg ctggtgaaac ctggcgggtc cctgaaactc 60

tcctgtactg ctagtggatt cacattcagc acttacgaca tgtcatgggt ccggcagacc 120

ccagagaaga gactggagtg ggtcagcact atctcctctt ctcggaccta tacgtattac 180

agtgccagcg tcaagggacg ctttaccatt agcagggata atgccagaaa catactgtac 240

ctgcagatgt caagcctccg aagtgaggac accgccatgt attactgcgc taggcaattt 300

tcacactggg gccaggggac attggttacc gtgtctgcag cgtccacaaa gggccccagc 360

gtgttccctc tggccccctg cagcagaagc accagcgagt ctacagccgc cctgggatgc 420

ctggtcaagg actacttccc cgagcccgtg accgtgtcct ggaactctgg cgccctgacc 480

agcggcgtgc acacctttcc agccgtgctg cagagcagcg gcctgtacag cctgagcagc 540

gtcgtgaccg tgcctagcag cagcctgggc accaagacct acacctgtaa cgtggaccac 600

aagcccagca acaccaaggt ggacaagcgg gtggaatcta agtacggccc tccctgcccc 660

ccctgcccag cccctgaatt tctgggcgga ccctccgtgt tcctgttccc cccaaagccc 720

aaggacaccc tgatgatcag ccggaccccc gaagtgacct gcgtggtggt ggacgtgtcc 780

caggaagatc ccgaggtcca gttcaactgg tacgtggacg gcgtggaagt gcacaacgcc 840

aagaccaagc ccagagagga acagttcaac agcacctacc gggtggtgtc cgtgctgacc 900

gtgctgcacc aggactggct gaacggcaaa gagtacaagt gcaaggtctc caacaagggc 960

ctgcccagct ccatcgagaa aaccatcagc aaggccaagg gccagccccg cgagccccag 1020

gtgtacacac tgcccccag ccaggaagag atgaccaaga accaggtgtc cctgacctgc 1080

ctcgtgaagg gcttctaccc cagcgatatc gccgtggaat gggagagcaa cggccagccc 1140

gagaacaact acaagaccac cccccctgtg ctggacagcg acggcagctt cttcctgtac 1200

tcccggctga ccgtcgacaa gagccggtgg caggaaggca acgtcttcag ctgcagcgtg 1260

atgcacgagg ccctgcacaa ccactacacc cagaagtccc tgagcctgag cctgggacct 1320

gtcggactca tcggaccggt gggcctgatt ggcaactggg tgaatgtaat aagtgatttg 1380

aaaaaaattg aagatcttat tcaatctatg catattgatg ctactttata tacggaaagt 1440

gatgttcacc ccagttgcaa agtaacagca atgaagtgct ttctcttgga gttacaagtt 1500

atttcacttg agtccggaga tgcaagtatt catgatacag tagaaaatct gatcatccta 1560

gcaaacaaca gtttgtcttc taatgggaat gtaacagaat ctggatgcaa agaatgtgag 1620

gaactggagg aaaaaaatat taaagaattt ttgcagagtt ttgtacatat tgtccaaatg 1680

ttcatcaaca cttct 1695

<210> 81
<211> 1716
<212> DNA
<213> Artificial

<220>
<223> NHS76_IgG1_Heavy Chain_PVGLIG_IPVGLIG_L15

<400> 81
caggtgcagc tgcaggagtc cggcccagga ctggtgaagc cttcggagac cctgtccctc 60

acctgcgctg tctctggtta ctccatcagc agtggttact actggggctg gattcggcag 120

cccccaggga aggggctgga gtggattggg agtatctatc atagtgggag cacctactac 180

aacccgtccc tcaagagtcg agtcaccata tcagtagaca cgtccaagaa ccagttctcc 240

ctgaagctga gctctgtgac cgccgcagac acggccgtgt attactgtgc aagagggaag 300

tggtcgaagt ttgactattg gggccaaggc accctggtca ccgtctcttc agcgtccaca 360

aagggcccaa gcgtgttccc gctagccccc agcagcaaga gcaccagcgg cggcacagcc 420

gccctgggct gcctggtgaa ggactacttc cccgagcccg tgaccgtgtc ctggaacagc 480

ggagccctga cctccggcgt gcacaccttc cccgccgtgc tgcagagcag cggcctgtac 540

agcctgagca gcgtggtgac cgtgcccagc agcagcctgg gcacccagac ctacatctgt 600

aacgtgaacc acaagcccag caacaccaag gtggacaaga gagtggagcc caagagctgt 660

gacaagaccc acacctgtcc cccctgccca gcccccgagc tgctgggcgg acccagcgtg 720

ttcctgttcc cccccaagcc caaggacacc ctgatgatca gcagaacccc cgaggtgacc 780

tgtgtggtgg tggacgtgtc ccacgaggac ccagaggtga agttcaactg gtacgtggac 840

ggcgtggagg tgcacaacgc caagaccaag cccagagagg agcagtacaa cagcacctac 900

agggtggtgt ccgtgctgac cgtgctgcac caggactggc tgaacggcaa ggagtacaag 960

tgtaaggtgt ccaacaaggc cctgccagcc cccatcgaaa agaccatcag caaggccaag 1020

ggccagccaa gagagcccca ggtgtacacc ctgccaccca gcagggagga gatgaccaag 1080

aaccaggtgt ccctgacctg tctggtgaag ggcttctacc caagcgacat cgccgtggag 1140

tgggagagca acggccagcc cgagaacaac tacaagacca cccccccagt gctggacagc 1200

gacggcagct tcttcctgta cagcaagctg accgtggaca agagcagatg gcagcagggc 1260

aacgtgttca gctgctccgt gatgcacgag gccctgcaca accactacac ccagaagagc 1320

ctgagcctgt ccccaggccc tgtcggactc atcggaccgg tgggcctgat tggcaactgg 1380

gtgaatgtaa taagtgattt gaaaaaaatt gaagatctta ttcaatctat gcatattgat 1440

gctactttat atacggaaag tgatgttcac cccagttgca aagtaacagc aatgaagtgc 1500

tttctcttgg agttacaagt tatttcactt gagtccggag atgcaagtat tcatgataca 1560

gtagaaaatc tgatcatcct agcaaacaac agtttgtctt ctaatgggaa tgtaacagaa 1620

tctggatgca aagaatgtga ggaactggag gaaaaaaata ttaaagaatt tttgcagagt 1680

tttgtacata ttgtccaaat gttcatcaac acttct 1716

<210> 82
<211> 1707
<212> DNA
<213> Artificial

<220>
<223> NHS76_IgG4_Heavy Chain_PVGLIG_IPVGLIG_L15

<400> 82
caggtgcagc tgcaggagtc cggcccagga ctggtgaagc cttcggagac cctgtccctc 60

acctgcgctg tctctggtta ctccatcagc agtggttact actggggctg gattcggcag 120

cccccaggga aggggctgga gtggattggg agtatctatc atagtgggag cacctactac 180

aacccgtccc tcaagagtcg agtcaccata tcagtagaca cgtccaagaa ccagttctcc 240

ctgaagctga gctctgtgac cgccgcagac acggccgtgt attactgtgc aagagggaag 300

tggtcgaagt ttgactattg gggccaaggc accctggtca ccgtctcttc agcgtccaca 360

aagggcccca gcgtgttccc tctggccccc tgcagcagaa gcaccagcga gtctacagcc 420

gccctgggat gcctggtcaa ggactacttc cccgagcccg tgaccgtgtc ctggaactct 480

ggcgccctga ccagcggcgt gcacaccttt ccagccgtgc tgcagagcag cggcctgtac 540

agcctgagca gcgtcgtgac cgtgcctagc agcagcctgg gcaccaagac ctacacctgt 600

aacgtggacc acaagcccag caacaccaag gtggacaagc gggtggaatc taagtacggc 660

cctccctgcc ccccctgccc agcccctgaa tttctgggcg gaccctccgt gttcctgttc 720

cccccaaagc ccaaggacac cctgatgatc agccggaccc ccgaagtgac ctgcgtggtg 780

gtggacgtgt cccaggaaga tcccgaggtc cagttcaact ggtacgtgga cggcgtggaa 840

gtgcacaacg ccaagaccaa gcccagagag gaacagttca acagcaccta ccgggtggtg 900

tccgtgctga ccgtgctgca ccaggactgg ctgaacggca aagagtacaa gtgcaaggtc 960

tccaacaagg gcctgcccag ctccatcgag aaaaccatca gcaaggccaa gggccagccc 1020

cgcgagcccc aggtgtacac actgcccccc agccaggaag agatgaccaa gaaccaggtg 1080

tccctgacct gcctcgtgaa gggcttctac cccagcgata tcgccgtgga atgggagagc 1140

aacggccagc ccgagaacaa ctacaagacc accccccctg tgctggacag cgacggcagc 1200

ttcttcctgt actcccggct gaccgtcgac aagagccggt ggcaggaagg caacgtcttc 1260

agctgcagcg tgatgcacga ggccctgcac aaccactaca cccagaagtc cctgagcctg 1320

agcctgggac ctgtcggact catcggaccg gtgggcctga ttggcaactg ggtgaatgta 1380

ataagtgatt tgaaaaaaat tgaagatctt attcaatcta tgcatattga tgctacttta 1440

tatacggaaa gtgatgttca ccccagttgc aaagtaacag caatgaagtg ctttctcttg 1500

gagttacaag ttatttcact tgagtccgga gatgcaagta ttcatgatac agtagaaaat 1560

ctgatcatcc tagcaaacaa cagtttgtct tctaatggga atgtaacaga atctggatgc 1620

aaagaatgtg aggaactgga ggaaaaaaat attaaagaat ttttgcagag ttttgtacat 1680

attgtccaaa tgttcatcaa cacttct 1707

<210>  83
<211>  663
<212>  PRT
<213>  Artificial

<220>
<223>  Ganitumab_IgG1_Heavy Chain_PVGLIG_IL15 [nc30-Sushi]

<400>  83

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Pro Val Gly Leu Ile Gly Asn Trp Val Asn Val Ile Ser Asp Leu Lys
    450                 455                 460

Lys Ile Glu Asp Leu Ile Gln Ser Met His Ile Asp Ala Thr Leu Tyr
465                 470                 475                 480

Thr Glu Ser Asp Val His Pro Ser Cys Lys Val Thr Ala Met Lys Cys
                485                 490                 495

Phe Leu Leu Glu Leu Gln Val Ile Ser Leu Glu Ser Gly Asp Ala Ser
            500                 505                 510

Ile His Asp Thr Val Glu Asn Leu Ile Ile Leu Ala Asn Asn Ser Leu
        515                 520                 525

Ser Ser Asn Gly Asn Val Thr Glu Ser Gly Cys Lys Glu Cys Glu Glu
    530                 535                 540

Leu Glu Glu Lys Asn Ile Lys Glu Phe Leu Gln Ser Phe Val His Ile
545                 550                 555                 560

Val Gln Met Phe Ile Asn Thr Ser Gly Gly Gly Gly Ser Gly Gly Gly
                565                 570                 575

Gly Ser Gly Gly Gly Gly Ser Gly Gly Gly Gly Ser Gly Gly Gly Gly
            580                 585                 590

Ser Gly Gly Gly Ala Ser Ile Thr Cys Pro Pro Pro Met Ser Val Glu
        595                 600                 605

His Ala Asp Ile Trp Val Lys Ser Tyr Ser Leu Tyr Ser Arg Glu Arg
    610                 615                 620

Tyr Ile Cys Asn Ser Gly Phe Lys Arg Lys Ala Gly Thr Ser Ser Leu
625                 630                 635                 640

Thr Glu Cys Val Leu Asn Lys Ala Thr Asn Val Ala His Trp Thr Thr
                645                 650                 655

Pro Ser Leu Lys Cys Ile Arg
            660

<210>  84
<211>  660
<212>  PRT
<213>  Artificial

<220>
<223>  Ganitumab_IgG4_Heavy Chain_PVGLIG_IL15 [nc30-Sushi]

<400>  84

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Pro Val Gly
        435                 440                 445

Leu Ile Gly Asn Trp Val Asn Val Ile Ser Asp Leu Lys Lys Ile Glu
    450                 455                 460

Asp Leu Ile Gln Ser Met His Ile Asp Ala Thr Leu Tyr Thr Glu Ser
465                 470                 475                 480

Asp Val His Pro Ser Cys Lys Val Thr Ala Met Lys Cys Phe Leu Leu
                485                 490                 495

Glu Leu Gln Val Ile Ser Leu Glu Ser Gly Asp Ala Ser Ile His Asp
            500                 505                 510

Thr Val Glu Asn Leu Ile Ile Leu Ala Asn Asn Ser Leu Ser Ser Asn
        515                 520                 525

Gly Asn Val Thr Glu Ser Gly Cys Lys Glu Cys Glu Glu Leu Glu Glu
    530                 535                 540

Lys Asn Ile Lys Glu Phe Leu Gln Ser Phe Val His Ile Val Gln Met
545                 550                 555                 560

Phe Ile Asn Thr Ser Gly Gly Gly Gly Ser Gly Gly Gly Gly Ser Gly
                565                 570                 575

Gly Gly Gly Ser Gly Gly Gly Gly Ser Gly Gly Gly Gly Ser Gly Gly
            580                 585                 590

Gly Ala Ser Ile Thr Cys Pro Pro Pro Met Ser Val Glu His Ala Asp
        595                 600                 605

Ile Trp Val Lys Ser Tyr Ser Leu Tyr Ser Arg Glu Arg Tyr Ile Cys
    610                 615                 620

Asn Ser Gly Phe Lys Arg Lys Ala Gly Thr Ser Ser Leu Thr Glu Cys
625                 630                 635                 640

Val Leu Asn Lys Ala Thr Asn Val Ala His Trp Thr Thr Pro Ser Leu
                645                 650                 655

Lys Cys Ile Arg
            660

<210> 85
<211> 675
<212> PRT
<213> Artificial

<220>
<223> Ganitumab_IgG1_Heavy Chain_PVGLIG_IL15 [nc30-Sushi+]

<400> 85
   Gln Val Gln Leu Gln Glu Ser Gly Pro Gly Leu Val Lys Pro Ser Gly
   1 5 10 15
   Thr Leu Ser Leu Thr Cys Ala Val Ser Gly Gly Ser Ile Ser Ser Ser
   20 25 30
   Asn Trp Trp Ser Trp Val Arg Gln Pro Pro Gly Lys Gly Leu Glu Trp
   35 40 45
   Ile Gly Glu Ile Tyr His Ser Gly Ser Thr Asn Tyr Asn Pro Ser Leu
   50 55 60
   Lys Ser Arg Val Thr Ile Ser Val Asp Lys Ser Lys Asn Gln Phe Ser
   65 70 75 80
   Leu Lys Leu Ser Ser Val Thr Ala Ala Asp Thr Ala Val Tyr Tyr Cys
   85 90 95
   Ala Arg Trp Thr Gly Arg Thr Asp Ala Phe Asp Ile Trp Gly Gln Gly
   100 105 110
   Thr Met Val Thr Val Ser Ser Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Arg Val Glu Pro Lys Ser Cys Asp Lys
   210 215 220
   Thr His Thr Cys Pro Pro Cys Pro Ala Pro Glu Leu Leu Gly Gly Pro
   225 230 235 240
   Ser Val Phe Leu Phe Pro Pro Lys Pro Lys Asp Thr Leu Met Ile Ser
   245 250 255
   Arg Thr Pro Glu Val Thr Cys Val Val Val Asp Val Ser His Glu Asp
   260 265 270
   Pro Glu Val Lys Phe Asn Trp Tyr Val Asp Gly Val Glu Val His Asn
   275 280 285
   Ala Lys Thr Lys Pro Arg Glu Glu Gln Tyr Asn Ser Thr Tyr Arg Val
   290 295 300
   Val Ser Val Leu Thr Val Leu His Gln Asp Trp Leu Asn Gly Lys Glu
   305 310 315 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Pro Val Gly Leu Ile Gly Asn Trp Val Asn Val Ile Ser Asp Leu Lys
       450                 455                 460
   Lys Ile Glu Asp Leu Ile Gln Ser Met His Ile Asp Ala Thr Leu Tyr
   465                 470                 475                 480
   Thr Glu Ser Asp Val His Pro Ser Cys Lys Val Thr Ala Met Lys Cys
                   485                 490                 495
   Phe Leu Leu Glu Leu Gln Val Ile Ser Leu Glu Ser Gly Asp Ala Ser
               500                 505                 510
   Ile His Asp Thr Val Glu Asn Leu Ile Ile Leu Ala Asn Asn Ser Leu
           515                 520                 525
   Ser Ser Asn Gly Asn Val Thr Glu Ser Gly Cys Lys Glu Cys Glu Glu
       530                 535                 540
   Leu Glu Glu Lys Asn Ile Lys Glu Phe Leu Gln Ser Phe Val His Ile
   545                 550                 555                 560
   Val Gln Met Phe Ile Asn Thr Ser Gly Gly Gly Gly Ser Gly Gly Gly
                   565                 570                 575
   Gly Ser Gly Gly Gly Gly Ser Gly Gly Gly Gly Ser Gly Gly Gly Gly
               580                 585                 590
   Ser Gly Gly Gly Ala Ser Ile Thr Cys Pro Pro Pro Met Ser Val Glu
           595                 600                 605
   His Ala Asp Ile Trp Val Lys Ser Tyr Ser Leu Tyr Ser Arg Glu Arg
       610                 615                 620
   Tyr Ile Cys Asn Ser Gly Phe Lys Arg Lys Ala Gly Thr Ser Ser Leu
   625                 630                 635                 640
   Thr Glu Cys Val Leu Asn Lys Ala Thr Asn Val Ala His Trp Thr Thr
                   645                 650                 655
   Pro Ser Leu Lys Cys Ile Arg Asp Pro Ala Leu Val His Gln Arg Pro
               660                 665                 670
   Ala Pro Pro
           675

<210>  86
<211>  672
<212>  PRT
<213>  Artificial

<220>
<223>  Ganitumab_IgG4_Heavy Chain_PVGLIG_IL15 [nc30-Sushi+]

<400>  86

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Pro Val Gly
        435                 440                 445

Leu Ile Gly Asn Trp Val Asn Val Ile Ser Asp Leu Lys Lys Ile Glu
    450                 455                 460

Asp Leu Ile Gln Ser Met His Ile Asp Ala Thr Leu Tyr Thr Glu Ser
465                 470                 475                 480

Asp Val His Pro Ser Cys Lys Val Thr Ala Met Lys Cys Phe Leu Leu
                485                 490                 495

Glu Leu Gln Val Ile Ser Leu Glu Ser Gly Asp Ala Ser Ile His Asp
            500                 505                 510

Thr Val Glu Asn Leu Ile Ile Leu Ala Asn Asn Ser Leu Ser Ser Asn
        515                 520                 525

Gly Asn Val Thr Glu Ser Gly Cys Lys Glu Cys Glu Glu Leu Glu Glu
    530                 535                 540

Lys Asn Ile Lys Glu Phe Leu Gln Ser Phe Val His Ile Val Gln Met
545                 550                 555                 560

Phe Ile Asn Thr Ser Gly Gly Gly Gly Ser Gly Gly Gly Gly Ser Gly
                565                 570                 575

Gly Gly Gly Ser Gly Gly Gly Gly Ser Gly Gly Gly Gly Ser Gly Gly
            580                 585                 590

Gly Ala Ser Ile Thr Cys Pro Pro Pro Met Ser Val Glu His Ala Asp
        595                 600                 605

Ile Trp Val Lys Ser Tyr Ser Leu Tyr Ser Arg Glu Arg Tyr Ile Cys
    610                 615                 620

Asn Ser Gly Phe Lys Arg Lys Ala Gly Thr Ser Ser Leu Thr Glu Cys
625                 630                 635                 640

Val Leu Asn Lys Ala Thr Asn Val Ala His Trp Thr Thr Pro Ser Leu
                645                 650                 655

Lys Cys Ile Arg Asp Pro Ala Leu Val His Gln Arg Pro Ala Pro Pro
            660                 665                 670

<210>  87
<211>  1989
<212>  DNA
<213>  Artificial

<220>
<223>  Ganitumab_IgG1_Heavy Chain_PVGLIG_IL15 [nc30-Sushi]

<400>  87
caggtgcagc tgcaggagtc gggaccagga ctggtgaagc cttcggggac cctgtccctc        60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggcccggtg ggcctgattg gcaactgggt gaatgtaata 1380

agtgatttga aaaaaattga agatcttatt caatctatgc atattgatgc tactttatat 1440

acggaaagtg atgttcaccc cagttgcaaa gtaacagcaa tgaagtgctt tctcttggag 1500

ttacaagtta tttcacttga gtccggagat gcaagtattc atgatacagt agaaaatctg 1560

atcatcctag caaacaacag tttgtcttct aatgggaatg taacagaatc tggatgcaaa 1620

gaatgtgagg aactggagga aaaaaatatt aaagaatttt tgcagagttt tgtacatatt 1680

gtccaaatgt tcatcaacac ttctggaggc ggaggatctg gtggtggtgg atctggcggc 1740

ggaggaagtg gcggaggcgg aagcggtggt ggcggttcag gtggcggagc tagcatcacc 1800

tgtcctccac ctatgagcgt ggaacacgcc gacatctggg tcaagagcta cagcctgtac 1860

agcagagagc ggtacatctg caacagcggc ttcaagagaa aggccggcac aagcagcctg 1920

accgagtgtg tgctgaacaa ggccacaaac gtggcccact ggaccacacc tagcctgaag 1980

tgcatcaga 1989

<210> 88
<211> 1980
<212> DNA
<213> Artificial

<220>
<223> Ganitumab_IgG4_Heavy Chain_PVGLIG_IL15 [nc30-Sushi]

<400> 88
   caggtgcagc tgcaggagtc gggaccagga ctggtgaagc cttcggggac cctgtccctc 60
   acctgcgctg tctctggtgg ctccatcagc agtagtaact ggtggagttg ggtccgccag 120
   cccccaggga aggggctgga gtggattggg gaaatctatc atagtgggag caccaactac 180
   aacccgtccc tcaagagtcg agtcaccata tcagtagaca agtccaagaa ccagttctcc 240
   ctgaagctga gctctgtgac cgccgcggac acggccgtgt attactgtgc gagatggacc 300
   gggcgtactg atgcttttga tatctggggc caagggacaa tggtcaccgt ctcaagcgcg 360
   tccacaaagg gccccagcgt gttccctctg gccccctgca gcagaagcac cagcgagtct 420
   acagccgccc tgggatgcct ggtcaaggac tacttccccg agcccgtgac cgtgtcctgg 480
   aactctggcg ccctgaccag cggcgtgcac acctttccag ccgtgctgca gagcagcggc 540
   ctgtacagcc tgagcagcgt cgtgaccgtg cctagcagca gcctgggcac caagacctac 600
   acctgtaacg tggaccacaa gcccagcaac accaaggtgg acaagcgggt ggaatctaag 660
   tacggccctc cctgcccccc ctgcccagcc cctgaatttc tgggcggacc ctccgtgttc 720
   ctgttccccc caaagcccaa ggacaccctg atgatcagcc ggacccccga agtgacctgc 780
   gtggtggtgg acgtgtccca ggaagatccc gaggtccagt tcaactggta cgtggacggc 840
   gtggaagtgc acaacgccaa gaccaagccc agagaggaac agttcaacag cacctaccgg 900
   gtggtgtccg tgctgaccgt gctgcaccag gactggctga acggcaaaga gtacaagtgc 960
   aaggtctcca acaagggcct gcccagctcc atcgagaaaa ccatcagcaa ggccaagggc 1020
   cagccccgcg agccccaggt gtacacactg ccccccagcc aggaagagat gaccaagaac 1080
   caggtgtccc tgacctgcct cgtgaagggc ttctacccca gcgatatcgc cgtggaatgg 1140
   gagagcaacg gccagcccga gaacaactac aagaccaccc cccctgtgct ggacagcgac 1200
   ggcagcttct tcctgtactc ccggctgacc gtcgacaaga gccggtggca ggaaggcaac 1260
   gtcttcagct gcagcgtgat gcacgaggcc ctgcacaacc actacaccca gaagtccctg 1320
   agcctgagcc tgggaccggt gggcctgatt ggcaactggg tgaatgtaat aagtgatttg 1380
   aaaaaaattg aagatcttat tcaatctatg catattgatg ctactttata tacggaaagt 1440
   gatgttcacc ccagttgcaa agtaacagca atgaagtgct ttctcttgga gttacaagtt 1500
   atttcacttg agtccggaga tgcaagtatt catgatacag tagaaaatct gatcatccta 1560
   gcaaacaaca gtttgtcttc taatgggaat gtaacagaat ctggatgcaa agaatgtgag 1620
   gaactggagg aaaaaaatat taaagaattt ttgcagagtt ttgtacatat tgtccaaatg 1680
   ttcatcaaca cttctggagg cggaggatct ggtggtggtg gatctggcgg cggaggaagt 1740
   ggcggaggcg gaagcggtgg tggcggttca ggtggcggag ctagcatcac ctgtcctcca 1800
   cctatgagcg tggaacacgc cgacatctgg gtcaagagct acagcctgta cagcagagag 1860
   cggtacatct gcaacagcgg cttcaagaga aaggccggca caagcagcct gaccgagtgt 1920
   gtgctgaaca aggccacaaa cgtggcccac tggaccacac ctagcctgaa gtgcatcaga 1980

<210> 89
<211> 2025
<212> DNA
<213> Artificial

<220>
<223> Ganitumab_IgG1_Heavy Chain_PVGLIG_IL15 [nc30-Sushi+]

<400> 89
caggtgcagc tgcaggagtc gggaccagga ctggtgaagc cttcggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540
ctgtacagcc tgagcagcgt ggtgaccgtg cccagcagca gcctgggcac ccagacctac 600
atctgtaacg tgaaccacaa gcccagcaac accaaggtgg acaagagagt ggagcccaag 660
agctgtgaca agacccacac ctgtcccccc tgcccagccc ccgagctgct gggcggaccc 720
agcgtgttcc tgttcccccc caagcccaag gacaccctga tgatcagcag aacccccgag 780
gtgacctgtg tggtggtgga cgtgtcccac gaggacccag aggtgaagtt caactggtac 840
gtggacggcg tggaggtgca caacgccaag accaagccca gagaggagca gtacaacagc 900
acctacaggg tggtgtccgt gctgaccgtg ctgcaccagg actggctgaa cggcaaggag 960
tacaagtgta aggtgtccaa caaggccctg ccagccccaa tcgaaaagac catcagcaag 1020
gccaagggcc agccaagaga gccccaggtg tacaccctgc cacccagcag ggaggagatg 1080
accaagaacc aggtgtccct gacctgtctg gtgaagggct tctacccaag cgacatcgcc 1140
gtggagtggg agagcaacgg ccagcccgag aacaactaca agaccacccc cccagtgctg 1200
gacagcgacg gcagcttctt cctgtacagc aagctgaccg tggacaagag cagatggcag 1260
cagggcaacg tgttcagctg ctccgtgatg cacgaggccc tgcacaacca ctacacccag 1320
aagagcctga gcctgtcccc aggcccggtg ggcctgattg gcaactgggt gaatgtaata 1380
agtgatttga aaaaaattga agatcttatt caatctatgc atattgatgc tactttatat 1440
acggaaagtg atgttcaccc cagttgcaaa gtaacagcaa tgaagtgctt tctcttggag 1500
ttacaagtta tttcacttga gtccggagat gcaagtattc atgatacagt agaaaatctg 1560
atcatcctag caaacaacag tttgtcttct aatgggaatg taacagaatc tggatgcaaa 1620
gaatgtgagg aactggagga aaaaaatatt aaagaatttt tgcagagttt tgtacatatt 1680
gtccaaatgt tcatcaacac ttctggaggc ggaggatctg gtggtggtgg atctggcggc 1740
ggaggaagtg gcggaggcgg aagcggtggt ggcggttcag gtggcggagc tagcatcacc 1800
tgtcctccac ctatgagcgt ggaacacgcc gacatctggg tcaagagcta cagcctgtac 1860
agcagagagc ggtacatctg caacagcggc ttcaagagaa aggccggcac aagcagcctg 1920
accgagtgtg tgctgaacaa ggccacaaac gtggcccact ggaccacacc tagcctgaag 1980
tgcatcagag accccgccct ggtgcaccag cggcccgccc ccccc 2025

<210> 90
<211> 2016

<212>  DNA
<213>  Artificial

<220>
<223>  Ganitumab_IgG4_Heavy Chain_PVGLIG_IL15 [nc30-Sushi+]

<400>  90
caggtgcagc tgcaggagtc gggaccagga ctggtgaagc cttcggggac cctgtccctc      60

acctgcgctg tctctggtgg ctccatcagc agtagtaact ggtggagttg ggtccgccag     120

cccccaggga aggggctgga gtggattggg gaaatctatc atagtgggag caccaactac     180

aacccgtccc tcaagagtcg agtcaccata tcagtagaca agtccaagaa ccagttctcc     240

ctgaagctga gctctgtgac cgccgcggac acggccgtgt attactgtgc gagatggacc     300

gggcgtactg atgcttttga tatctggggc caagggacaa tggtcaccgt ctcaagcgcg     360

tccacaaagg gccccagcgt gttccctctg gccccctgca gcagaagcac cagcgagtct     420

acagccgccc tgggatgcct ggtcaaggac tacttccccg agcccgtgac cgtgtcctgg     480

aactctggcg ccctgaccag cggcgtgcac acctttccag ccgtgctgca gagcagcggc     540

ctgtacagcc tgagcagcgt cgtgaccgtg cctagcagca gcctgggcac caagacctac     600

acctgtaacg tggaccacaa gcccagcaac accaaggtgg acaagcgggt ggaatctaag     660

tacggccctc cctgcccccc ctgcccagcc cctgaatttc tgggcggacc ctccgtgttc     720

ctgttccccc caaagcccaa ggacaccctg atgatcagcc ggacccccga agtgacctgc     780

gtggtggtgg acgtgtccca ggaagatccc gaggtccagt tcaactggta cgtggacggc     840

gtggaagtgc acaacgccaa gaccaagccc agagaggaac agttcaacag cacctaccgg     900

gtggtgtccg tgctgaccgt gctgcaccag gactggctga acggcaaaga gtacaagtgc     960

aaggtctcca acaagggcct gcccagctcc atcgagaaaa ccatcagcaa ggccaagggc    1020

cagccccgcg agccccaggt gtacacactg cccccagcc aggaagagat gaccaagaac    1080

caggtgtccc tgacctgcct cgtgaagggc ttctacccca gcgatatcgc cgtggaatgg    1140

gagagcaacg gccagcccga gaacaactac aagaccaccc cccctgtgct ggacagcgac    1200

ggcagcttct tcctgtactc ccggctgacc gtcgacaaga gccggtggca ggaaggcaac    1260

gtcttcagct gcagcgtgat gcacgaggcc ctgcacaacc actacaccca gaagtccctg    1320

agcctgagcc tgggaccggt gggcctgatt ggcaactggg tgaatgtaat aagtgatttg    1380

aaaaaaattg aagatcttat tcaatctatg catattgatg ctactttata tacggaaagt    1440

gatgttcacc ccagttgcaa agtaacagca atgaagtgct ttctcttgga gttacaagtt    1500

atttcacttg agtccggaga tgcaagtatt catgatacag tagaaaatct gatcatccta 1560

gcaaacaaca gtttgtcttc taatgggaat gtaacagaat ctggatgcaa agaatgtgag 1620

gaactggagg aaaaaaatat taaagaattt ttgcagagtt ttgtacatat tgtccaaatg 1680

ttcatcaaca cttctggagg cggaggatct ggtggtggtg gatctggcgg cggaggaagt 1740

ggcggaggcg gaagcggtgg tggcggttca ggtggcggag ctagcatcac ctgtcctcca 1800

cctatgagcg tggaacacgc cgacatctgg gtcaagagct acagcctgta cagcagagag 1860

cggtacatct gcaacagcgg cttcaagaga aaggccggca caagcagcct gaccgagtgt 1920

gtgctgaaca aggccacaaa cgtggcccac tggaccacac ctagcctgaa gtgcatcaga 1980

gaccccgccc tggtgcacca gcggcccgcc cccccc 2016

<210> 91
<211> 669
<212> PRT
<213> Artificial

<220>
<223> Ganitumab_IgG1_Heavy Chain_PVGLIG_PVGLIG_IL15 [nc30-Sushi]

<400> 91

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Pro Val Gly Leu Ile Gly Pro Val Gly Leu Ile Gly Asn Trp Val Asn
    450                 455                 460

Val Ile Ser Asp Leu Lys Lys Ile Glu Asp Leu Ile Gln Ser Met His
465                 470                 475                 480

Ile Asp Ala Thr Leu Tyr Thr Glu Ser Asp Val His Pro Ser Cys Lys
                485                 490                 495

Val Thr Ala Met Lys Cys Phe Leu Leu Glu Leu Gln Val Ile Ser Leu
            500                 505                 510

Glu Ser Gly Asp Ala Ser Ile His Asp Thr Val Glu Asn Leu Ile Ile
        515                 520                 525

Leu Ala Asn Asn Ser Leu Ser Ser Asn Gly Asn Val Thr Glu Ser Gly
    530                 535                 540

Cys Lys Glu Cys Glu Glu Leu Glu Glu Lys Asn Ile Lys Glu Phe Leu
545                 550                 555                 560

Gln Ser Phe Val His Ile Val Gln Met Phe Ile Asn Thr Ser Gly Gly
                565                 570                 575

Gly Gly Ser Gly Gly Gly Gly Ser Gly Gly Gly Gly Ser Gly Gly Gly
            580                 585                 590

Gly Ser Gly Gly Gly Gly Ser Gly Gly Gly Ala Ser Ile Thr Cys Pro
        595                 600                 605

Pro Pro Met Ser Val Glu His Ala Asp Ile Trp Val Lys Ser Tyr Ser
    610                 615                 620

Leu Tyr Ser Arg Glu Arg Tyr Ile Cys Asn Ser Gly Phe Lys Arg Lys
625                 630                 635                 640

Ala Gly Thr Ser Ser Leu Thr Glu Cys Val Leu Asn Lys Ala Thr Asn
                645                 650                 655

Val Ala His Trp Thr Thr Pro Ser Leu Lys Cys Ile Arg
            660                 665

<210>  92
<211>  681
<212>  PRT
<213>  Artificial

<220>
<223>  Ganitumab_IgG1_Heavy Chain_PVGLIG_PVGLIG_IL15 [nc30-Sushi+]

<400>  92

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Ser
            20                  25                  30

Asn Trp Trp Ser Trp Val Arg Gln Pro Pro Gly Lys Gly Leu Glu Trp
        35                  40                  45

Ile Gly Glu Ile Tyr His Ser Gly Ser Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Trp Thr Gly Arg Thr Asp Ala Phe Asp Ile Trp Gly Gln Gly
            100                 105                 110

Thr Met Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Pro Val Gly Leu Ile Gly Pro Val Gly Leu Ile Gly Asn Trp Val Asn
    450                 455                 460

Val Ile Ser Asp Leu Lys Lys Ile Glu Asp Leu Ile Gln Ser Met His
465                 470                 475                 480

Ile Asp Ala Thr Leu Tyr Thr Glu Ser Asp Val His Pro Ser Cys Lys
                485                 490                 495

Val Thr Ala Met Lys Cys Phe Leu Leu Glu Leu Gln Val Ile Ser Leu
            500                 505                 510

Glu Ser Gly Asp Ala Ser Ile His Asp Thr Val Glu Asn Leu Ile Ile
        515                 520                 525

Leu Ala Asn Asn Ser Leu Ser Ser Asn Gly Asn Val Thr Glu Ser Gly
    530                 535                 540

Cys Lys Glu Cys Glu Glu Leu Glu Glu Lys Asn Ile Lys Glu Phe Leu
545                 550                 555                 560

Gln Ser Phe Val His Ile Val Gln Met Phe Ile Asn Thr Ser Gly Gly
                565                 570                 575

Gly Gly Ser Gly Gly Gly Gly Ser Gly Gly Gly Gly Ser Gly Gly Gly
            580                 585                 590

Gly Ser Gly Gly Gly Gly Ser Gly Gly Gly Ala Ser Ile Thr Cys Pro
        595                 600                 605

Pro Pro Met Ser Val Glu His Ala Asp Ile Trp Val Lys Ser Tyr Ser
    610                 615                 620

Leu Tyr Ser Arg Glu Arg Tyr Ile Cys Asn Ser Gly Phe Lys Arg Lys
625                 630                 635                 640

Ala Gly Thr Ser Ser Leu Thr Glu Cys Val Leu Asn Lys Ala Thr Asn
                645                 650                 655

Val Ala His Trp Thr Thr Pro Ser Leu Lys Cys Ile Arg Asp Pro Ala
            660                 665                 670

Leu Val His Gln Arg Pro Ala Pro Pro
        675                 680

<210>  93
<211>  2007
<212>  DNA
<213>  Artificial

<220>
<223>  Ganitumab_IgG1_Heavy Chain_PVGLIG_PVGLIG_IL15 [nc30-Sushi]

<400>  93
caggtgcagc tgcaggagtc gggaccagga ctggtgaagc cttcggggac cctgtccctc      60

acctgcgctg tctctggtgg ctccatcagc agtagtaact ggtggagttg ggtccgccag     120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggccctgtc ggactcatcg gaccggtggg cctgattggc 1380

aactgggtga atgtaataag tgatttgaaa aaaattgaag atcttattca atctatgcat 1440

attgatgcta ctttatatac ggaaagtgat gttcacccca gttgcaaagt aacagcaatg 1500

aagtgctttc tcttggagtt acaagttatt tcacttgagt ccggagatgc aagtattcat 1560

gatacagtag aaaatctgat catcctagca aacaacagtt tgtcttctaa tgggaatgta 1620

acagaatctg gatgcaaaga atgtgaggaa ctggaggaaa aaaatattaa agaatttttg 1680

cagagttttg tacatattgt ccaaatgttc atcaacactt ctggaggcgg aggatctggt 1740

ggtggtggat ctggcggcgg aggaagtggc ggaggcggaa gcggtggtgg cggttcaggt 1800

ggcggagcta gcatcacctg tcctccacct atgagcgtgg aacacgccga catctgggtc 1860

aagagctaca gcctgtacag cagagagcgg tacatctgca acagcggctt caagagaaag 1920

gccggcacaa gcagcctgac cgagtgtgtg ctgaacaagg ccacaaacgt ggcccactgg 1980

accacaccta gcctgaagtg catcaga 2007

<210> 94
<211> 2043
<212> DNA
<213> Artificial

<220>
<223> Ganitumab_IgG1_Heavy Chain_PVGLIG_PVGLIG_IL15 [nc30-Sushi+]

<400> 94
caggtgcagc tgcaggagtc gggaccagga ctggtgaagc cttcggggac cctgtccctc 60

acctgcgctg tctctggtgg ctccatcagc agtagtaact ggtggagttg ggtccgccag 120

cccccaggga aggggctgga gtggattggg gaaatctatc atagtgggag caccaactac 180

aacccgtccc tcaagagtcg agtcaccata tcagtagaca agtccaagaa ccagttctcc 240

ctgaagctga gctctgtgac cgccgcggac acggccgtgt attactgtgc gagatggacc 300

gggcgtactg atgcttttga tatctggggc caagggacaa tggtcaccgt ctcaagcgcg 360

tccacaaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgtcccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac 840

gtggacggcg tggaggtgca caacgccaag accaagccca gagaggagca gtacaacagc 900

acctacaggg tggtgtccgt gctgaccgtg ctgcaccagg actggctgaa cggcaaggag 960

tacaagtgta aggtgtccaa caaggccctg ccagccccaa tcgaaaagac catcagcaag 1020

gccaagggcc agccaagaga gccccaggtg tacaccctgc cacccagcag ggaggagatg 1080

accaagaacc aggtgtccct gacctgtctg gtgaagggct tctacccaag cgacatcgcc 1140

gtggagtggg agagcaacgg ccagcccgag aacaactaca agaccacccc cccagtgctg 1200

gacagcgacg gcagcttctt cctgtacagc aagctgaccg tggacaagag cagatggcag 1260

cagggcaacg tgttcagctg ctccgtgatg cacgaggccc tgcacaacca ctacacccag 1320

aagagcctga gcctgtcccc aggccctgtc ggactcatcg gaccggtggg cctgattggc 1380

aactgggtga atgtaataag tgatttgaaa aaaattgaag atcttattca atctatgcat 1440

attgatgcta ctttatatac ggaaagtgat gttcacccca gttgcaaagt aacagcaatg 1500

aagtgctttc tcttggagtt acaagttatt tcacttgagt ccggagatgc aagtattcat 1560

gatacagtag aaaatctgat catcctagca aacaacagtt tgtcttctaa tgggaatgta 1620

acagaatctg gatgcaaaga atgtgaggaa ctggaggaaa aaaatattaa agaatttttg 1680

cagagttttg tacatattgt ccaaatgttc atcaacactt ctggaggcgg aggatctggt 1740

ggtggtggat ctggcggcgg aggaagtggc ggaggcggaa gcggtggtgg cggttcaggt 1800

ggcggagcta gcatcacctg tcctccacct atgagcgtgg aacacgccga catctgggtc 1860

aagagctaca gcctgtacag cagagagcgg tacatctgca acagcggctt caagagaaag 1920

gccggcacaa gcagcctgac cgagtgtgtg ctgaacaagg ccacaaacgt ggcccactgg 1980

accacaccta gcctgaagtg catcagagac cccgccctgg tgcaccagcg gcccgccccc 2040

ccc 2043

## Description

### INTRODUCTION

The invention relates to a new protein complex comprising an immunocytokine and a cofactor, and its use for treating cancer.

While therapeutic success has been achieved for various types of haematological malignancies and some solid tumours (*e.g*., metastatic testicular cancer), the majority of disseminated forms of solid cancer remain incurable. The therapeutic efficacy of conventional cancer therapeutics is often limited by the inability of small organic molecules to accumulate in sufficient amounts at the site of disease (see e.g., van der Veldt AA, et at. Clin Cancer Res. 19: 4163-4173, 2013).

New strategies are now developed that preferentially activate relevant immune subsets, such as T effectors, monocytes and NK cells, while limiting the activation of regulatory T cells. However, substantial side effects and unfavourable pharmacokinetic properties have been a major drawback hampering the administration of therapeutically relevant doses. Notably, cytokine immunotherapy often results in the development of severe dose-limiting side effects (Pachella et al., Pract Oncol 6:212-221, 2015). Two properties shared by most cytokines are thought to play a crucial role in the development of treatment-associated adverse effects. Firstly, cytokines are pleiotropic, meaning they are able to influence more than a single cell type. Furthermore, cytokines have a short serum half-life and, thus, need to be administered at high doses to achieve their therapeutic effects. While effectively enhancing therapeutic efficacy, high doses exacerbate pleiotropic activities that manifest as adverse effects in patients.

One approach aimed at increasing efficacy attempts to deliver cytokines to tumour sites by genetically fusing cytokines to antibodies, or antibody components such as a single chain variable fragment (scFv). Such fusion proteins, designated immunocytokines, combine the binding specificity of an antibody with the potency of cytokines such as, for example, IL-2 (Sondel & Gillies, Antibodies 1: 149-171, 2012; Skrombolas & Frelinger, Expert Rev Clin Immunol. 10(2): 207-217, 2014; Kiefer & Neri, Immunol Rev. 270(1): 178-192, 2016). Delivery of the cytokine to the tumour site is improved by the use of immunocytokines, notably for cancers with easily accessible tumours. One such molecule currently tested clinically is N-803 (previously known as ALT-803), an IL-15 superagonist mutant complexed to a dimeric IL-15RaSushi-Fc fusion protein (Guo et al., Cytokine Growth Factor Rev. 38: 10-21, 2017). In another instance, the immunocytokine comprises a cytokine (IL-12) joined to a specific inhibitory anti-IL-12 scFv by a MMP9-cleavage site (Skrombolas et al., J Interferon Cytokine Res. 39(4): 233-245, 2019) However, the treatment of disseminated, systemic diseases might benefit from immunocytokines that have been optimised for tumour targeting and activation at the tumour site (Sondel & Gillies, Antibodies 1: 149-171, 2012). In particular, binding of the antibody outside of the tumour may result in unwanted cytokine activity and potential side effects. This problem is all the more crucial as certain payloads have been reported to completely abrogate the tumour-targeting potential of the parental antibody in mouse models of cancer (see e.g., Hess, Doctoral Thesis, ETH Zürich, 2015).

Thus, there is still a need for an immunocytokine which can deliver and activate the cytokine safely and efficiently to the tumour site.

### FIGURE LEGENDS

### Figure 1. Protein complexes used.

***Figure 2******: List of the protein complexes used.*** For each molecule are indicated its code, its name, and each of its components. The mode of interaction (covalent or coexpression) between the cofactor and the immunocytokine is also mentioned.
***Figure 3******: Productivity and monomer levels of the molecules produced in HEK293 cells.*** The bar chart represents productivity of the cells expressing the different molecules (colour scale from light to dark: low to high productivity). These molecules have been characterised by SEC analysis and the monomer rate of the molecule is reported on the graph (cross); the values obtained for K03201-077 and K03201-079 were 84% and 87%, respectively. In the event that the monomer level is lower than 80% molecules were submitted to a supplementary round of purification.

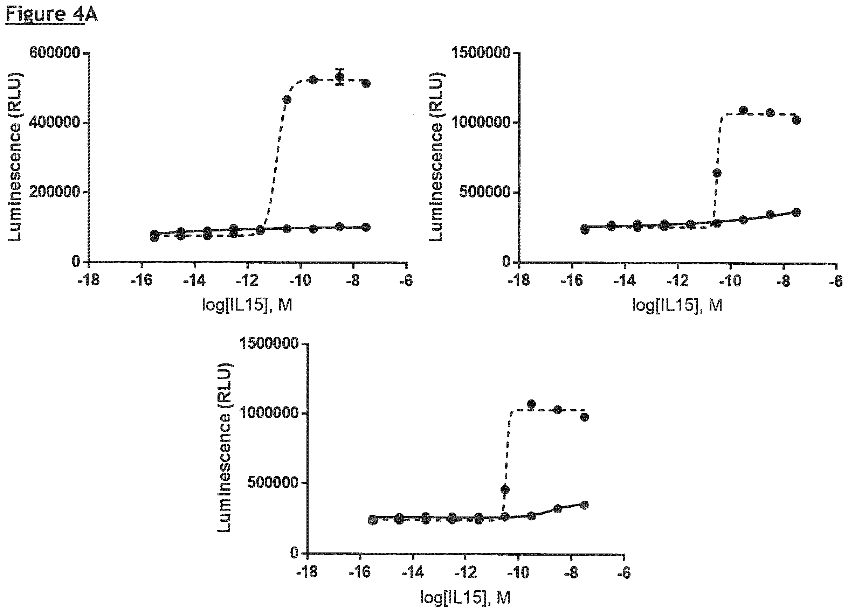

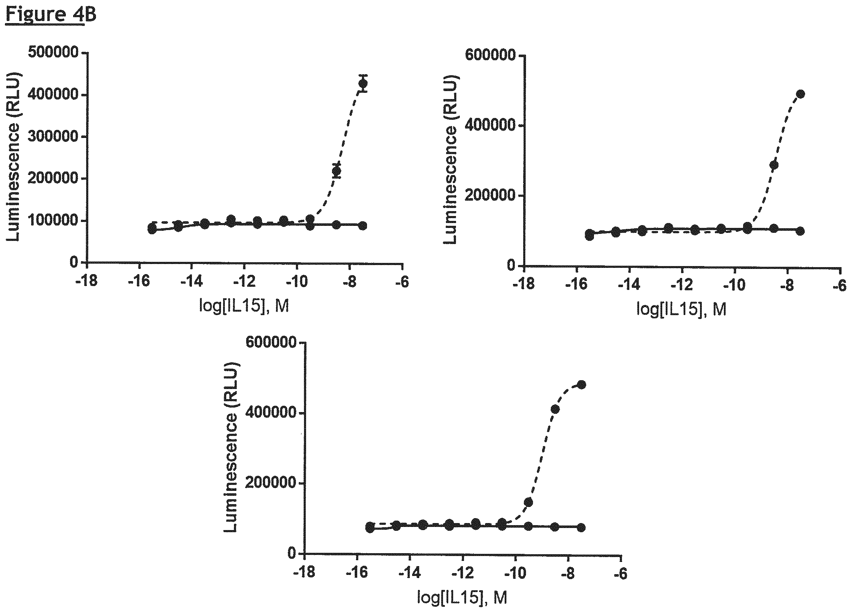

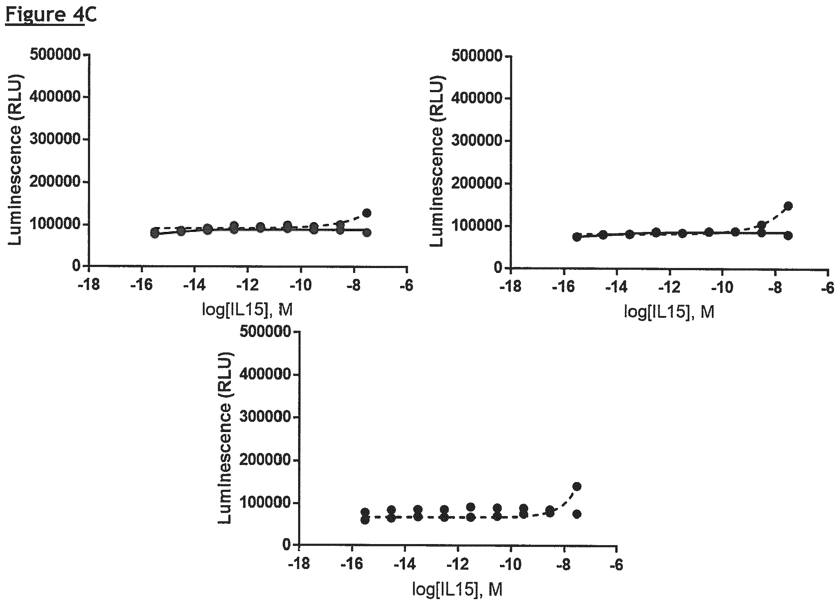

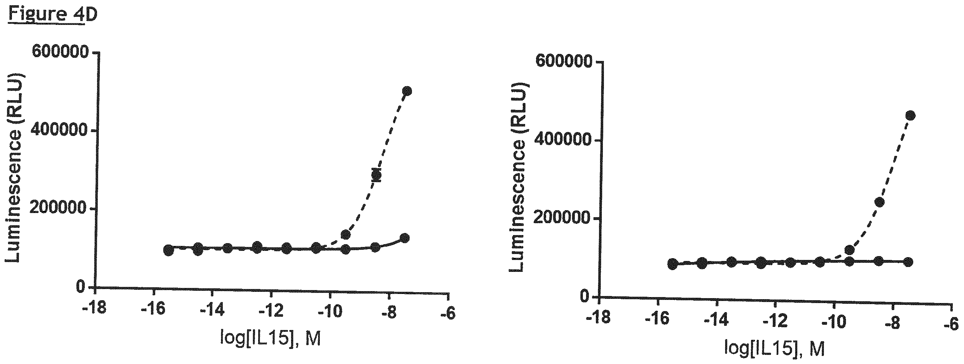

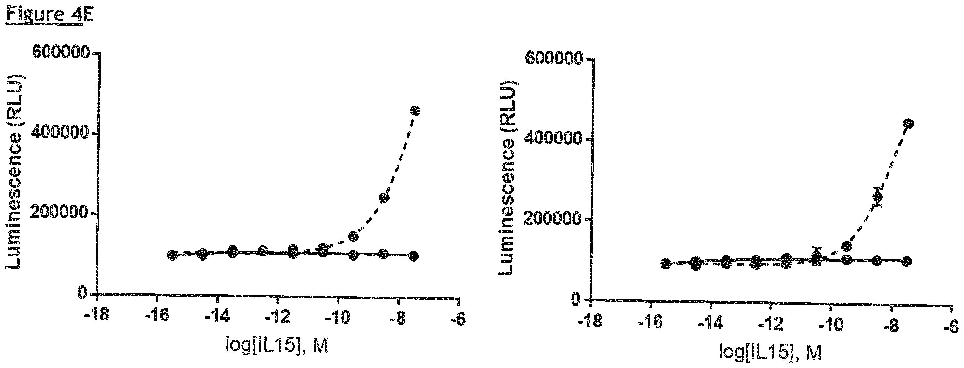

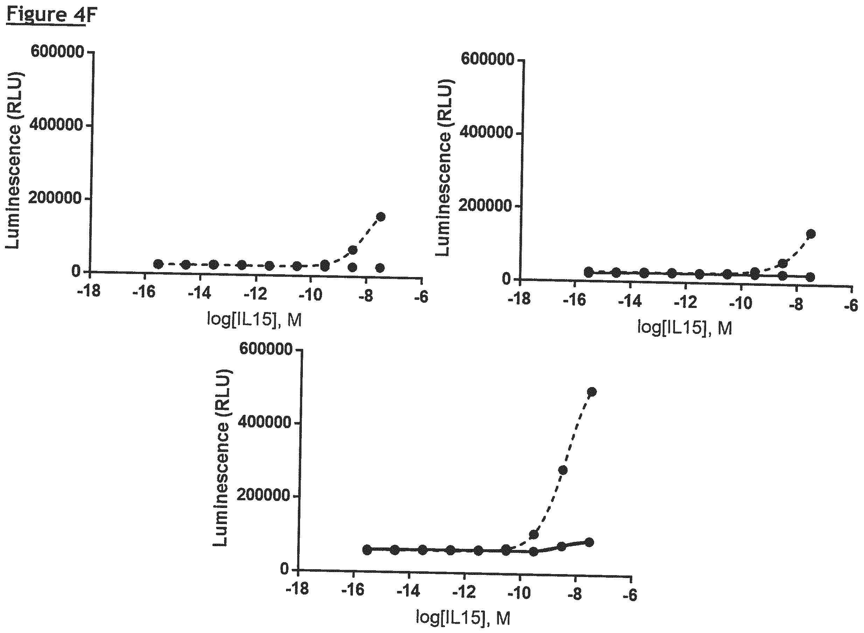

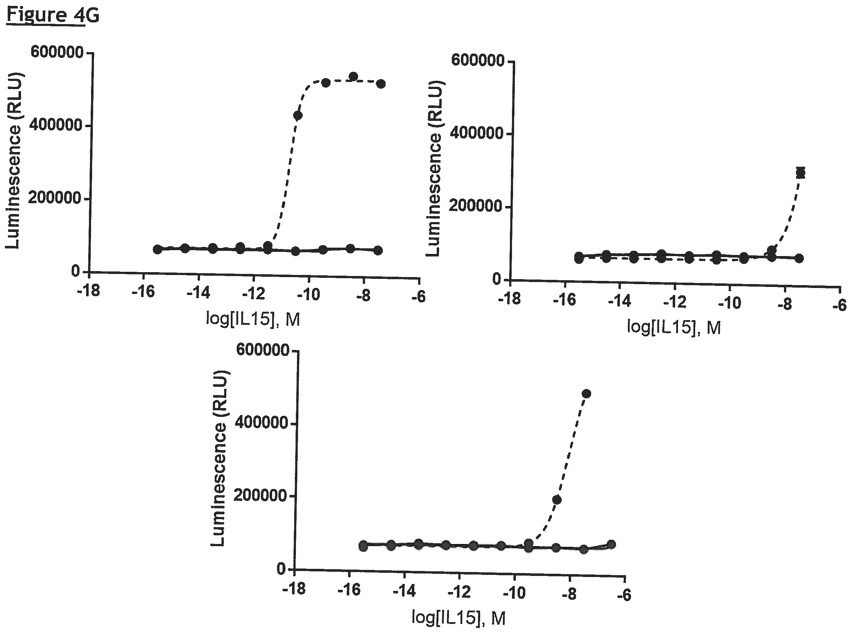

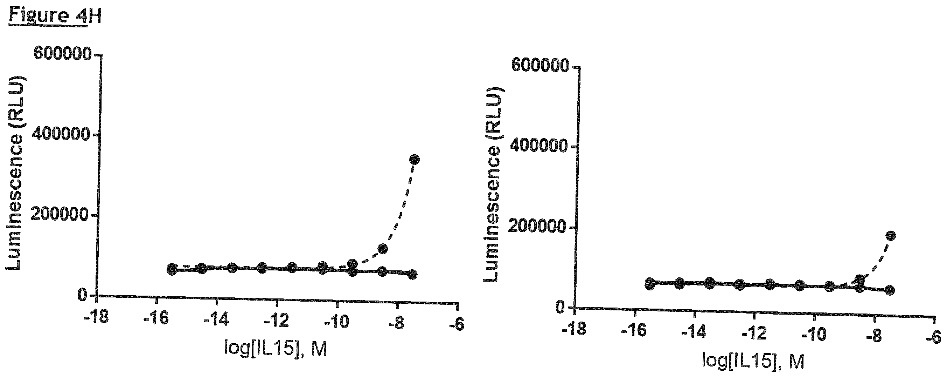

***Figure 4******: Evaluation of IL-15 activity after MMP-9-mediated cleavage of the ICC.*** Relative IL-15 activity is determined using the IL-15 Bioassay (Promega) and expressed as luminescence. ICC were cleaved by MMP-9 (dotted lines) or not (solid lines) prior to the assay. **(A)** Molecules without cofactor; clockwise, from top left: K03201-002, K03201-076, K03201-073; **(B)** Molecule with sushi/covalent; clockwise, from top left: K03201-034, K03201-046, K03201-074; **(C)** Molecule with sushi+ / covalent; clockwise, from top left: K03201-069, K03201-072, K03201-075; **(D)** Molecule with sushi+/coexpression; left: K03201-077, right: K03201-071; **(E)** Molecule with sIL-15Ra/coexpression; left: K03201=027, right: K03201-070; **(F)** Molecules with 9G4 antibody; clockwise, from top left: K03201-086, K03201-029, K00901-006; **(G)** Molecules with NHS76 antibody, clockwise, from top left: K03001-002, K03001-025, K03001-023; **(H)** Molecules with NHS76 antibody; left: K03001-024, right: K03001-026.
***Figure 5******: Evaluation of ICC construct activity in a NK cell assay.*** The bars represent levels of IFNγ produced in pg/mL, open circles the % of CD69⁺ NK cells, and filled circles total NKp46⁺ NK cells.
***Figure 6******: AUC(0-last)*/*dose for total antibody measured in mouse plasma after a single IV dosing in study 1.***
***Figure 7******: AUC(0-last)*/*dose for (A) total antibody and (B) total ICC measured in mouse plasma after a single IV dosing in study 2.***
***Figure 8******: In vivo effect of K03201-079 on NK cells in a renal cell carcinoma model.* (A)** Evaluation of *in vivo* effect of K03201-079 on NK cells in the RENCA model. (B) Comparison of *in vivo* effect of K03201-079 and rlL15 on NK cells in the RENCA model.

### DESCRIPTION

The present disclosure provides a protein complex with prolonged *in vivo* half-life and increased *in vitro* activity, and capable of activating an immune response in the tumour microenvironment, thereby conferring protective anti-tumour immunity.

This protein complex comprises an "immunocytokine", *i.e.,* a fusion between an antibody or a fragment or a derivative thereof and a cytokine. The antibody moiety in the present immunocytokine targets the tumour where the cytokine is released to exert its action. This confers greater specificity to the fusion protein, *i.e.* it generates fewer side effects than immunocytokines of the prior art which merely rely on localised proteolysis for targeting cytokine activity to the tumour (Skrombolas *et al.,* 2019) or than molecules such as the IL-15 superagonist whose bioactivity is not restricted to the tumour (Guo *et al.,* 2017). The immunocytokine of the present protein complex comprises a peptide linker which can be cleaved between the two moieties, allowing better control of the therapeutic activity of the molecule: the fusion protein is surprisingly inactive in the blood but is activated upon reaching the tumour site. The cleavable peptide linker is preferentially cleaved in the tumour microenvironment, thus releasing the cytokine. Targeted delivery of the cytokine thus potentiates its anti-tumour activity, whilst reducing the risks of cytokine-associated toxicity. In addition, the present inventors have surprisingly found that this immunocytokine is even more effective when complexed with a specific cofactor. Not only is the activity of the immunocytokine still tightly controlled, *i.e.*, the cytokine moiety is only activated when released at the tumour site, but the presence of the cofactor results in an immunocytokine with a longer half-life in the plasma. This is particularly advantageous since lower doses are needed to reach similar therapeutic effects as the immunocytokine alone.

Moreover, the present protein complex of immunocytokine and cofactor can surprisingly be produced at high levels and with a high degree of purity. The expression of the immunocytokine in the presence of the cofactor, either as a fusion or by coexpression, results in higher yields than in the absence, and with lower levels of aggregates. Smaller culture volumes and fewer expression steps are needed to obtain therapeutically active amounts of the immunocytokine. Expressing the cofactor with the immunocytokine thus improves the quantity and quality of the efficient expression and production of this valuable biomolecule while minimising time and cost.

This makes the present immunocytokine, in complex with the cofactor, an excellent candidate for a protein therapeutic drug.

In a first aspect, the invention thus relates to a protein complex comprising:
i) a fusion protein comprising an antibody, or antigen-binding fragment thereof, a cleavable peptide linker, and a cytokine or a functional fragment thereof; and
ii) a cofactor.

As used herein, the "protein complex" of the present invention refers to a protein formed by binding of two different monomeric proteins. In the present disclosure, the two monomeric proteins are the immunocytokine and the cofactor. The cofactor may or may not be covalently linked to the fusion protein. In a first aspect, the cofactor is covalently bound to the fusion protein. In another embodiment, the cofactor is not covalently bound to the fusion protein.

Preferably, the cytokine is IL-15. In another preferred embodiment, the cofactor is IL-15Rα or an IL-15-binding fragment thereof. More preferably, the cytokine is IL-15 and the cofactor is IL-15Ra or an IL-15-binding fragment thereof.

A "fusion protein" refers to a chimeric protein encoding two or more separate protein sequences that are recombinantly expressed as a single moiety. "Fused," "fusion," "conjugated", and "connected" are used interchangeably herein. These terms refer to the joining together of two more chemical elements or components, by whatever means including chemical conjugation or recombinant means. These terms are meant to encompass all conjugates, wherein said antibody, or antigen-binding protein thereof is somehow bound to the cleavable peptide linker and the cytokine or functional fragment thereof, by, *e.g*. covalent and/or non-covalent bonds. For example, two distinct proteins can be connected together by "in- frame fusion", which refers to the joining of two or more open reading frames (ORFs) to form a continuous longer ORF, in a manner that maintains the correct reading frame of the original ORFs. Thus, the resulting recombinant fusion protein is a single protein containing two or more segments that correspond to polypeptides encoded by the original ORFs (which segments are not normally so joined in nature). For another example, the two proteins can also be linked together by use of a chemical crosslinker, which results in a protein conjugate that contains two individual polypeptides connected by a crosslinker. These terms encompass all binding arrangements. Preferred arrangements include antibody - linker - cytokine and cytokine - linker - antibody.

### ANTIBODIES

An "antibody" as used herein refers to an immunoglobulin (Ig) molecule capable of specific binding to a target, the "antigen", such as a carbohydrate, polynucleotide, lipid, polypeptide, etc., through at least one antigen recognition site, located in the variable region of the immunoglobulin molecule. The antibody or antigen-binding protein thereof of the present fusion protein mediates the targeted delivery of immunocytokines into disease environments and/or to specific cell subsets. Preferred target antigens are those that are overexpressed in diseased tissues, while remaining at low levels elsewhere. Such antigens are well-known to the skilled person, as-they have been the subject of numerous studies over the years. For example, the antibody moiety of the present immunocytokine may target antigens overexpressed on the surface of malignant cells (*e.g.*, epithelial cell adhesion molecule, EGFR, IGF-1R, GD2 disialoganglioside, HER2/neu, CD20 and CD30), as well as targeting of neoangiogenic antigens found in tumours and chronic inflammation sites (*e.g*., fibronectin, splice variants EDA/EDB and A1 domain of tenascin C).

As used herein, the term "antibody" encompasses not only intact polyclonal or monoclonal antibodies, but also any antigen binding fragment (*i.e.*, "antigen-binding fragment") or single thereof, fusion proteins comprising an antibody, and any other modified configuration of the immunoglobulin molecule that comprises an antigen recognition site including, for example without limitation, scFv, single domain antibodies {*e.g.*, shark and camelid antibodies), maxibodies, minibodies, intrabodies, diabodies, triabodies, tetrabodies, v-NAR and bis-scFv. As used herein, the term "antibody" encompasses both full-length antibodies and their antigen-binding fragments, as well as any derivative thereof. Preferably, the antibody according to the invention, or its derived compounds or antigen-binding fragments, is a monoclonal antibody.

A "monoclonal antibody", as used herein, means an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterised by heavy chains of one and only one class and subclass, and light chains of only one type. Monoclonal antibodies are highly specific and are directed against a single antigen. In addition, in contrast with preparations of polyclonal antibodies which typically include various antibodies directed against various determinants, or epitopes, each monoclonal antibody is directed against a single epitope of the antigen. Since these antibodies are directed against a single epitope, they are highly specific.

An "epitope" is the site on the antigen to which binds the antibody. It can be formed by contiguous residues or by non-contiguous residues brought into close proximity by the folding of an antigenic protein. Epitopes formed by contiguous amino acids are typically retained on exposure to denaturing solvents, whereas epitopes formed by non-contiguous amino acids are typically lost under said exposure.

The generation of the antibody reactive with a specific antigen can be realised by any method known by the man skilled in the art, such as for example, fusion of a myeloma cell with spleen cells from immunised mice or other species compatible with the selected myeloma cells (Kohler & Milstein, Nature, 256:495-497, 1975). The immunised animals could include transgenic mice with human immunoglobulin loci which then directly produce human antibodies. AlternatiVely, aη antibody can be generated by recombinant methods such as selection of libraries of recombinant antibodies in phage or similar vectors. In general, for the preparation of monoclonal antibodies or their functional fragments, especially of murine origin, it is possible to refer to techniques which are described in particular in the manual "Antibodies" (Harlow and Lane, Antibodies: A Laboratory Manual, Cold Spring Harbor Laboratory, Cold Spring Harbor NY, pp. 726, 1988) or to the technique of preparation from hybridomas described by Kohler and Milstein (Nature, 256:495-497, 1975).

An antibody includes an antibody of any class, such as IgG, IgA, or IgM (or subclass thereof), and the antibody needs not be of any particular class. Depending on the antibody amino acid sequence of the constant region of its heavy chains, immunoglobulins can be assigned to different classes.

A typical IgG antibody is composed of two identical heavy chains and two identical light chains that are joined by disulphide bonds. Each heavy and light chain contains a constant region and a variable region. Each variable region contains three segments called "complementarity-determining regions" ("CDRs") or "hypervariable regions", which are primarily responsible for binding an epitope of an antigen. They are usually referred to as CDR1, CDR2, and CDR3, numbered sequentially from the N-terminus. The more highly conserved portions of the variable regions are called the "framework regions".

There are three heavy-chain CDRs and 3 light-chain CDRs. The term "CDR" or "CDRs" is used here in order to indicate, according to the case, one of these regions or several, or even the whole, of these regions which contain the majority of the amino acid residues responsible for the binding by affinity of the antibody for the antigen or the epitope which it recognises.

As used herein, "VH" or "V_{H}" refers to the variable region of an immunoglobulin heavy chain of an antibody, including the heavy chain of an Fv, scFv, dsFv, Fab, Fab', or F(ab')2 fragment. Reference to "VL" or "V_{L}" refers to the variable region of the immunoglobulin light chain of an antibody, including the light chain of an Fv, scFv, dsFv, Fab, Fab', or F(ab')2 fragment.

Antibody constant domains are not involved directly in binding an antibody to an antigen, but exhibit various effector functions. The heavy chain constant regions that correspond to the different classes of immunoglobulins are called α, δ, ε, γ, and µ, respectively. Depending on the amino acid sequence of the constant region of their heavy chains, antibodies or immunoglobulins can be assigned to different classes, i.e., IgA, IgD, IgE, IgG, and IgM, and several of these may be further divided into subclasses (isotypes), e.g., IgG1, IgG2, IgG3, and IgG4; IgA1 and IgA2 (see, W. E. Paul, ed., 1993, Fundamental Immunology, Raven Press, New York, New York).

Papain digestion of antibodies produces two identical antigen binding fragments, called Fab fragments, each with a single antigen binding site, and a residual "Fc" fragment. The crystal structure of the human IgG Fc domain has been determined (Deisenhofer, Biochemistry, 20, 2361-2370, 1981). As used in the specification and claims, "immunoglobulin Fc domain or Fc" means the carboxyl-terminal portion of the immunoglobulin heavy chain constant region. A "native sequence Fc domain", as used herein, comprises an amino acid sequence identical to the amino acid sequence of a Fc domain found in nature. Native sequence human Fc domains include a native sequence human IgG1 Fc domain (non-A and A allotypes); native sequence human IgG2 Fc domain; native sequence human IgG3 Fc domain; and native sequence human IgG4 Fc domain as well as naturally occurring variants thereof.

Although the boundaries of the Fc domain of an immunoglobulin heavy chain might vary, the human IgG heavy chain Fc domain is usually defined to stretch from an amino acid residue at position Cys226 or Pro230 in the hinge region, to the carboxyl-terminus thereof containing the CH2 and CH3 domain of the heavy chain. Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991). The "EU index as in Kabat" refers to the residue numbering of the human IgG1 EU antibody.

The term "hinge region" is generally defined as stretching from Glu216 to Pro230 of human IgG1 (Burton, Mol Immunol, 22: 161-206, 1985). Hinge regions of other IgG isotypes may be aligned with the IgG1 sequence by placing the first and last cysteine residues forming inter-heavy chain S-S bonds in the same positions. The "CH2 domain" of a human IgG Fc portion (also referred to as "Cy2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain (Burton, Mol. Immunol., 22: 161-206, 1985). The "CH3 domain" comprises the stretch of residues C- terminal to a CH2 domain in an Fc portion (*i.e.,* from about amino acid residue 341 to about amino acid residue 447 of an IgG).

The Fc domains are central in determining the biological functions of the immunoglobulin and these biological functions are termed "effector functions". These Fc domain-mediated activities are mediated via immunological effector cells, such as killer cells, natural killer cells, and activated macrophages, or various complement components. These effector functions involve activation of receptors on the surface of said effector cells, through the binding of the Fc domain of an antibody to the said receptor or to complement component(s). The antibody-dependent cellular cytotoxicity (ADCC) and complement-dependent cytotoxicity (CDC) activities involve the binding of the Fc domain to Fc-receptors such as FcγRI, FcγRII, FcγRIII of the effector cells or complement components such as C1q. Of the various human immunoglobulin classes, human IgG1 and IgG3 mediate ADCC more effectively than IgG2 and IgG4.

The present antibodies also comprise chimeric or humanised antibodies.

A chimeric antibody is one containing a natural variable region (light chain and heavy chain) derived from an antibody of a given species in combination with constant regions of the light chain and the heavy chain of an antibody of a species heterologous to said given species.

The antibodies, or chimeric fragments of same, can be prepared by using the techniques of recombinant genetics. For example, the chimeric antibody could be produced by cloning recombinant DNA containing a promoter and a sequence coding for the variable region of a nonhuman monoclonal antibody of interest, notably murine, and a sequence coding for the human antibody constant region. A chimeric antibody coded by one such recombinant gene could be, for example, a mouse-human chimera, the specificity of this antibody being determined by the variable region derived from the murine DNA and its isotype determined by the constant region derived from human DNA. It will be appreciated that in this case, the Fc domain of the chimeric antibody is of human origin. Refer to Verhoeyn et al. (BioEssays, 8:74, 1988) for methods for preparing chimeric antibodies.

In addition, the present disclosure also relates to humanised antibodies arising from the murine antibodies described above. "Humanised antibody" refers herein to an antibody that contains CDR regions derived from an antibody of nonhuman origin, the other parts of the antibody molecule being derived from one (or several) human antibodies. In addition, some of the skeleton segment residues (called FR) can be modified to preserve binding affinity (Jones et al., Nature, 321:522-525, 1986; Verhoeyen et al., Science, 239:1534-1536, 1988; Riechmann et al., Nature, 332:323-327, 1988). The Fc domain of a humanised antibody will be of human origin, as in chimeric antibodies.

The humanised antibodies or fragments of same can be prepared by techniques known to a person skilled in the art (such as, for example, those described in the documents Singer et a/., J. Immun., 150:2844-2857, 1992; Mountain et al., Biotechnol. Genet. Eng. Rev., 10:1-142, 1992; and Bebbington et al., Bio/Technology, 10: 169-175, 1992). Such humanised antibodies are preferred for their use in methods involving *in vitro* diagnoses or preventive and/or therapeutic treatment *in vivo.* Other humanisation techniques, also known to a person skilled in the art, such as, for example, the "CDR grafting" technique described by PDL in patents EP 0 451 261, EP 0 682 040, EP 0 939 127, EP 0 566 647 or US 5,530,101, US 6,180,370, US 5,585,089 and US 5,693,761. US patents 5,639,641 or 6,054,297, 5,886,152 and 5,877,293 can also be cited.

Although it is possible to use antibody fragments in the present immunocytokines, it is preferred to use full-length, bivalent antibodies. A monovalent antibody such as a Fab or a scFv has only a single binding site for an antigen (as distinct from natural 'bivalent' antibodies), *i.e*., is composed of a single antigen-binding arm. As known to the skilled person, the greater an immunoglobulin's valency (number of antigen binding sites), the greater the amount of antigen it can bind. There is a significant affinity change between monovalent and bivalent bindings with usually a ca. 1,500-fold change in Kd values. Bivalent antibodies can thus be used at a lower dose to attain similar therapeutic efficiency as monovalent Fab or scFv fragments, thus limiting the risks of secondary effects.

Preferably, the antibody which can be used in the immunocytokines described herein is an antibody which does not bind specifically the cytokine moiety of said immunocytokine. For example, if the cytokine is IL-12, the antibody according to this embodiment is not an antibody which binds IL-12.

In another embodiment the antibody used in the present immunocytokine is an antibody which binds specifically a tumour-associated antigens (TAA) or a tumour-specific antigens (TSA). As used herein, a "tumour-associated antigen" is a protein or other molecule that is found on cancer cells whilst a "tumour-specific antigen" is a protein or other molecule that is found on cancer cells and not on normal cells. Tumour-specific antigens are known in the art.

Tumour antigens can be classified in a variety of ways. Tumour antigens include antigens encoded by genes that have undergone chromosomal alteration. Many of these antigens are found in lymphoma and leukaemia. Even within this classification, antigens can be characterised as those that involve activation of quiescent genes. These include BCL-1 and IgH (Mantel cell lymphoma), BCL-2 and IgH (Follicular lymphoma), BCL-6 (Diffuse large B-cell lymphoma), TAL-1 and TCR delta or SIL (T-cell acute lymphoblastic leukaemia), c-MYC and IgH or IgL (Burkitt lymphoma), MUN/IRF4 and IgH (Myeloma), PAX-5 (BSAP) (Immunocytoma).

Other tumour antigens that involve chromosomal alteration and thereby create a novel fusion gene and/or protein include RARoa, PML, PLZF, NPMor NuM4 (Acute promyelocytic leukaemia), BCR and ABL (Chronic myeloid/acute lymphoblastic leukaemia), MLL (HRX) (Acute leukaemia), E2A and PBXor HLF (B-cell acute lymphoblastic leukaemia), NPM, ALK (Anaplastic large cell leukaemia), and NPM, MLF-1 (Myelodysplastic syndrome/acute myeloid leukaemia).

Other tumour antigens are specific to a tissue or cell lineage. These include cell surface proteins such as CD20, CD22 (Non-Hodgkin's lymphoma, B-cell lymphoma, Chronic lymphocytic leukaemia (CLL)), CD52 (B-cell CLL), CD33 (Acute myelogenous leukaemia (AML)), CD 10 (gp100) (Common (pre-B) acute lymphocytic leukaemia and malignant melanoma), CD3/T-cell receptor (TCR) (T-cell lymphoma and leukaemia), CD79/B-cell receptor (BCR) (B-cell lymphoma and leukaemia), CD26 (Epithelial and lymphoid malignancies), Human leukocyte antigen (HLA)-DR, HLA-DP, and HLA-DQ (Lymphoid malignancies), RCAS1 (Gynaecological carcinomas, biliary adenocarcinomas and ductal adenocarcinomas of the pancreas), and Prostate specific membrane antigen (Prostate cancer).

Tissue- or lineage-specific tumour antigens also include epidermal growth factor receptors (high expression) such as EGFR (HER1 or erbB1) and EGFRvIII (Brain, lung, breast, prostate and stomach cancer), erbB2 (HER2 or HER2/neu) (Breast cancer and gastric cancer), erbB3 (HER3) (Adenocarcinoma), and erbB4 (HER4) (Breast cancer).

Tissue- or lineage-specific tumour antigens also include cell-associated proteins such as Tyrosinase, Melan-A/MART-1, tyrosinase related protein (TRP)-1/gp75 (Malignant melanoma), Polymorphic epithelial mucin (PEM) (Breast tumours), and Human epithelial mucin (MUC1) (Breast, ovarian, colon and lung cancers).

Tissue- or lineage-specific tumour antigens also include secreted proteins such as Monoclonal immunoglobulin (Multiple myeloma and plasmacytoma), Immunoglobulin light chains (Multiple Myeloma), alpha-fetoprotein (Liver carcinoma), Kallikreins 6 and 10 (Ovarian cancer), Gastrin-releasing peptide/bombesin (Lung carcinoma), and Prostate specific antigen (Prostate cancer).

Still other tumour antigens are cancer testis (CT) antigens that are expressed in some normal tissues such as testis and in some cases placenta. Their expression is common in tumours of diverse lineages and as a group the antigens form targets for immunotherapy. Examples of tumour expression of CT antigens include MAGE-A1, -A3, -A6, -A12, BAGE, GAGE, HAGE, LAGE-1, NY-ESO-1, RAGE, SSX-1, -2, -3, -4, -5, -6, -7, - 8, -9, HOM-TES-14/SCP-1, HOM-TES-85 and PRAME. Still other examples of CT antigens and the cancers in which they are expressed include SSX-2, and -4 (Neuroblastoma), SSX-2 (HOM-MEL-40), MAGE, GAGE, BAGE and PRAME (Malignant melanoma), HOM-TES-14/SCP-1 (Meningioma), SSX-4 (Oligodendroglioma), HOM-TES-14/SCP-1, MAGE-3 and SSX-4 (Astrocytoma), SSX member (Head and neck cancer, ovarian cancer, lymphoid tumours, colorectal cancer and breast cancer), RAGE-1, -2, -4, GAGE-1-2, -3, -4, -5, - 6, -7 and -8 (Head and neck squamous cell carcinoma (HNSCC)), HOM-TES14/SCP-1, PRAME, SSX-1 and CT-7 (Non-Hodgkin's lymphoma), and PRAME (Acute lymphoblastic leukaemia (ALL), acute myelogenous leukaemia (AML) and chronic lymphocytic leukaemia (CLL)).

Other tumour antigens are not specific to a particular tissue or cell lineage. These include members of the carcinoembryonic antigen (CEA) family: CD66a, CD66b, CD66c, CD66d and CD66e. These antigens can be expressed in many different malignant tumours and can be targeted by immunotherapy.

Still other tumour antigens are viral proteins and these include Human papilloma virus protein (cervical cancer), and EBV-encoded nuclear antigen (EBNA)-1 (lymphomas of the neck and oral cancer).

Still other tumour antigens are mutated or aberrantly expressed molecules such as but not limited to CDK4 and beta-catenin (melanoma).

In some embodiments, the antigen is a tumour antigen. The tumour antigen may be selected from the group consisting of MART-1/Melan-A, gp100, adenosine deaminase-binding protein (ADAbp), FAP, cyclophilin b, colorectal associated antigen (CRC)-C017-1A/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T-cell receptor/CD3-zeta chain, and CD20. The tumour antigen may also be selected from the group consisting of MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5). In still another embodiment, the tumour antigen is selected from the group consisting of GAGE-1, GAGE-2, GAGE-3, GAGE-4, GAGE-5, GAGE-6, GAGE-7, GAGE-8, GAGE-9. And in yet a further embodiment, the tumour antigen is selected from the group consisting of BAGE, RAGE, LAGE-1, NAG, GnT-V, MUM-1, CDK4, tyrosinase, p53, MUC family, HER2/neu, p21 ras, RCAS 1, α-fetoprotein, E-cadherin, α-catenin, β-catenin, .gamma.-catenin, p120ctn, gp100Pmel117, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, Smad family of tumour antigens, Imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2.

Cancer or tumour antigens can also be classified according to the cancer or tumour they are associated with (*i.e.,* expressed by). Cancers or tumours associated with tumour antigens include acute lymphoblastic leukaemia (etv6; am11; cyclophilin b), B cell lymphoma (Ig-idiotype); Burkitt's (Non-Hodgkin's) lymphoma (CD20); glioma (E-cadherin; α-catenin; β-catenin; .gamma.-catenin; p120ctn), bladder cancer (p21ras), biliary cancer (p21ras), breast cancer (MUC family; HER2/neu; c-erbB-2), cervical carcinoma (p53; p21ras), colon carcinoma (p21ras; HER2/neu; c-erbB-2; MUC family), colorectal cancer (Colorectal associated antigen (CRC)-0017-1A/GA733; APC), choriocarcinoma (CEA), epithelial cell-cancer (cyclophilin b), gastric cancer (HER2/neu; c-erbB-2; ga733 glycoprotein), hepatocellular cancer (a-fetoprotein), Hodgkin's lymphoma (lmp-1; EBNA-1), lung cancer (CEA; MAGE-3; NY-ESO-1), lymphoid cell-derived leukaemia (cyclophilin b), melanoma (p15 protein, gp75, oncofoetal antigen, GM2 and GD2 gangliosides), myeloma (MUC family; p21 ras), non-small cell lung carcinoma (HER2/neu; c-erbB-2), nasopharyngeal cancer (lmp-1; EBNA-1), ovarian cancer (MUC family; HER2/neu; c-erbB-2), prostate cancer (Prostate Specific Antigen (PSA) and its immunogenic epitopes PSA-1, PSA-2, and PSA-3; PSMA; HER2/neu; c-erbB-2), pancreatic cancer (p21ras; MUC family; HER2/neu; c-erbB-2; ga733 glycoprotein), renal (HER2/neu; c-erbB-2), squamous cell cancers of cervix and oesophagus (viral products such as human papilloma virus proteins and non-infectious particles), testicular cancer (NY-ESO-1), T cell leukaemia (HTLV-1 epitopes), and melanoma (Melan-A/MART-1; cdc27; MAGE-3; p21ras; gp100^{Pme117}). More preferably, the antibody of the present immunocytokine is an antibody which does not bind specifically the cytokine moiety of said immunocytokine, but binds specifically a tumour-associated antigen (TAA) or a tumour-specific antigen (TSA).

Examples of antibodies which can be used in the present immunocytokines include: Alemtuzumab (CD52), Alirocumab (PCSK9), Arcitumomab (Human CEA (carcinoembryonic antigen)), Atezolizumab (PD-L1), Avelumab (PD-L1), AVE1642 (IGF-1R), Basiliximab (CD25 (a chain of IL2 receptor)), Belimumab (BLyS), Bevacizumab (VEGF), Blinatumomab (CD19), Brodalumab (IL-17RA), Capromab (Tumour surface antigen PSMA), Catumaxomab (EpCAM and CD3), Catumaxomab (EpCAM), Certolizumab pegol (TNFa), Cetuximab, (EGFR), Cixutumumab (IGF-1R), Daclizumab (CD25 (a chain of IL2 receptor)), Dalotuzumab (IGF-1R), Daratumumab (CD38), Dinutuximab (GD2), Dupilumab (IL-4Ra), Durvalumab (PD-L1), Efalizumab (CD11a), Elotuzumab (SLAMF7), Evolocumab (LDL-C / PCSK9), Fanolesomab (CD15), Figitumumab (IGF-1R), Ganitumab (IGF-1R), Golimumab (TNFa), Ibritumomab tiuxetan (CD20), Infliximab (TNFa), Ipilimumab (CTLA-4), Necitumumab (EGFR), Nivolumab (PD-1), Nofetumomab (Carcinoma-associated antigen), Obinutuzumab (CD20), Ocrelizumab (CD20), Ofatumumab (CD20), Olaratumab (PDGFR-a), Panitumumab (EGFR), Pembrolizumab (PD-1), Pertuzumab (HER2), R1507 (IGF-1R), Ramucirumab (VEGF), Ranibizumab (VEGF-A), Rituximab (CD20), Siltuximab (cCLB8), Tocilizumab (IL-6 receptor), Trastuzumab (HER-2), Vedolizumab (Integrin-α4β7), Votumumab (Cytokeratin tumour-associated antigen).

### CLEAVABLE PEPTIDE LINKERS

A "peptide linker" as used herein refers to an amino acid stretch between two different peptide or polypeptide subunits, e.g. between an antibody and a cytokine. Linkers have often been used in the art. They generally adopt an extended conformation to allow for maximal flexibility. In addition, they may contain a site recognised by an enzyme.

A "cleavable peptide linker" as used herein refers to a polyvalent linker covalently bonded to an antibody, or an antigen-binding fragment thereof, and covalently bonded to a cytokine, or fragment thereof, which is enzymatically cleavable (*e.g*. at a cleavage site). According to the invention, upon hydrolysis (proteolytic cleavage) of the cleavable peptide linker, the cytokine moiety, preferably IL-15, is released, enabling it to exert its therapeutic activity. In preferred embodiments the cleavable peptide linker is recombinantly expressed as part of the immunocytokine. In other embodiments, the cleavable peptide linker is a linker formed by reacting a functional (reactive) group attached to the linker with an antibody, or an antigen-binding fragment thereof using, for example, conjugate chemistry. In yet other embodiments, the cleavable peptide linker is a linker formed by reacting a functional (reactive) group attached to the linker with a cytokine, preferably IL-15, or fragment thereof, using, for example, conjugate chemistry. In a preferred embodiment, the cleavable peptide linker connects the cytokine, preferably IL-15, or fragment thereof, to the heavy chain of the antibody, or an antigen-binding fragment thereof. In another embodiment, the cleavable peptide linker connects the cytokine, preferably IL-15, or fragment thereof, to the light chain of the antibody, or an antigen-binding fragment thereof. The cleavable peptide linker may connect the cytokine, preferably IL-15, or fragment thereof, to the N-terminus of one of the heavy and light chains of the antibody, or an antigen-binding fragment thereof. It is also possible that the cleavable peptide linker connects the cytokine, preferably IL-15, or fragment thereof, to the C-terminus of the heavy and light chains of the antibody, or an antigen-binding fragment thereof. Most preferably, the cleavable peptide linker connects the cytokine, preferably IL-15, or fragment thereof, to the N-terminus or C-terminus of the heavy chain of the antibody, or an antigen-binding fragment thereof.

In some embodiments, the immunocytokine may contain only one cleavable peptide linker. In some other embodiments, the immunocytokine may contain more than one cleavable peptide linker. Preferably, in that case, the more than one cleavable peptide linker are contiguous, *i.e.* they are attached one to the other, with the cleavable peptide linker at one end being bound to the antibody and the cleavable peptide linker at the other end being bound to the cytokine, preferably Il-15, or a functional fragment thereof. In a particular embodiment, the immunocytokine may comprise at least 1, at least 2, at least 3; at least 4, at least 5 cleavable peptide linkers. In a specific embodiment, the immunocytokine comprises 2 cleavable peptide linkers.

The cleavable peptide linker provided herein may include a protease cleavage site.

A "cleavage site" as used herein, refers to a recognisable site for cleavage of a portion of a linker (*e.g*. cleavable peptide linker as described hereinabove) present in an immunocytokine described herein. Thus, a cleavage site may be found in the sequence of a cleavable peptide linker as described herein, including embodiments thereof. In embodiments, the cleavage site is an amino acid sequence that is recognised and cleaved by a cleaving agent (*e.g*. a peptidyl sequence). Exemplary cleaving agents include proteins, enzymes, DNAzymes, RNAzymes, metals, acids, and bases. Exemplary cleavage sites include notably PVGLIG (SEQ ID NO. 39), also referred to herein as L6, and dimers thereof (PVGLIGPVGLIG, SEQ ID NO. 40, L6-L6).

A "protease cleavage site" as used herein is a cleavage site which is recognised and specifically cleaved by a protease. According to a preferred embodiment, the protease cleavage site is a tumour-associated protease cleavage site. A "tumour-associated protease cleavage site" as used herein refers to an amino acid sequence recognised by a protease, whose expression is specific for a tumour cell or tumour cell environment thereof or mainly expressed in the tumour cell or tumour environment compared to healthy tissues or is only/mainly active in the tumour cell or tumour environment. In embodiments, the protease cleavage site is a matrix metalloprotease (MMP) cleavage site, a prostate specific antigen (PSA) protease cleavage site, a membrane type serine protease 1 (MT-SP1) protease cleavage site, an uPA urokinase plasminogen activator cleavage site, or a legumain protease cleavage site. In some embodiments, the matrix metalloprotease (MMP) cleavage site is a MMP 9 cleavage site, a MMP 13 cleavage site, or a MMP 2 cleavage site. Protease cleavage sites may be designated by a specific amino acid sequence but may also encompass any variation of this canonical amino acid sequence which is still recognised and cleaved by the protease of interest.

Preferably, the cleavable peptide linker is a matrix metalloprotease (MMP) cleavage site. More preferably, the cleavable peptide linker comprises a MMP 9 cleavage site or a MMP 2 cleavage site. Examples of MMP cleavage sites include GPLGIAGQ, GPLGLWAQ, GPLGMLSQ, PLGLAG, and PVGLIG.

In another preferred embodiment, the cleavable peptide linker is a urokinase plasminogen activator (uPA) cleavage site. Examples of uPA cleavage sites include SGRS, SGRSA, and PSSSRRRVN.

The term "MMP 2" or "MMP 2 protease" as used herein refers to the matrix metalloproteinase 2 (MMP 2). MMP-2 is the protein identified by the NCBI sequence reference Gl: 189217853. The term "MMP-9" or "MMP9 protease" as used herein refers to the matrix metalloproteinase 9 (MMP-9). MMP9 is the protein identified by the NCBI sequence reference Gl: 74272287. The term "MMP 13" or "MMP 13 protease" as used herein refers the matrix metalloproteinase 13 (MMP 13). MMP 13 is the protein identified by the NCBI sequence reference GL4505209. The term "PSA" or "PSA protease" as used herein refers to the prostate-specific antigen (PSA), also known as gamma seminoprotein or kallikrein-3. PSA is the protein identified by the NCBI sequence reference GL71834853. The term "PSMA" or "PSMA protease" as used herein refers to the prostate-specific membrane antigen (PSMA), also known as glutamate carboxypeptidase II (GCPII), N-acetyl-L-aspartyl-L-glutamate peptidase I (NAALADase I) or NAAG peptidase. PSMA is the protein identified by the NCBI sequence reference GL62548858. The term "fibroblast associated protein" as used herein refers to the fibroblast associated protein. Fibroblast associated protein is the protein as identified by the NCBI sequence reference GL 1888316. The term "MT-SPl" or "MT-SPl protease" as used herein refers to the membrane type serine protease 1 (MT-SPl). MT-SPl is the protein identified by the NCBI sequence reference Gl: 1 1415040. The term "legumain" or "legumain protease" as used herein refers to the legumain protein. Legumain is the protein identified by the NCBI sequence reference GL2842759. The term uPA as used herein refers to the urokinase-type plasminogen activator identified by the NCBI sequence reference Gene ID: 5328.

In some embodiment, the cleavable peptide linker comprises at least 2, at least 3, at least 4, at least 5, at least 6, at least 7, at least 8, at least 9, at least 10, at last 11, at least 12, or at least 13 amino acids. In other embodiments, cleavable peptide linkers are 5-mers (*i.e.* peptides 5 amino acids in length), 6-mers (*i.e.* peptides 6 amino acids in length), 7-mers (*i.e.* peptides 7 amino acids in length), 8-mers (*i.e.* peptides 8 amino acids in length), 9-mers (*i.e.* peptides 9 amino acids in length), 10-mers (*i.e.* peptides 10 amino acids in length), 11-mers (*i.e.* peptides 11 amino acids in length), 12-mers (*i.e.* peptides 12 amino acids in length), or 13-mers (*i.e*. peptides 13 amino acids in length).

Most preferably, said the sequence of said cleavage peptide linker is selected from the group consisting of: GPLGIAGQ, GPLGLWAQ, GPLGMLSQ, PLGLAG, PVGLIG, SGRS, SGRSA, and PSSSRRRVN.

### CYTOKINES

The term "cytokine" as used herein refers to a member of a family of small secreted regulatory proteins which have an effect on the immune system. Cytokines are involved in cell-to-cell communication and regulate many cellular functions, such as cell survival and growth, as well as induction of the expression of many genes. Secretion of cytokines thus enables the rapid propagation of immune signalling in a multifaceted and efficient manner. Cytokines regulate the nature, intensity and duration of the immune response by exerting a variety of effects on lymphocytes and/or other cells. Indeed, cytokines are usually classified into pro-and antiinflammatory cytokines. Some cytokines are also capable of mobilising the immune system to fight cancer (see e.g., Floros & Tarhini, Semin. Oncol. 42(4): 539-548, 2015). Cytokines can be produced by many cell types, including immune and non-immune cells. Examples of cytokines include interleukins, lymphokines, monokines, interferons, colony stimulating factors, and chemokines, *inter alia.* A "cytokine" as used herein may be any one of IL-1, IL-2, IL-3, IL-4, IL-5, IL-6, IL-7, IL-8, IL-9, IL-10, IL-11, IL-12, IL-13, IL-15, IL-16, IL-17, IL-18, IL-19, IL-20, IL-21, IL-22, IL-23, , IL-24, IL-26, IL-28, IL-29, IL-33, IL-36, IL37, IL-38, IFN-α (including IFN-α1/13, IFN-α2, IFN-α4, IFN-α5, IFN-α6, IFN-α7, IFN-α8, IFN-α10, IFN-α14, IFN-α16, IFN-α17, and IFN-α21), IFN-β, IFN-γ, IFN-λ, TNF-α, TNF-β, TGF-β1, M-CSF, G-CSF, GM-CSF, and CXL10. According to the invention, the cytokine is preferably inactivated or attenuated when linked to the ICC and became active only after cleavage of ICC by the protease.

The term "functional fragment" with regard to said cytokines is to be interpreted essentially in analogy to the same term for antibodies (see below). Functional fragments and derivatives of cytokines are those that essentially have the same physiological function/activity as the naturally occurring cytokines. Notably, functional fragments and derivatives of cytokines are capable of binding the cytokine's cognate receptor.

Preferably, the cytokine is IL-15. By "IL-15" or "interleukin-15", it is herein referred to a cytokine that regulates T and natural killer cell activation and proliferation. Interleukin-15 (IL-15) is a 14 to 15 kDa member of the 4α-helix bundle family of cytokines composed of 114 amino acids whose sequence is available under the accession number NP_000576.1. There is 97% sequence identity between human and simian IL-15 and 73% between human and mouse. This appears to be sufficient for hulL-15 to render it biologically active on simian and murine cells.

IL-15 displays high structural similarity to Interleukin-2 (IL-2). Like IL-2, IL-15 binds to and signals through a complex composed of IL-2/IL-15 receptor beta chain (CD122) and the common gamma chain (gamma-C, CD132). Specificity of the signalling is ensured by IL15 being recognised by the alpha unit of its receptor (IL15Rα), whilst IL-2 binds IL2Rα; IL-15 stimulates the production of proinflammatory cytokines (*e.g*. TNFα, IL-1, IFNγ), the proliferation and lg synthesis of activated B cells, the activation of T_{H}1, monocytes and lymphokine activated killer cells, the proliferation of mast cells and T cells and inhibits the apoptosis of T and B cells. In addition to the mentioned functional activities IL-15 plays a pivotal role in the development, survival and function of NK cells [Joost J. Oppenheim et al., Cytokine Reference; 213-221, (2002)]. IL-15 is a cytokine that primarily stimulates the proliferation and cytotoxic functions of CD8T cells and NK cells leading to enhanced anti-tumour responses (Waldmann, J. Investi.g Dermatol. Symp. Proc. 16(1): S28-30, 2013). While initially showing promise as a cancer therapeutic, the efficacy of IL-15 was limited by its short in vivo half-life. However, new IL-15-based therapies have been developed and are currently in clinical trials (Robinson & Schluns, Immunol. Lett. 190: 159-168, 2017). The inventors have now shown that IL-15 is not active when fused to an antibody moiety and becomes activated only when released by the cleavage of the linker. Immunocytokines comprising IL-15 localise *in vivo* to the tumour where they are cleaved. This allows for circumventing the short half-life problem. In addition, the active cytokine is delivered to the site where it is needed, reducing the risks of side effects.

According to this embodiment, the invention relates to a protein complex comprising:
i) a fusion protein comprising an antibody, or antigen-binding fragment thereof, a cleavable peptide linker, and a cytokine, preferably IL-15, or a functional fragment thereof; and
ii) a cofactor.

### COFACTORS

The term "cofactor" as used herein refers to a compound which is capable of interacting with the cytokine moiety, such as *e.g*., IL-15, of the immunocytokine.

By "interaction" or "interact," it is meant any type of physical association between proteins, whether covalent or non-covalent. Examples of non-covalent bonds include electrostatic bonds, hydrogen bonds, and Van der Waals forces. Furthermore, the interactions between proteins may either be direct or indirect. Depending upon the type of interaction present, various methods may be used to measure the level of interaction. For example, the strengths of covalent bonds are often measured in terms of the energy required to break a certain number of bonds (*i.e.,* kcal/mol). Non-covalent interactions are often described as above, and also in terms of the distance between the interacting molecules. Indirect interactions may be described in a number of ways, including the number of intermediary agents involved, or the degree of control exercised over the one interacting polypeptide over the other.

Preferably, the cofactor is capable of binding to the cytokine moiety (*e.g*., IL-15). By "binding", "binds", or the like, it is meant a direct interaction between the cofactor and the immunocytokine, thus forming a complex which is relatively stable under physiological conditions. Methods for determining whether two molecules bind are well known in the art and include, for example, equilibrium dialysis, surface plasmon resonance, and the like. In a particular embodiment, said cofactor thereof, binds to the cytokine moiety with an affinity that is at least two-fold greater than its affinity for binding to a non-specific molecule such as BSA or casein.

Preferably, the cofactor is a protein or a fragment thereof which is capable of binding the cytokine moiety, such as IL-15. For example, the cofactor may be the physiological receptor for the cytokine, or a cytokine-binding fragment thereof.

In a preferred aspect, the cofactor is a receptor for IL-15 or an IL-15-binding fragment thereof.

IL-15 binds and signals through a high affinity receptor, IL-15R, which comprises the IL-2Rβ-chain and IL-2Rγ-chain. These two subunits are also part of the IL-2 receptor (IL-2R). Binding specificity for IL-15 or IL-2 is conferred by a third subunit, *i.e.* IL-15R alpha-chain (IL-15Rα) or IL-2R alpha-chain, respectively. IL-15Rα is capable of binding IL-15 independently of other subunits.

Preferably, the cofactor is IL-15Rα or an IL-15-binding fragment thereof.

As used herein, "IL-15Rα" or "IL-15R alpha-chain" can be any IL-15Rα from any species, such as human or non-human mammalian IL-15Rα or non-mammal IL-15Rα. Exemplary non-human mammals comprise such as pigs, rabbits, monkeys, chimpanzees, mice, and the like; non-mammals comprise such as chickens and the like. Preferably, "IL-15Rα" as used herein refers human IL-15Rα, *i.e.,* a 267-residue polypeptide (Uniprot accession number: Q13261) encoded by the human *IL15RA* gene ((Gene ID: 3601). "IL-15Rα" as used herein also encompasses all isoforms and variants of this polypeptide, provided they bind to IL-15. Isoforms which do not bind to IL-15 include isoforms 5, 6, 7, and 8, as described on the Uniprot web site. Variants of IL-15Ra which are still capable of binding IL-15 are known in the art (see *e.g.,* EP 3 235 830 A1). IL-15Rα is a transmembrane polypeptide, whose extracellular domain is responsible for binding IL-15. This extracellular domain can be generated by proteolysis shedding of the membrane-anchored receptor.

The cofactor may also be an IL-15-binding fragment of IL-15Rα, such as a soluble IL-15Rα (sIL-15Rα). As used herein, "soluble IL-15Rα" or "sIL-15Rα" refer to the extracellular domain of IL-15Rα. Preferably, sIL-15Rα has the sequence represented by SEQ ID NO. 53. The sIL-15Rα polypeptide is capable of binding IL-15 independently of other polypeptides. This binding is notably mediated by a specific structural domain, called the sushi domain, present in the IL15Rα extracellular domain. Sushi domain, also known as Complement control protein (CCP) module, or short consensus repeat (SCR), is an evolutionary conserved protein domain characterised by a consensus sequence spanning ∼60 residues containing four invariant cysteine residues forming two disulphide-bridges (I-III and II-IV), a highly conserved tryptophan, and conserved glycine, proline, and hydrophobic residues. ILR15α comprises a unique sushi domain, which is located between residues 31 and 95 of the receptor's extracellular domain. Preferably, the IL-15-binding fragment of IL15Rα comprises the sushi domain present in this receptor. More preferably, the IL-15-binding fragment of IL15Rα consists of the IL-15Ra sushi domain. Even more preferably, the IL-15Rα sushi domain has the sequence represented by SEQ ID NO. 51. Alternatively, the IL-15-binding fragment of IL15Rα provided herein encompasses molecules comprising a sushi domain obtained by one or more amino acid substitutions, insertions or deletions. In particular, the IL-15-binding fragment of IL15Rα may comprise the sushi domain and additional amino acids of IL-15Rα. For example, the IL-15-binding fragment may further comprise at least 5, at least 10, at least 15 additional amino acids of IL-15Rα. More preferably, the IL-15 binding fragment consists of the sushi domain and 11 additional IL15Ra residues. This polypeptide is herein referred to as "sushi+" or "IL-15Rα sushi+". Specifically, IL-15Rα sushi+ as used herein has the sequence represented by SEQ ID NO. 52.

Accordingly, the invention relates to a protein complex comprising:
i) a fusion protein comprising an antibody, or antigen-binding fragment thereof, a cleavable peptide linker, and IL-15 or a functional fragment thereof; and
ii) IL-15Rα or an IL-15-binding fragment thereof,
wherein the IL-15-binding fragment is selected in the group consisting of: soluble IL-15Rα of SEQ ID NO. 53, IL-15Rα sushi of SEQ ID NO. 51, and IL-15Rα sushi+ of SEQ ID NO. 52.

The cofactor may be covalently linked to the immunocytokine. In a first embodiment, the cofactor is bound to the immunocytokine by a peptide bond. For example, the cofactor may be expressed in a fusion with the immunocytokine. The cofactor may be linked to the antibody moiety. In such a case, the cofactor may be fused to the same immunoglobulin chain as the cytokine (*e.g*., IL-15) or a functional fragment thereof, or it may be fused to the other chain. Alternatively, the cofactor may be directly linked to the cytokine, *e.g.* IL-15, or a functional fragment thereof. This configuration is preferred as it seems to be more favourable to the binding of the cofactor to the cytokine moiety of the immunocytokine.

According to this embodiment, the present protein complex comprises:
i) a fusion protein comrising an antibody, or antigen-binding fragment thereof, a cleavable peptide linker, and IL-15 or a functional fragment thereof; and
ii) IL-15Rα or an IL-15-binding fragment thereof,
wherein the IL-15-binding fragment is selected in the group consisting of: soluble IL-15Rα of SEQ ID NO. 53, IL-15Rα sushi of SEQ ID NO. 51, and IL-15Rα sushi+ of SEQ ID NO. 52, and
IL-15Rα or the IL-15-binding fragment thereof covalently linked to the fusion protein, preferably to IL-15 or the functional fragment thereof.

When the cofactor (*e.g*. IL-15Rα or an IL-15-binding fragment thereof) is fused to the immunocytokine (*e.g*., IL-15 or a functional fragment thereof), the cofactor may be separated from the immunocytokine by a linker. Preferably, the peptide linker is an unstructured flexible linker. Without being bound by theory, it is thought that a cofactor may interact more easily and more efficiently with the cytokine moiety, *e.g.* IL-15, or a functional fragment thereof, when the linker is flexible and does not present any specific structure. Preferably, the linker is not a cleavable linker, as described above. Preferably, the linker mostly comprises glycine and serine residues. In some embodiment, the peptide linker comprises at least 2, at least 3, at least 4, at least 5, at least 10, at least 15, at least 20, at least 25, or at least 30 amino acids. In other embodiments, cleavable peptide linkers are 2-mers (*i.e*. peptides 2 amino acids in length), 3-mers (*i.e*. peptides 3 amino acids in length), 4-mers (*i.e.* peptides 4 amino acids in length), 5-mers (*i.e.* peptides 5 amino acids in length), 10-mers (*i.e.* peptides 10 amino acids in length), 15-mers (*i.e.* peptides 15 amino acids in length), 20-mers (*i.e*. peptides 20 amino acids in length), 25-mers (*i.e*. peptides 25 amino acids in length), or 30-mers (*i.e.* peptides 30 amino acids in length). If the sequence of the linker peptide is too short, the advanced structure folding of two proteins may be affected, and thus interfered with each other; if the sequence of the linker peptide is too long, the problem of immunogenicity is concerned, since the linker peptide sequence itself is a new antigen.

The cofactor may also be conjugated to the immunocytokine by use of a chemical crosslinker, which results in a protein conjugate that contains two individual polypeptides connected by a crosslinker.

Alternatively, the cofactor may bind to the immunocytokine through non-covalent interactions. Preferably, the cofactor binds to the cytokine moiety of the immunocytokine through non-covalent interactions. In non-covalent interactions (*e.g*., electrostatic, hydrogen bonding and Van der Waals interactions), although the interaction energy per unit interaction is quite small (less than 40 kJ/interaction), the cumulative effect of multiple points of interaction along two surfaces can be substantial and can lead to strong binding between two polypeptides. In the present embodiment, the multiple interactions between the cytokine, or functional fragment thereof, and the cofactor ensure the formation of a stable complex which retains its activity when administered in vivo. For example, the combination of IL-15 with sIL-15Rα in solution results in the generation of a complex with high biological potency, the IL-15 superagonist (IL-15 SA), which is undergoing clinical trials as N-803 (Guo et al., Cytokine Growth Factor Rev. 38: 10-21, 2017).

According to this embodiment, the present protein complex comprises:
i) a fusion protein comprising an antibody, or antigen-binding fragment thereof, a cleavable peptide linker, and IL-15 or a functional fragment thereof; and
ii) IL-15Rα or an IL-15-binding fragment thereof,
wherein the IL-15-binding fragment is selected in the group consisting of: soluble IL-15Rα of SEQ ID NO. 53, IL-15Ra sushi of SEQ ID NO. 51, and IL-15Ra sushi+ of SEQ ID NO. 52, and
IL-15Ra or the IL-15-binding fragment thereof is linked non-covalently to the fusion protein, preferably to IL-15 or the functional fragment thereof.

### POLYNUCLEOTIDES

Also provided herein are polynucleotides comprising a nucleotide sequence encoding a fusion protein or a cofactor as described above. Also provided herein are polynucleotides that hybridise under high stringency, intermediate or lower stringency hybridisation conditions, *e.g*., as defined supra, to polynucleotides that encode a fusion protein or cofactor provided herein.

In a first aspect, one or more polynucleotides provided herein encode a protein complex wherein the cofactor (*e.g*., IL-15Ra or an IL-15-binding fragment thereof) is covalently linked to the fusion protein (*e.g*., an immunocytokine comprising IL-15 or a functional fragment thereof), as described above. In certain embodiments, nucleic acid molecules provided herein comprise or consist of a nucleic acid sequence encoding a V_{H} or a V_{L} amino acid sequence fused to a cleavable peptide linker and a cytokine, notably IL-15, and a cofactor, such as IL-R15α, and optionally a linker, as provided herein. In other embodiments, nucleic acid molecules provided herein comprise or consist of a nucleic acid sequence encoding a V_{H} or a V_{L} amino acid sequence which is not fused to additional sequences. In yet other embodiments, the nucleic acid molecules provided herein comprise or consist of combinations of a nucleic acid sequence encoding a V_{H} or a V_{L} amino acid sequence fused to a cleavable peptide linker and a cytokine, notably IL-15, and a cofactor, such as IL-R15α, and optionally a linker, and of a nucleic acid sequence encoding a V_{H} or a V_{L} amino acid sequence which is not fused to additional sequences. Preferably, a nucleic acid sequence encoding a V_{H} amino acid sequence fused to a cleavable peptide linker and a cytokine, notably IL-15, and a cofactor, such as IL-R15α, and optionally a linker, is combined with a nucleic acid sequence encoding a V_{L} amino acid sequence which is not fused to additional sequences. Alternatively, said combination comprises a nucleic acid sequence encoding a V_{L} amino acid sequence fused to a cleavable peptide linker and a cytokine, notably IL-15, and a cofactor, such as IL-R15α, and optionally a linker, and a nucleic acid sequence encoding a V_{H} amino acid sequence which is not fused to additional sequences.

In another aspect, the fusion protein and the cofactor encoded by the polynucleotides provided herein interact through non-covalent interactions. In certain embodiments, nucleic acid molecules provided herein comprise or consist of a nucleic acid sequence encoding a V_{H} or a V_{L} amino acid sequence fused to a cleavable peptide linker and a cytokine, as provided herein. In other embodiments, nucleic acid molecules provided herein comprise or consist of a nucleic acid sequence encoding a V_{H} or a V_{L} amino acid sequence which is not fused to additional sequences. In yet other embodiments, nucleic acid molecules provided herein comprise or consist of a nucleic acid sequence encoding a cofactor as described herein. In yet other embodiments, the nucleic acid molecules provided herein comprise or consist of combinations of a nucleic acid sequence encoding a V_{H} or a V_{L} amino acid sequence fused to a cleavable peptide linker and a cytokine, a nucleic acid sequence encoding a VH or a VL amino acid sequence which is not fused to additional sequences, and a nucleic acid sequence encoding a cofactor.

Preferably, a nucleic acid sequence encoding a V_{H} amino acid sequence fused to a cleavable peptide linker and a cytokine is combined with a nucleic acid sequence encoding a V_{L} amino acid sequence which is not fused to additional sequences and with a nucleic acid sequence encoding the cofactor. Alternatively, said combination comprises a nucleic acid sequence encoding a V_{L} amino acid sequence fused to a cleavable peptide linker and a cytokine and a nucleic acid sequence encoding a V_{H} amino acid sequence which is not fused to additional sequences and a nucleic acid sequence encoding the cofactor.

### RECOMBINANT EXPRESSION OF A PROTEIN COMPLEX

A variety of expression systems may be used to express the present protein complex as described herein. The skilled person will be able to choose an expression system appropriate for expressing the immunocytokine and cofactor described herein, either as a fusion (*i.e.* when the cofactor is covalently linked to the immunocytokine) or by coexpression (*i.e.* when the immunocytokine and the cofactor are bound non-covalently). The expression of the cofactor with the immunocytokine, be they covalently or non-covalently bound, leads to higher yields and lower levels of aggregation (*i.e.,* a higher purity) of the immunocytokine than expression of the immunocytokine alone.

In one aspect, such expression systems represent vehicles by which the coding sequences of interest may be produced and subsequently purified, but also represent cells which may, when transiently transfected with the appropriate nucleotide coding sequences, express the protein complex *in situ.*

The invention provides vectors comprising the polynucleotides described herein. In one embodiment, the vector contains a polynucleotide encoding a heavy chain of the immunocytokine provided herein, wherein said heavy chain is fused or not to a cleavable peptide linker and a cytokine. In this embodiment, the immunocytokine may be fused or not to the cofactor. In another embodiment, said polynucleotide encodes the light chain of an immunocytokine of the invention, wherein said light chain is fused or not to a cleavable peptide linker and a cytokine. In this embodiment, the immunocytokine may be fused or not to the cofactor. In yet another embodiment, the polynucleotide encodes the cofactor. The present disclosure also provides vectors comprising polynucleotide molecules encoding fusion proteins, modified antibodies, antibody fragments, and probes thereof.

In order to express the heavy and/or light chain of an immunocytokine and/or the cofactor disclosed herein, the polynucleotides encoding said heavy and/or light chains and/or cofactor are inserted into expression vectors such that the genes are operatively linked to transcriptional and translational sequences.

"Operably linked" sequences include both expression control sequences that are contiguous with the gene of interest and expression control sequences that act in trans or at a distance to control the gene of interest. The term "expression control sequence" as used herein refers to polynucleotide sequences which are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (*i.e*., Kozak consensus sequence); sequences that enhance protein stability; and when desired, sequences that enhance protein secretion. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is essential for expression and processing and can also include additional components whose presence is advantageous, for example, leader sequences and fusion partner sequences.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammlian vectors) can be integrated into the genome of a host cell upon introduction into the host cell, and thereby are replicated along with the host genome.

Certain vectors are capable of directing the expression of genes to which they are operatively linked. Such vectors are referred to herein as "recombinant expression vectors" (or simply, "expression vectors"). In general, expression vectors of utility in recombinant DNA techniques are in the form of plasmids. In the present specification, "plasmid" and "vector" may be used interchangeably as the plasmid is the most commonly used form of vector. However, the invention is intended to include such forms of expression vectors, such as bacterial plasmids, YACs, cosmids, retrovirus, EBV-derived episomes, and all the other vectors that the one skilled in the art will know to be convenient for ensuring the expression of the heavy and/or light chains and/or cofactor of the invention. The skilled man will realise that the polynucleotides encoding the heavy and the light chains and the cofactor can be cloned into different vectors or in the same vector. In a preferred embodiment, said polynucleotides are cloned into at least two vectors. In particular, when the immunocytokine and the cofactor are expressed as a fusion, said polynucleotides are preferably cloned in at least two vectors. When the immunocytokine and the cofactor are co-expressed, *i.e.* not as a fusion, said polynucleotides are preferably cloned in at least one, preferably at least two, preferably three vectors.

Polynucleotides of the invention and vectors comprising these molecules can be used for the transformation of a suitable host cell. The term "host cell", as used herein, is intended to refer to a cell into which a recombinant expression vector has been introduced in order to express the present protein complex. It should be understood that such terms are intended to refer not only to the particular subject cell but also to the progeny of such a cell. Because certain modifications may occur in succeeding generations due to either mutation or environmental influences, such progeny may not, in fact, be identical to the parent cell, but are still included within the scope of the term "host cell" as used herein.

Transformation can be performed by any known method for introducing polynucleotides into a cell host. Such methods are well known of the man skilled in the art and include dextran-mediated transformation, calcium phosphate precipitation, polybrene-mediated transfection, protoplast fusion, electroporation, encapsulation of the polynucleotide into liposomes, biolistic injection and direct microinjection of DNA into nuclei.

The host cell may be co-transfected with two or more expression vectors, including the vector expressing the protein complex described herein. In particular, the other expression vectors may encode enzymes involved in post-translational modifications, such as glycosylation. For example, a host cell can be transfected with a first vector encoding protein complex as described above, and a second vector encoding a glycosyltransferase polypeptide. Alternatively, the host cell can be transformed with a first vector encoding a protein complex, a second vector encoding a glycosyltransferase, as described above, and a third vector encoding another glycosyltransferase. Mammalian cells are commonly used for the expression of a recombinant therapeutic immunoglobulins, especially for the expression of whole recombinant antibodies. For example, mammalian cells such as HEK293 or CHO cells, in conjunction with a vector, containing the expression signal such as one carrying the major intermediate early gene promoter element from human cytomegalovirus, are an effective system for expressing the present protein complex (Foecking et al., 1986, Gene 45:101-105; Cockett et al., 1990, Bio/Technology 8(7):662-667).

It is also possible to select a host cell which modulates the expression of the inserted sequences or modifies and processes the gene product in the specific fashion desired. Such modifications (*e.g*., glycosylation) and processing of protein products may be important for the function of the protein. Different host cells have features and specific mechanisms for the post-translational processing and modification of proteins and gene products. Appropriate cell lines or host systems are chosen to ensure the correct modification and processing of the expressed proteins of interest (*i.e.,* the immunocytokine and the cofactor provided herein). Hence, eukaryotic host cells which possess the cellular machinery for proper processing of the primary transcript, glycosylation of the gene product may be used. Such mammalian host cells include, but are not limited to, CHO, COS, HEK293, NS/0, BHK, Y2/0, 3T3 or myeloma cells (all these cell lines are available from public depositories such as the Collection Nationale des Cultures de Microorganismes, Paris, France, or at the American Type Culture Collection, Manassas, VA, U.S.A.).

For long-term, high-yield production of recombinant proteins, stable expression is preferred. In one embodiment of the invention, cell lines which stably express the immunocytokine and the cofactor may be engineered. Rather than using expression vectors which contain viral origins of replication, host cells are transformed with DNA under the control of the appropriate expression regulatory elements, including promoters, enhancers, transcription terminators, polyadenylation sites, and other appropriate sequences known to the person skilled in art, and a selectable marker. Following the introduction of the foreign DNA, engineered cells may be allowed to grow for one to two days in an enriched media, and then are moved to a selective media. The selectable marker on the recombinant plasmid confers resistance to the selection and allows cells to stably integrate the plasmid into a chromosome and be expanded into a cell line. Other methods for constructing stable cell lines are known in the art. In particular, methods for site-specific integration have been developed. According to these methods, the transformed DNA under the control of the appropriate expression regulatory elements, including promoters, enhancers, transcription terminators, polyadenylation sites, and other appropriate sequences is integrated in the host cell genome at a specific target site which has previously been cleaved (Moele et al., Proc. Natl. Acad. Sci. U.S.A., 104(9): 3055-3060; US 5,792,632; US 5,830,729; US 6,238,924; WO 2009/054985; WO 03/025183; WO 2004/067753, all of which are incorporated herein by reference).

A number of selection systems may be used, including but not limited to the Herpes simplex virus thymidine kinase (Wigler et al., Cell 11:223, 1977), hypoxanthine-guanine phosphoribosyltransferase (Szybalska et al., Proc. Natl. Acad. Sci. USA 48:202, 1992), glutamate synthase selection in the presence of methionine sulfoximide (Adv Drug Del Rev, 58:671, 2006, and website or literature of Lonza Group Ltd.) and adenine phosphoribosyltransferase (Lowy et al., Cell 22:817, 1980) genes in tk, hgprt or aprt cells, respectively. Also, antimetabolite resistance can be used as the basis of selection for the following genes: dhfr, which confers resistance to methotrexate (Wigler et al., Proc Natl Acad Sci USA 77: 357, 1980); gpt, which confers resistance to mycophenolic acid (Mulligan et al., Proc Natl Acad Sci USA 78: 2072, 1981); neo, which confers resistance to the aminoglycoside, G-418 (Wu et al., Biotherapy 3: 87, 1991); and hygro, which confers resistance to hygromycin (Santerre et al., Gene 30: 147, 1984). Methods known in the art of recombinant DNA technology may be routinely applied to select the desired recombinant clone, and such methods are described, for example, in Ausubel et al., eds., Current Protocols in Molecular Biology, John Wiley & Sons (1993). The expression levels of an immunocytokine and/or cofactor can be increased by vector amplification. When a marker inthe vector system expressing an immunocytokine and/or cofactor is amplifiable, an increase in the level of inhibitor present in the culture will increase the number of copies of the marker gene. Since the amplified region is associated with the gene encoding the immunocytokine and/or cofactor of interest, production of said immunocytokine and/or cofactor will also increase (Crouse et al., Mol Cell Biol 3: 257-266, 1983). Alternative methods of expressing the gene of the invention exist and are known to the person of skills in the art. For example, a modified zinc finger protein can be engineered that is capable of binding the expression regulatory elements upstream of the gene of the invention; expression of the said engineered zinc finger protein (ZFN) in the host cell of the invention leads to increases in protein production (see *e.g*., Reik et al., Biotechnol. Bioeng., 97(5), 1180-1189, 2006). Moreover, ZFN can stimulate the integration of a DNA into a predetermined genomic location, resulting in high-efficiency site-specific gene addition (Moehle et al, Proc Natl Acad Sci USA 104:3055-3060, 2007).

The protein complex of the invention may be prepared by growing a culture of the transformed host cells under culture conditions necessary to express the desired immunocytokine and cofactor, as a fusion or not as described above. The resulting expressed complex may then be purified from the culture medium or cell extracts. Soluble forms of the complex can be recovered from the culture supernatant. It may then be purified by any method known in the art for purification of an immunoglobulin molecule, for example, by chromatography (*e.g*., ion exchange, affinity, particularly by Protein A affinity for Fc, and so on), centrifugation, differential solubility or by any other standard techniques for the purification of proteins. Suitable methods of purification will be apparent to a person of ordinary skills in the art.

### PHARMACEUTICAL COMPOSITIONS

When addressed to the tumour site, the immunocytokines provided herein in a complex with a cofactor are cleaved, thus releasing a cytokine such as IL-15, which has anti-tumour activity. In addition, some of the antibody moieties are capable of inducing ADCC and/or CDC responses and/or have an intrinsic anti-tumour activity. It will thus be appreciated by the skilled person that the protein complexes of immunocytokines and cofactors provided herein are useful in the treatment of metastatic tumours and diseases such as cancer.

The terms "treating" or "treatment" refer to administering or the administration of a composition described herein in an amount, manner, and/or mode effective to improve a condition, symptom, or parameter associated with a disorder or to prevent progression or exacerbation of the disorder (including secondary damage caused by the disorder) to either a statistically significant degree or to a degree detectable to one skilled in the art.

Another aspect of the invention relates to pharmaceutical compositions of the immunocytokines in a complex with a cofactor, as described herein.

The pharmaceutical composition of the invention may contain, in addition to the protein complex of immunocytokine and cofactor, various diluents, fillers, salts, buffers, stabilizers, solubilisers, and other materials well known in the art.

As used herein, "pharmaceutically acceptable carrier" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of media and agents for pharmaceutically active substances is well known in the art. As detailed below, additional active compounds can also be incorporated into the compositions, such as anti-cancer and/or anti-angiogenesis agents; in particular, the additional active compound can be an anti-angiogenic agent, a chemotherapeutic agent, or a low-molecular weight agent. A typical pharmaceutical composition for intravenous infusion could be made up to contain 250 ml of sterile Ringer's solution, and 100 mg of the protein complex. Actual methods for preparing parenterally administrable compounds will be known or apparent to those skilled in the art and are described in more detail in for example, Remington's Pharmaceutical Science, 17th ed., Mack Publishing Company, Easton, Pa. (1985), and the 18^{th} and 19th editions thereof, which are incorporated herein by reference.

The protein complex of immunocytokine and cofactor present in the composition preferably is formulated in an effective amount. An "effective amount" refers to an amount effective, at dosages and for periods of time necessary, to achieve the desired result, such as induction of apoptosis in tumour cells. A "therapeutically effective amount" means an amount sufficient to influence the therapeutic course of a particular disease state. A therapeutically effective amount is also one in which any toxic or detrimental effects of the agent are outweighed by the therapeutically beneficial effects.

For therapeutic applications, the protein complex is administered to a mammal, preferably a human, in a pharmaceutically acceptable dosage form such as those discussed above, including those that may be administered to a human intravenously as a bolus or by continuous infusion over a period of time, by intramuscular, intraperitoneal, intracerebrospinal, subcutaneous, intraarticular, intrasynovial, intrathecal, oral, topical, or inhalation routes. The protein complex is also suitably administered by intratumoural, peritumoural, intralesional, or perilesional routes, to exert local as well as systemic therapeutic effects. The intraperitoneal route is expected to be particularly useful, for example, in the treatment of ovarian tumours. Dosage regimens may be adjusted to provide the optimum response. For example, a single bolus may be administered, several divided doses may be administered over time, or the dose may be proportionally reduced or increased. One skilled in the art in the field of preparing formulations can readily select the proper form and mode of administration depending upon the particular characteristics of the product selected, the disease or condition to be treated, the stage of the disease or condition and other relevant circumstances

The present compositions can be administered to a subject to effect cell growth activity in a subject. As used herein, the term "subject" is intended to include living organisms in which apoptosis can be induced, and specifically includes mammals, such as rabbits, dogs, cats, mice, rats, monkey transgenic species thereof, and preferably humans. A human subject may be called a "patient".

The effectiveness of the protein complex of immunocytokine and cofactor in preventing or treating cancer may be improved by administering said protein complex serially or in combination with another agent that is effective for those purposes, such as tumour necrosis factor (TNF), an antagonist capable of inhibiting or neutralising the angiogenic activity of acidic or basic fibroblast growth factor (FGF), platelet-derived growth factor (PDGF), or hepatocyte growth factor (HGF), an antagonist capable of inhibiting or neutralising the coagulant activities of tissue factor, protein C, or protein S (see WO 91/01753), an antagonist such as an antibody capable of binding to HER2 receptor (see US 5,772,997), or one or more conventional therapeutic agents such as, for example, alkylating agents, folic acid antagonists, anti-metabolites of nucleic acid metabolism, antibiotics, pyrimidine analogs, 5-fluorouracil, cisplatin, purine nucleosides, amines, amino acids, triazol nucleosides, or corticosteroids.

In addition, the pharmaceutical composition may also comprise another agent which is capable of modulating immune cell, notably T cell or monocyte, activation and/or function. In particular, the pharmaceutical composition may further comprise a therapeutically effective amount of an antagonist to a co-inhibitory molecule. In some embodiments, the co-inhibitory molecule is selected from the group consisting of CD86, CD80, PDL-1, PDL-2, CTLA-4, PD1, LAG3, BTNL2, B7-H3, B7-H4, a butyrophilin, CD48, CD244, TIM-3, CD200R, CD200, CD160, BTLA, HVEM, LAIR1, TIM1, Galectin 9, TIM3, CD48, 2B4, CD155, CD112, CD113 and TIGIT. The antagonist to the co-inhibitory molecule includes an antibody against the co-inhibitory molecule. It is recognised that antagonist to other co-inhibitory molecules are well known in the art, such as those described in Mercier et al., Frontiers in Immunology, 6:418 (2015), Kyi et al., FEBS Letters, 588:368-376 (2014) and Pardoll, Nature Reviews, 12:252-264 (2012). In some other embodiments, the pharmaceutical composition described herein further comprises a therapeutically effective amount of an agonist to a co-stimulatory molecule. In some embodiments, the co-stimulatory molecule is selected from the group consisting of CD154, TNFRSF25, GITR, 4-1BB, OX40, CD27, TMIGD2, ICOS, CD28, CD40, TL1A, GITRL, 41BBL, OX40L, CD70, HHLA2, ICOSL, a cytokine, LIGHT, HVEM, CD30, CD30L, B7-H2, CD80, CD86, CD40L, TIM4, TIM1, SLAM, CD48, CD58, CD155, CD112, DR3, GITR, CD2, and CD226. The agonist to the co-stimulatory molecule includes an agonistic antibody against the co-stimulatory molecule. It is recognised that agonists to co-stimulatory molecules are well known in the art, such as those described in Mercier et al., Frontiers in Immunology, 6:418 (2015), Kyi et al., FEBS Letters, 588:368-376 (2014) and Capece et al., J. Biomed. Biotechnol. 2012:926321, (2012).

In another aspect of the invention, the administration is combined with an administration of therapeutically effective amount of chemotherapeutic agent, such as for example, taxol (paclitaxel) or taxotere (docetaxel).

Chemotherapeutic agents include without any limitations, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and antifolate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; and cell cycle signalling inhibitors. In addition, the methods of the invention can be combined with another anti-cancer treatment, anti-angiogenic agent, or chemotherapeutic agent or radiation therapy. A preferred example is docetaxel or taxotere. Other examples include, gemcitabine, cisplatin diterpenoids and vinca alkaloids, paclitaxel, vinblastine, vincristine, and vinorelbine, carboplatin, cyclophosphamide, melphalan, and chlorambucil, busulfan, carmustine, dacarbazine, cyclophosphamide, melphalan, chlorambucil, busulfan, carmustine, dacarbazine, anti-neoplastic agents including, but not limited to, actinomycins such as dactinomycin, anthrocyclins such as daunorubicin and doxorubicin, bleomycins, epipodophyllotoxins, etoposide and teniposide; antimetabolite neoplastic agents, 5-fluorouracil, methotrexate, cytarabine, mecaptopurine, thioguanine, camptothecins, irinotecan HCl, and topotecan HCl.

A variety of different chemotherapeutic agents or anti-cancer polypeptides can also be selected. Information sources such as www.clinicaltrials.gov, www.ncbi.nlm.nih and www.drugs.com, include references to polypeptides and agents that can be selected.

### METHODS OF MODULATING AN IMMUNE RESPONSE

The protein complex provided herein can be used to modulate an immune response, since a cytokine is released when the linker is cleaved and may then activate specific immune cells. For example, IL-15 regulates the expansion of lymphocyte subsets; notably, IL-15 primarily stimulates the proliferation and cytotoxic functions of CD8⁺ T cells and NK cells. Indeed, administration of the protein complex disclosed herein results in enhance proliferation of NK cells in the tumour microenvironment. In addition, the present immunocytokine is able to stimulate T cell activation in vivo when cleaved, but not in the uncleaved form.

The present disclosure thus relates to the protein complex disclosed herein or a pharmaceutical composition comprising said complex for use in stimulating an immune response in a subject.

The present disclosure also relates to a method for stimulating an immune response in a subject, comprising administering to the subject the protein complex disclosed herein or a pharmaceutical composition comprising said complex.

Also provided herein is the use of the protein complex disclosed herein or a pharmaceutical composition comprising said complex for making a medicament for stimulating an immune response in a subject.

The term "immune response" as used herein is meant to refer to the process whereby immune cells are stimulated and recruited from the blood to lymphoid as well as non-lymphoid tissues via a multifactorial process that involves distinct adhesive and activation steps. Activation conditions cause the release of cytokines, growth factors, chemokines and other factors, upregulate expression of adhesion and other activation molecules on the immune cells, promote adhesion, morphological changes, and/or extravasation concurrent with chemotaxis through the tissues, increase cell proliferation and cytotoxic activity, stimulate antigen presentation and provide other phenotypic changes including generation of memory cell types. Immune response if also meant to refer to the activity of immune cells to suppress or regulate inflammatory or cytotoxic activity of other immune cells. Exemplary immune responses include B cell responses (e.g., antibody production) T cell responses (e.g., cytokine production, and cellular cytotoxicity) and activation of cytokine responsive cells, e.g., NK cells and macrophages.

The protein complexes described herein are useful to expand lymphocyte subsets, such as specific T/NK subsets. The present invention thus relates to the use of a product of the invention as an agent for expanding one or several lymphocyte populations, such as NK cells, NK-T cells, CD8⁺ T cells, and to the adjuvants, compositions and kits intended for such a use, including the pharmaceutical compositions and drugs, which comprise at least one product of the invention.

The present disclosure thus relates to the protein complex disclosed herein or a pharmaceutical composition comprising said complex for use in stimulating an immune response in a mammal, wherein the immune response involves expanding one or several lymphocyte populations, such as NK cells, NK-T cells, CD8⁺ T cells.

The present disclosure also relates to a method for stimulating an immune response in a mammal, wherein the immune response involves expanding one or several lymphocyte populations, such as NK cells, NK-T cells, CD8⁺ T cells, the method comprising administering to the mammal the protein complex disclosed herein or a pharmaceutical composition comprising said complex.

Activation of lymphocyte expansion by release of IL-15 from the protein complex as a means of stimulating an immune response is useful in therapy.

Stimulation of immune responses can be in the form of enhancing an existing immune response or eliciting an initial immune response. For example, enhancing an immune response through activation of lymphocyte expansion is useful in cases of infections with microbes, e.g., bacteria, viruses, or parasites, or in cases of immunosuppression. Activation of lymphocyte expansion by release of IL-15 from the protein complex can also be useful in the treatment of tumour immunity. Tumour cells (e.g., colorectal cancer, sarcoma, melanoma, lymphoma, leukaemia, neuroblastoma, or carcinoma) can be contacted *in vitro* or *in vivo* with the present protein complex, thereby releasing the cytokine, e.g. IL-15, from said complex. If desired, the tumour cells can also be transfected with other polypeptides which stimulate immune responses (e.g., antibodies against immune checkpoints such as PD-1, PD-L1, or VISTA).

### METHODS OF TREATMENT

The protein complexes of immunocytokines and cofactors and the pharmaceutical compositions described herein are especially useful in the treatment or prevention of several types of cancers.

Another aspect of the present disclosure thus relates to the protein complex of immunocytokine and cofactor described herein for use in the treatment of cancer. It also relates to a method of treating cancer in a subject in need thereof, comprising administering a therapeutically effective amount of the protein complex described herein to the subject.

The disclosure also relates to a pharmaceutical composition comprising the protein complex described herein for use in the treatment of cancer. It also relates to a method of treating cancer in a subject in need thereof, comprising administering a pharmaceutical composition comprising a therapeutically effective amount of the protein complex described herein to the subject.

The cancers which may be treated by this protein complex of immunocytokine and cofactor are cancers in which the antigen recognised by the antibody moiety of the immunocytokine is expressed. These cancers include (but not limited to) the following: carcinomas and adenocarcinomas, including that of the bladder, breast, colon, head-and-neck, prostate, kidney, liver, lung, ovary, pancreas, stomach, cervix, thyroid and skin, and including squamous cell carcinoma ; hematopoietic tumours of lymphoid lineage, including multiple myeloma, leukaemia, acute and chronic lymphocytic (or lymphoid) leukaemia, acute and chronic lymphoblastic leukaemia, B-cell lymphoma, T-cell lymphoma, non-Hodgkin lymphoma (e.g. Burkitt's lymphoma) ; hematopoietic tumours of myeloid lineage, including acute and chronic myelogenous (myeloid or myelocytic) leukaemia, and promyelocytic leukaemia; tumours of mesenchymal origin, including fibrosarcoma, osteosarcoma and rhabdomyosarcoma; tumours of the central and peripheral nervous system, including astrocytoma, neuroblastoma, glioma, and schwannomas; and other tumours, including melanoma, teratocarcinoma, xeroderma pigmentosum, keratoacanthoma, and seminoma, and other cancers yet to be determined in which said antigen is expressed. By cancers in which the antigen recognised by the antibody moiety of the immunocytokine is expressed, it is herein referred to cancers displaying high expression of said antigen, relative to the expression level of said antigen on a normal adult cell.

Other agents described above, *e.g.* anti-angiogenic agents or chemotherapeutic agents may be present in the composition being administered or may be administered separately. In one aspect of the invention, the administration is performed with the other active principle, either simultaneously, separately or sequentially over time. When the administration is performed simultaneously, the two active principles may be combined in a single pharmaceutical composition, comprising the two compositions, such as a tablet or a gel capsule. On the other hand, the two active principles may, whether or not they are administered simultaneously, be present in separate pharmaceutical compositions. To this end, the combination may be in the form of a kit comprising, on the one hand, the protein complex of immunocytokine and cofactor described herein and, on the other hand, the second active principle, the protein complex described herein and the second active principle being in separate compartments and being intended to be administered simultaneously, separately, or sequentially over time.

The present protein complex of immunocytokine and cofactor can be administered especially for treating cancer in combination with chemotherapy, protein therapy (*i.e*., using a therapeutic agent such as an antibody or recombinant protein), gene therapy, radiotherapy, immunotherapy, surgical intervention, or a combination of these. Long-term therapy is equally possible as is adjuvant therapy in the context of other treatment strategies, as described above.

Cytokines such as IL-15 are required for NK cell activation and proliferation. They are also capable of stimulating tumour-specific T cell responses that are highly-specific. For example, administration of IL-15 induces the selective activation and proliferation in CD8 T cells and NK cells, the very cell types most amenable to mediating anti-tumour responses (Waldmann, J Investig Dermatol Symp Proc. 16(1): S28-30, 2013).

In some embodiments, the protein complex of immunocytokine and cofactor can be used in a method of modulating immune cell function, mediated by binding of the cytokine moiety, preferably IL-15, of the immunocytokine. Preferably, the immune cell is a T cell or a monocyte. Such methods can include contacting the immune cell, preferably a T cell or a monocyte, with the immunocytokine of the protein complex described herein. In some embodiments, the method for modulating the immune cell (notably T cell or monocyte) function includes administering an effective amount of a composition comprising the protein complex of immunocytokine and cofactor provided herein to a subject. In some aspects, the T cell function that is modulated includes increasing T cell activation. Such T cell activation can further include increasing T cell proliferation. In some aspects, the monocyte function that is modulated includes increasing secretion of anti-cancer cytokines. Methods for assaying the modulation of an immune response are well known to one of skill in the art, and it is understood that a skilled artisan would be able to readily conduct such assays.

In some embodiments, a protein complex comprising immunocytokine and cofactor or a composition comprising this protein complex, including as described herein, can be used either alone or in combination with another compound or treatment. For example, in some embodiments, the other compound is an antagonist to a co-inhibitory molecule or an agonist to a co-stimulatory molecyle. In such embodiments, the combined therapy leads to reinvigoration or de novo activation of the immune system through activated T cells that is greater than the administration of either compound or treatment individually. This activation of the immune system will result in a highly beneficial physiological response in the treatment of cancer.

In some embodiments, the methods described herein can include administering a therapeutically effective amount of a protein complex comprising immunocytokine and cofactor, in combination with a therapeutically effective amount of an antagonist to a co-inhibitory molecule. In some embodiments, the co-inhibitory molecule is selected from the group consisting of CD86, CD80, PDL-1, PDL-2, CTLA-4, PD1, LAG3, BTNL2, B7-H3, B7-H4, a butyrophilin, CD48, CD244, TIM-3, CD200R, CD200, CD160, BTLA, HVEM, LAIR1, TIM1, Galectin 9, TIM3, CD48, 2B4, CD155, CD112, CD113 and TIGIT. The antagonist to the co-inhibitory molecule includes an antibody against the co-inhibitory molecule. It is recognised that antagonist to other co-inhibitory molecules are well known in the art, such as those described in Mercier et al., Frontiers in Immunology, 6:418 (2015), Kyi et al., FEBS Letters, 588:368-376 (2014) and Pardoll, Nature Reviews, 12:252-264 (2012). According to this embodiment, the invention relates to a protein complex comprising immunocytokine and cofactor for use in treatment of cancer as described above, said use further comprising the administration of an antagonist to a co-inhibitory molecule, wherein said co-inhibitory molecule is selected from the group consisting of CD86, CD80, PDL-1, PDL-2, CTLA-4, PD1, LAG3, BTNL2, B7-H3, B7-H4, a butyrophilin, CD48, CD244, TIM-3, CD200R, CD200, CD160, BTLA, HVEM, LAIR1, TIM1, Galectin 9, TIM3, CD48, 2B4, CD155, CD112, CD113 and TIGIT.

In some embodiments, the methods described herein can include administering a therapeutically effective amount of a protein complex of immunocytokine and cofactor in combination with a therapeutically effective amount of an agonist to a co-stimulatory molecule. In some embodiments, the co-stimulatory molecule is selected from the group consisting of CD154, TNFRSF25, GITR, 4-1BB, OX40, CD27, TMIGD2, ICOS, CD28, CD40, TL1A, GITRL, 41BBL, OX40L, CD70, HHLA2, ICOSL, a cytokine, LIGHT, HVEM, CD30, CD30L, B7-H2, CD80, CD86, CD40L, TIM4, TIM1, SLAM, CD48, CD58, CD155, CD112, DR3, GITR, CD2, and CD226. The agonist to the co-stimulatory molecule includes an agonistic antibody against the co-stimulatory molecule. It is recognised that agonists to co-stimuiatory molecules are well known in the art, such as those described in Mercier et al., Frontiers in Immunology, 6:418 (2015), Kyi et al., FEBS Letters, 588:368-376 (2014) and Capece et al., J. Biomed. Biotechnol. 2012:926321, (2012). According to this embodiment, the invention relates to a protein complex comprising immunocytokine and cofactor for use in treatment of cancer as described above, said use further comprising the administration of an agonist to a co-stimulatory molecule, wherein said co-stimulatory molecule is selected from the group consisting of CD154, TNFRSF25, GITR, 4-1BB, OX40, CD27, TMIGD2, ICOS, CD28, CD40, TL1A, GITRL, 41BBL, OX40L, CD70, HHLA2, ICOSL, a cytokine, LIGHT, HVEM, CD30, CD30L, B7-H2, CD80, CD86, CD40L, TIM4, TIM1, SLAM, CD48, CD58, CD155, CD112, DR3, GITR, CD2, and CD226.

The examples that follow are merely exemplary of the scope of this invention and content of this disclosure. One skilled in the art can devise and construct numerous modifications to the examples listed below without departing from the scope of this invention.

### EXAMPLES

### EXAMPLE 1: Constructions used

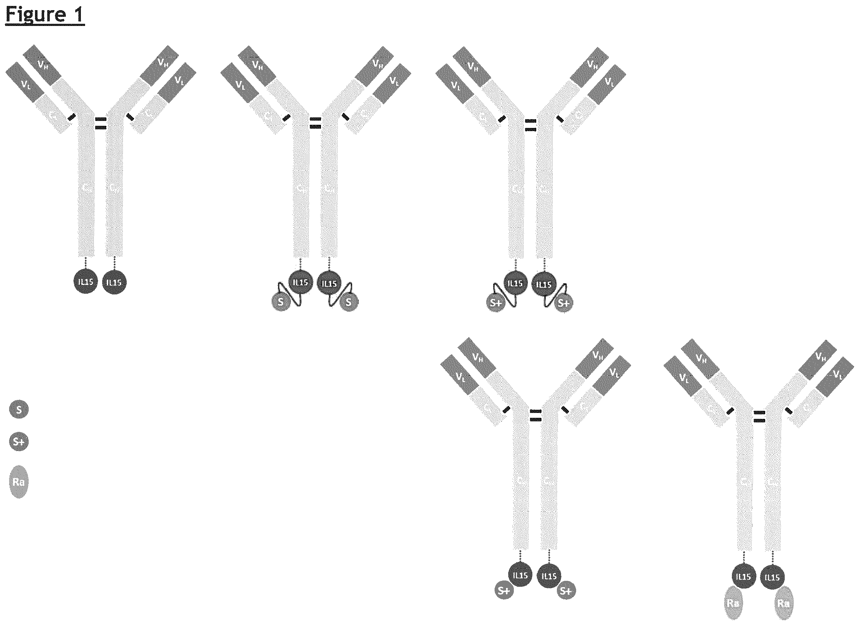

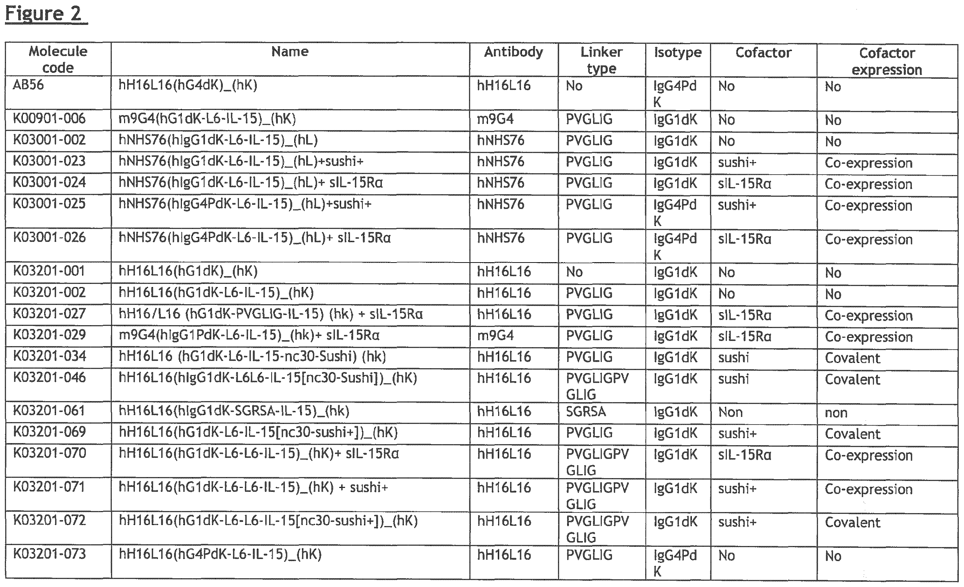

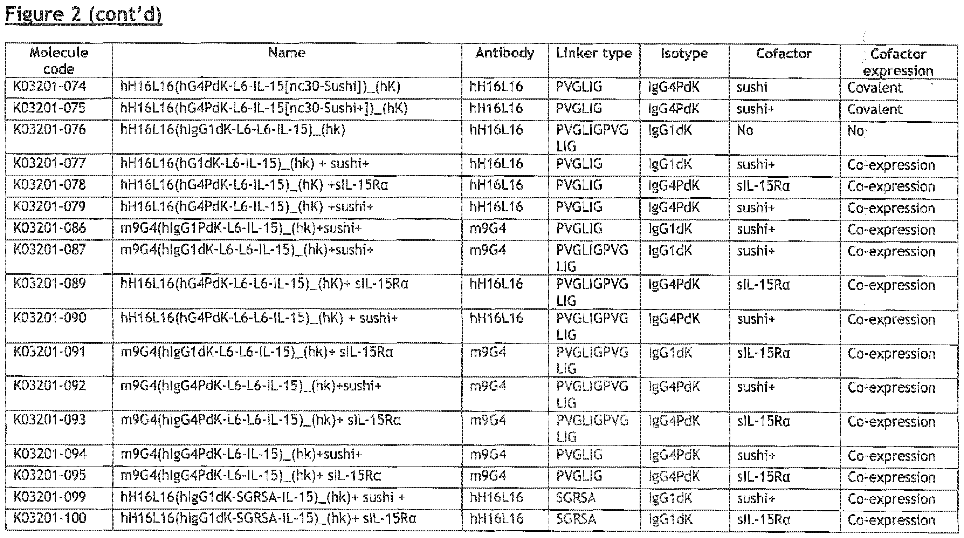

A schematic representation of the constructions used in the present study is shown in **Fig. 1****.** These constructions are listed in **Fig. 2****.**

### 1. Sequences

**Table 1: Antibody constant regions**

| SEQ ID NO. | Light chain: constant region | | Heavy chain: constant region | |
|---|---|---|---|---|
| | Protein | DNA | Protein | DNA |
| Human κ constant | 1 | 3 | | |
| Human λ constant | 2 | 4 | | |
| hIgG1dK | | | 17 | 19 |
| hIgG4Pdk | | | 18 | 20 |

**Table 2: Antibody variable regions**

| SEQ ID NO. | Light chain: variable region | | Heavy chain: variable region | |
|---|---|---|---|---|
| | protein | DNA | protein | DNA |
| hH16L16 | 5 | 8 | 21 | 24 |
| m9G4 | 6 | 9 | 22 | 25 |
| hNHS76 | 7 | 10 | 23 | 26 |

**Table 3: Full-length antibody chains**

| SEQ ID NO. | Light chain: full-length | | Heavy chain: full-length | |
|---|---|---|---|---|
| | protein | DNA | protein | DNA |
| hH16L16_LhK | 11 | 14 | | |
| m9g4_LhK | 12 | 15 | | |
| hNHS76_LhL | 13 | 16 | | |
| hH16L16_IgG1dK | | | 27 | 33 |
| hH16L16_IgG4PdK | | | 28 | 34 |
| m9G4_IgG1dK | | | 29 | 35 |
| m9G4_IgG4PdK | | | 30 | 36 |
| hNHS76_IgG1dK | | | 31 | 37 |
| hNHS76_IgG4PdK | | | 32 | 38 |

**Table 4: Linkers**

| SEQ ID NO. | linkers | |
|---|---|---|
| | protein | DNA |
| PVGLIG | 39 | 44 |
| PVGLIGPVGLIG | 40 | 45 |
| nc30 | 41 | 46 |
| SGRS | 42 | 47 |
| SGRSA | 43 | 48 |

**Table 5: IL-15**

| SEQ ID NO. | protein | DNA |
|---|---|---|
| IL-15 | 49 | 50 |

**Table 6: Cofactors**

| SEQ ID NO. | Cofactors | |
|---|---|---|
| | protein | DNA |
| sushi | 51 | 54 |
| sushi+ | 52 | 55 |
| sIL-15Rα | 53 | 56 |

**Table 7: ICC heavy chains**

| SEQ ID NO. | C-term Heavy Chain - cytokine | |
|---|---|---|
| | protein | DNA |
| Single linker | | |
| PL51_hH16L16_HhG1dK_L6_I L-15IL-15 | 57 | 64 |
| PL63_hH16L16_HhG4PdK_L6_ _IL-15IL-15 | 58 | 65 |
| PL147_p3.4_m9G4_HhG1dK_ L6_IL-15 | 59 | 68 |
| PL141_p3.4_m9G4_HhG4PdK _L6_IL-15 | 60 | 69 |
| PL146_hNHS76HhG1dK_L6_I L-15 | 61 | 66 |
| PL142_hNHS76_HhG4PdK_L6 _IL-15 | 62 | 67 |
| PL149_hH16L16_HhG1dK_SG RSA_IL-15 | 63 | 70 |

| Double linker | | |
|---|---|---|
| PL34_hH16L16_HhG1dK_L6-L6_IL-15 | 71 | 77 |
| PL140_hH16L16_HhG4PdK_L 6-L6_IL-15 | 72 | 78 |
| PL166_p3.4_m9G4_HhG1dK_ L6_L6_IL-15 | 73 | 79 |
| PL167_p3.4_m9G4_HhG4PdK _L6_L6_IL-15 | 74 | 80 |
| PL164_hNHS76_HhG1dK_L6-L6_IL-15 | 75 | 81 |
| PL165_hNHS76_HhG4PdK_L6 -L6_IL-15 | 76 | 82 |

**Table 8: Protein fusion of ICC and sushi or sushi+**

| SEQ ID NO. | C-term Heavy Chain - cytokine | |
|---|---|---|
| | protein | DNA |
| Single linker | | |
| PL44_hH16L16_HhG1dK_L6_I L-15[nc30-sushi] | 83 | 87 |
| PL64_hH16L16_HhG4PdK_L6 _IL-15[nc30-sushi] | 84 | 88 |
| PL35_hH16L16_HhG1dK_PVG LIG_IL-15[nc30-sushi+] | 85 | 89 |
| PL65_hH16L16_HhG4PdK_PV GLIG_IL-15[nc30-sushi+] | 86 | 90 |

| Double linker | | |
|---|---|---|
| PL45_hH16L16_HhG1dK_L6L 6_IL-15[nc30-sushi] | 91 | 93 |
| PL62_hH16L16_HhG1dK_L6-L6_IL-15[nc30-sushi+] | 92 | 94 |

### 2. Obtention of the Immunocytokines (ICC)

Sequences coding for the complete fusion proteins (antibody-linker-cytokine) were cloned into pCDNA3.4 vectors (Thermo FisherScientific) using the *HindIII*/*BamHI* restriction sites, including a signal peptide (METDTLLLWVLLLWVPGSTG Thermo FisherScientific). Fusion proteins were obtained by transient protein expression in Expi HEK293 cells (Thermo FisherScientific) grown to a density of 2.5 x 10⁶ cells/ml in Expi293 Expression Medium (Thermo FisherScientific) and co-transfected with 1.25 µg/ml DNA (HC/LC: 1/1 w/w) using polyethyleneimine (PEI, Polyscience, DNA/PEI ratio: 1/4). 2 mM valproic acid (VPA, Sigma-Aldrich) was then added 3 hours post transfection. Supernatants containing the produced fusion proteins were harvested 6 days post-transfection. The fusion protein yield was measured in each supernatant.

### 3. Purification and characterisation of ICCs

Proteins were purified by affinity chromatography on Protein A-Sepharose and formulated by overnight dialysis against 25 mM sodium citrate, 150 mM NaCl, 6% Saccharose pH 5.5 or 25 mM His/His-HCl, 150 mM NaCl, pH 6.5.

Purified ICCs were analysed by SDS-PAGE and Size Exclusion chromatography (SEC). The SEC running buffer corresponded to the formulation buffer of the analysed ICC. At the research level SEC acceptance criterium is 80% monomers.

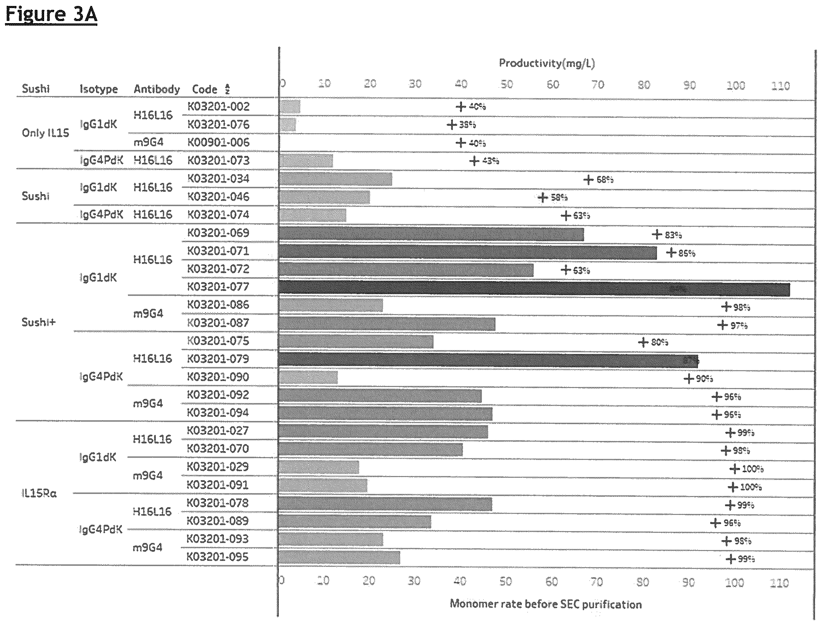

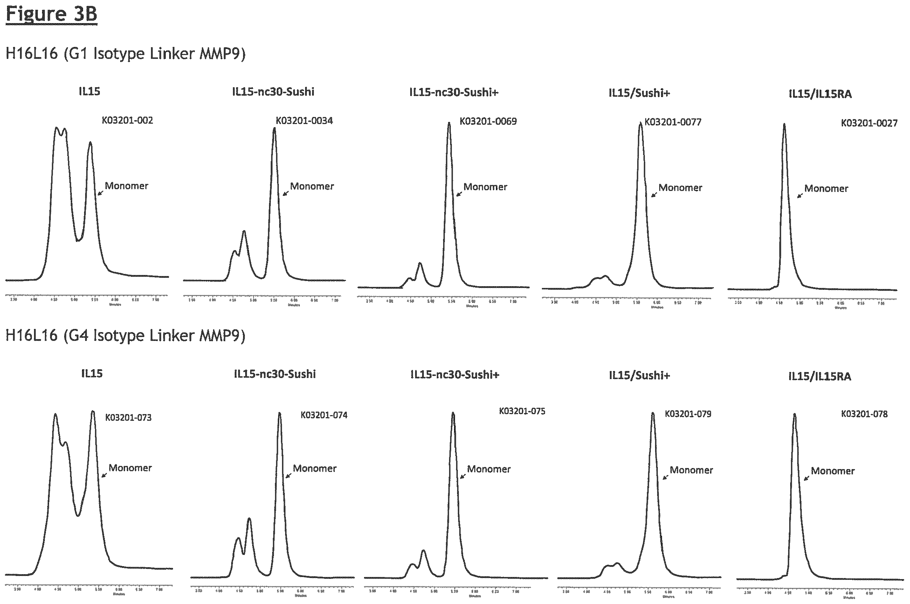

As illustrated in **Fig. 3**, expression of fusion proteins with a cofactor leads to a higher productivity and a lower level of aggregates (*i.e.,* a higher level of monomers). For example, K03201-076 is a fusion protein consisting of the hH16L16 antibody fused to two copies of the L6 linker (PVGLIG) and IL-15. Expression of this protein does not go above 10 mg/L and the monomer rate is 38%. By contrast, expression of sushi+ with this ICC, either as a fusion protein (K03201-072) or through coexpression (K03201-071), results in significant increases in productivity (ca. 60 mg/L and 80 mg/L, respectively) and monomer levels (63% and 86%, respectively). Similar results were obtained with both sushi (K03201-046) and ILR15a (K03201-070).

Thus, the expression of the fusion protein is significantly enhanced by the cofactor.

### EXAMPLE 2: Attenuation of IL-15 when fused to an antibody

To evaluate and compare the biological activity ofIL-15, when linked to the ICC or after cleavage by MMP9, a bioluminescent cell-based assay designed to measure IL-15 stimulation was performed.

### 1. Materials and Methods

### 1.1.Materials and Reagents

Recombinant human pro-MMP-9 was purchased from R&D Systems and activated with 1 mM 4-Aminophenylmercuric acetate (APMA) in buffer containing 50 mM Tris, 150 mM NaCl, 10 mM CaCl2, 0.05% Brij-35 (w/v), pH7.5. APMA was then removed using Zeba™ Spin Desalting Columns (ThermoFisher Scientific) and APMA-free MMP-9 was immediately stored at -80°C until needed. Recombinant human IL-15 was purchased from PeproTech.

IL-15 activity was monitored using the IL-15 Bioassay (Promega). This luciferase reporter bioassay consists of a genetically engineered cell line, which comprises the full cytokine signalling pathway and a reporter gene. This cell emits luminescence upon binding of IL-15 to its receptor. This luminescence can then be detected and quantified with a luminometer. In the absence of IL-15, no signalling occurs downstream of IL-15R and a luminescent signal is not generated.

### 1.2. Methods

The activity of different immunocytokines was assessed. All samples were incubated for 24 hours in presence (+MMP-9) or absence (-MMP-9) of recombinant hMMP-9 in an assay buffer containing 50 mM Tris, 150 mM NaCl, 10 mM CaCl2 pH 7.5 with one molecule of MMP9 for 12 molecules of immunocytokine. Cleavage efficiency was controlled by SDS-PAGE analysis and samples were immediately stored at -20°C until processing. IL-15 Bioassay cells were treated with either cleaved or uncleaved fusion proteins and controls for 6 hours at 37°C according to the manufacturers protocol.

Detection reagent was added, and luminescence intensity was recorded with a microplate reader (Infinite M1000Pro, Tecan). Data analysis was performed with Prism 7.01 software (GraphPad).

### 2. Results

The results of the IL-15-induced luminescence emission for the various immunocytokines tested are presented in **Fig. 4** as a function of IL-15 concentration.

All the molecules tested comprised IL-15 covalently linked to an antibody (H16L16, m9G4, or NHS76) through one or two copies of the linker L6 (PVGLIG). These molecules were expressed with or without different forms of IL15-Rα (sushi, sushi+ or the entire sIL-15Rα). These cofactors were either covalently linked to the ICC or co-expressed and co purified with the ICC.

In the absence of MMP-9, the evaluated ICC had no significant activity on IL-15 signalling pathway. None of the evaluated fusion proteins induced light emission. By contrast, pre-treatment of the same molecules with MMP-9 results in recovered cytokine activity through IL-15-induced light emission. The same results were obtained independently of the antibody, the number of linker copies, or of the cofactor. In addition, whether the cofactor is covalently linked or coexpressed with the tested fusion protein does not influence neither IL-15 attenuation in the absence of MMP-9 nor the induction of IL-15 activity in the presence of MMP-9.

It appears thus clearly from these experiments that IL-15 in each of the various configurations tested shows a highly attenuated cytokine activity when fused to an antibody. After cleavage of the fusion proteins by MMP-9, IL-15 is liberated in its active form and is able to fulfil its biological activity.

### EXAMPLE 3: In vitro evaluation of ICC constructs in an NK cell assay

Since IL-15 is critical for the development and expansion of NK cells, biological activity of the IL-15 moiety of the ICC, with or without cleavage by MMP9, - was measured and evaluated in an assay designed to measure NK cell activation.

### 1. Methods:

Murine NK cells were purified from spleen of Balb/c byJ mouse (Charles River), using the NK Cell Isolation Kit Mouse and following the manufacturer's instructions (Miltenyi).

After purification, NK cells were plated at the density of 50 000 cells per well in a 96 multi well plate and incubated with either IL-15 (100ng/ml) or ICC (dose equivalent to IL-15 100ng/ml), pre-incubated or not with MMP9. After 72h incubation, IFNγ was dosed in the supernatant and percentage of NKp46 positive cells as well as CD69 expression were evaluated on NK cells by flow cytometry.

### 2. Results:

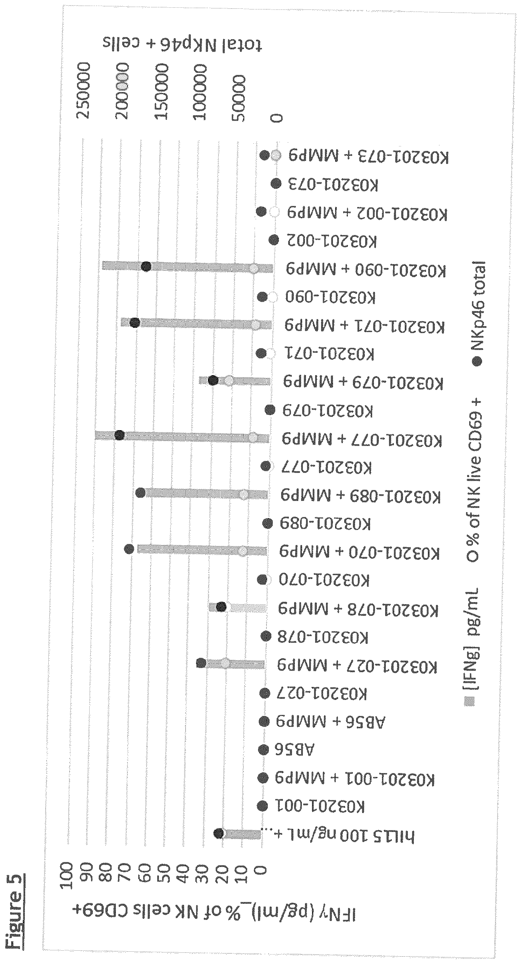

The results of the NK activation assay for the various immunocytokines tested are displayed in **Fig. 5****.** In particular, the % of CD69+ NK cells and the level of IFNγ produced were determined for each fusion protein, as well as the total number of NKp46 positive cells.

Each of the tested fusion proteins contained IL-15 covalently linked to an antibody (H16L16, m9G4, or NHS76) through one or two copies of the linker L6 (PVGLIG). These molecules were expressed with or without different forms of IL15-Ra (sushi+ or the entire sIL-15Rα). These cofactors were either covalently linked to the ICC or co-expressed and co purified with the ICC. In addition, negative controls (naked antibodies) or positive controls (IL-15) were also tested to confirm the validity of the experiment.

As expected, neither naked antibodies nor uncleaved ICC were able to activate NK cells in the tested conditions. After cleavage, K03201-002 and K03201-073 were not able to activate NK cells, as no IFNγ was detected in the supernatant and there was no CD69 positive cells. After cleavage all other constructs induced higher activation than the rIL-15 introduced as positive control (**Fig. 5**). On the other hand, activation of NK cells was not observed when these ICCs are uncleaved.

It appears thus clearly from this experiment that IL-15 shows an attenuated activity when linked to any of the antibodies. After cleavage of the ICC by MMP-9, IL-15 is liberated in its active form and is again able to fulfil its biological activity

### EXAMPLE 4: Pharmacokinetic evaluation

### 1. Methods

The clearance of immunocytokine constructs was assessed in mice following single intravenous administration (bolus dosing at tail vein) of 0.53 nmol in female BALB/cByJ mice *i.e.* about 20-25nmol/kg. Animals were housed in accordance with regulations set out in Directive 2010/63/EU of the European Parliament and of the Council of 22 September 2010 and French decree No. 2013-118 of 01 February 2013 (Official Journal of the French Republic of 07 February 2013).

Blood samples were collected using K2 EDTA micro capillary glass tubes at the following sampling times:
T 0.5, 1, 3, 6, 24 and 48 hours after dosing (sampling to tail vein) for the first study **Erreur ! Source du renvoi introuvable.]** and T 0.05, 1, 5, 24, 48, 72, 168 hours after dosing (sampling to saphenous vein) for the second study **Erreur ! Source du renvoi introuvable.].**

Animals were monitored during all of the in life experimental phase.

Plasma were prepared, and 5-fold diluted in PBS, then stored at -80°C until analysis.

Concentrations (ng/mL) of total antibody (cleaved and uncleaved ICC) and of total ICC (antibody with at least one remaining IL-15) were determined in plasma samples by ligand binding assay using MesoScale Discovery technology. mAb anti-human IgG (CH2 domain) for total antibody assay and pAb anti-human IL-15 for total ICC assay were used as capture; pAb anti-human IgG (Fc specific) SulfoTAG labelled was used for detection in both assays.

The area under curve, *i.e.* AUC(0-last) (h*nmol/L), was calculated from each plasma concentration-time profile using the linear up-log down trapezoidal rule using Phoenix WinNonlin Certara (version 8.1.0).

To facilitate the comparison, AUC were corrected for the dose expressed in nmol/kg.

Sample compounds in study 1 evaluated IgG1 and IgG4 isotypes and covalently linked sushi or sushi+ domains. Sample compounds in study 2 evaluated IgG1 and IgG4 isotypes and co-expressed (non-covalent) sushi+ or complete IL-15Ra extracellular domain. Evaluated samples were generated with either a single or double copy of the protease sensitive linker between antibody Fc and IL-15.

### 2. Results

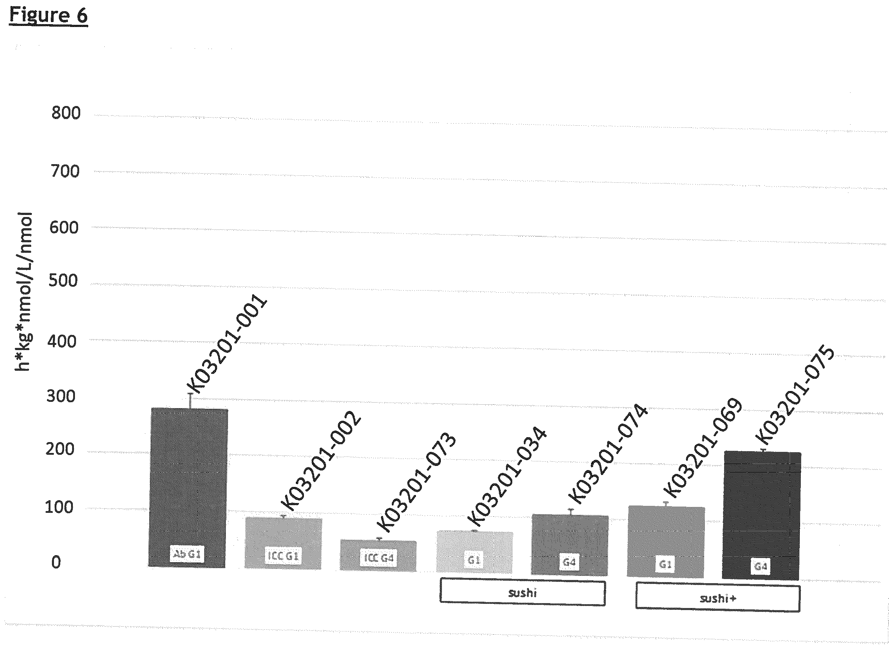

In the first study, all constructs were compared regarding their AUC(0-last)/dose for total antibody which translates to the exposure of animals. Of the 6 ICCs evaluated, the best exposures were obtained for ICC-IL-15-sushi+ covalent constructs (see **Fig. 6**). Among all constructs, the IgG4 isotype sushi+ construct (K03201-075) displayed the profile closest to that of the parental antibody with an IgG1 isotype, with a plasma exposure equivalent to 74.5% of the parent antibody.

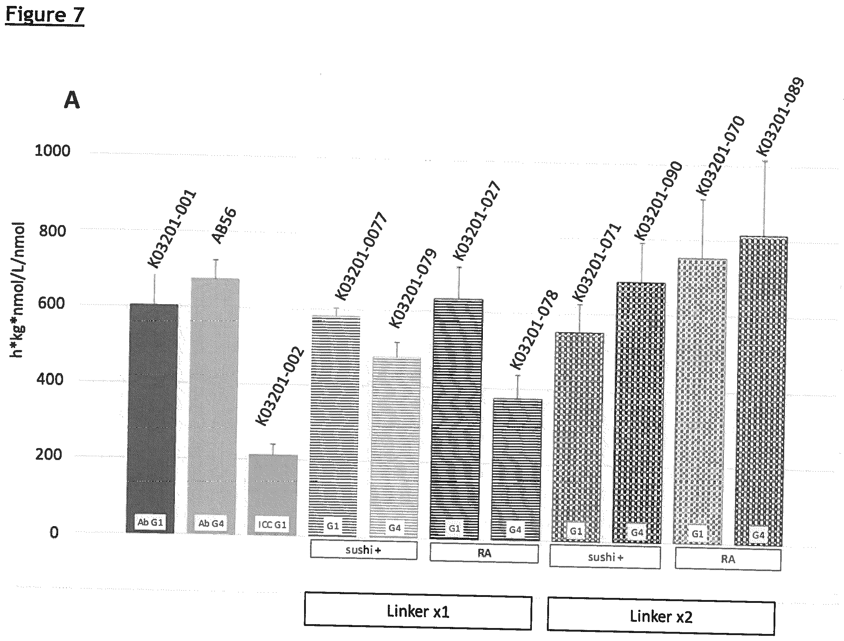

In the second study, the total antibody plasma exposure of animals for sushi+ and sIL-15Rα constructs were better than for the basic construct (K03201-002) for all construct variants irrespective of linker repeats, isotype or co-expressed factor (see **Fig. 7A**). The sushi+ simple linker constructs were better tolerated than those with double linkers.

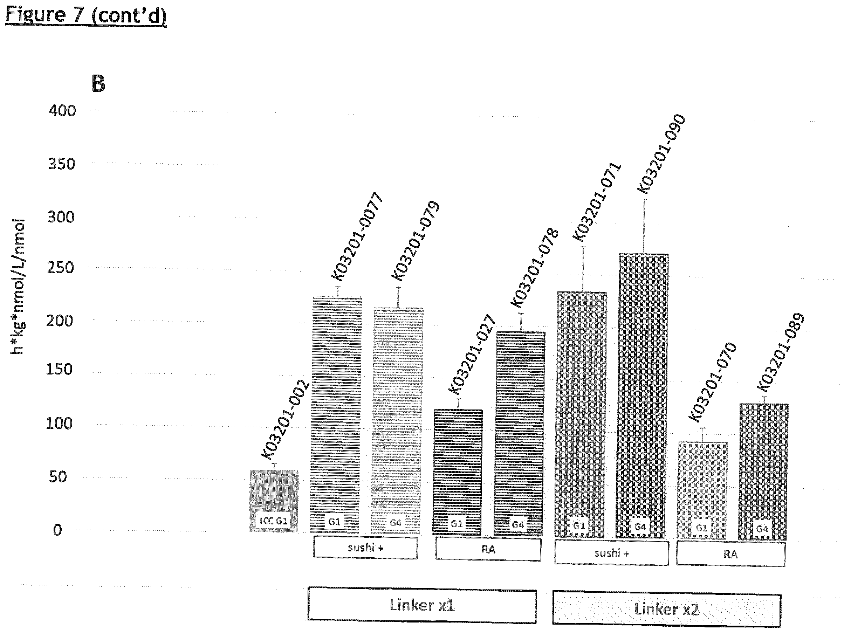

In addition, the exposure of animals to total ICC for sushi+ and IL15Ra constructs was determined. The plasma exposure of animals to total ICC was also higher for sushi+ than for s1L-15Ra constructs (see **Fig. 7B**).

### EXAMPLE 5: In vivo effects of the ICCs on a renal carcinoma cell line.

### 1. In vivo evaluation of K03201-079 effect on NK cells in the RENCA model

### 1.1. Material and Methods:

Ten to twelve-week-old female Balb/c byJ mice (Charles River) were engrafted with 0,5.10⁶ RENCA cells, subcutaneously using a needle for each mouse. Mice were maintained in individual cages (10 mice/cage) at constant temperature and humidity in accordance with regulations set out in Directive 2010/63/EU of the European Parliament and of the Council of 22 September 2010 and French decree No. 2013-118 of 01 February 2013 (Official Journal of the French Republic of 07 February 2013).

Eleven days after cell engraftment, when tumours reached around 100mm³, mice were randomised and allocated in treatment groups (8 mice per group). Mice received IV injection of either vehicle or K03201 -079 dosed at 20µg and 50µg, at D0 and D3 post-randomisation.

Twenty-four hours post last injection, mice were euthanised and tumours were sampled for flow cytometry analysis of the NK cell population.

Briefly, tumours were dissociated using the Tumor Dissociation Kit Mouse (Miltenyi) and the Gentle MACS Octo Dissociator (protocol 37C_m_TDK_2) (Miltenyi). Tumour cell suspensions were filtered on 70µm Smart Stainers and count with ViCell. Tumour cell suspensions were then centrifuged and cell concentration adjusted to 20.10⁶ cells/mL in cold FACS buffer.

2.10⁶ of tumour cell suspension were plated on 96 well plate with V bottom, Fc Receptor on cells were blocked using FcR Blocking Reagent Mouse, and then 10µg/mL of alone or mixed fluorescent antibodies were added in each well., and samples were analysed by flow cytometry.

### 1.2. Results

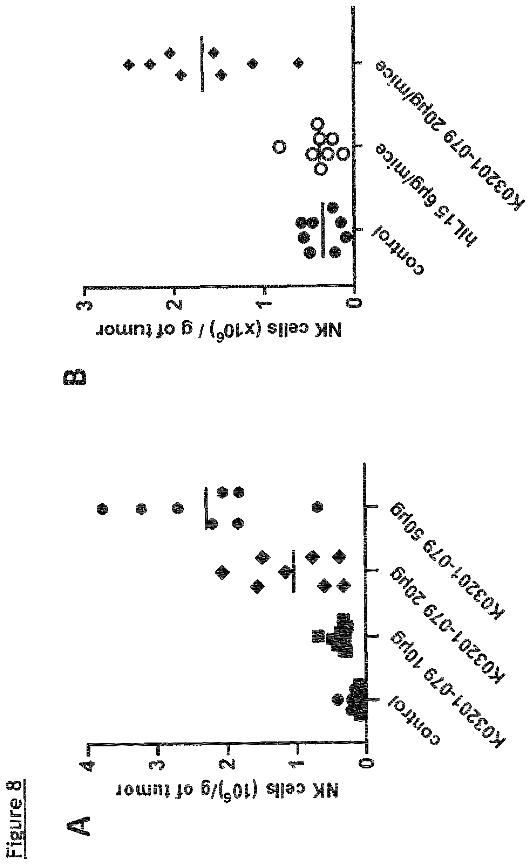

K03201-079 comprises the IL-15 cytokine bound to the H16L16 antibody (ganitumab) through the L6 linker and co-expressed with sushi +. A dose-dependent increase of the number of NK cells within the tumours is induced following administration of K03201-079 (see **Fig. 8A**). IL-15 is thus targeted correctly to the tumour where it induces the expected immune response.

### 2. In vivo comparison of K03201-079 and rIL-15 effect on NK cells in the RENCA model

### 2.1. Material and Methods:

Ten to twelve-week-old female Balb/c byJ mice (Charles River) were engrafted with 0.5.10⁶ RENCA cells, subcutaneously using a needle for each mouse. Mice were maintained in individual cages (10 mice/cage) at constant temperature and humidity following European Guidelines recommendations.

Eleven days after cell engraftment, when tumours reached around 100mm³, mice were randomised and allocated in groups of treatment (8 mice per group). Mice received IV injection of either vehicle, K03201-079 dosed at 20µg or subcutaneous injection of rIL-15IL-15 dosed at 6µg (equivalent dose to 20µg of K033201-079) at D0 post-randomisation.

Ninety-six hours post last injection, mice were euthanised and tumours were sampled for flow cytometry analysis of the NK cell population.

Briefly, tumours were dissociated using the Tumor Dissociation Kit Mouse (Miltenyi) and the Gentle MACS Octo Dissociator (protocol 37C_m_TDK_2) (Miltenyi). Tumour cell suspensions were filtered on 70µm Smart Strainers and enumerated by ViCell. Tumour cell suspensions were then centrifuged and cell concentration adjusted to 20.10⁶ cells/mL in cold FACS buffer.

Then 2.10⁶ of tumour cell suspension was plated in 96 well plates with V bottoms. Fc Receptor on cells were blocked using FcR Blocking Reagent Mouse, and then 10µg/mL of simple or mixed fluorescent antibodies were added in each well, and the samples were analysed by flow cytometry

### 2.2. Results:

When rIL-15 was injected subcutaneously at 6µg, no increase of the NK cell number was observed within the tumours. However, the equivalent dose of IL-15 administrated via the K03201-079 is able to induce a 4.8 increase fold of the NK cell number in the tumours (see **Fig. 8B**).

## Claims

1. A protein complex comprising:
(i) a fusion protein comprising an antibody, or antigen-binding fragment thereof, a cleavable peptide linker, and IL-15 or a functional fragment thereof; and
(ii) a cofactor, wherein said cofactor is IL-15Ra or an IL-15-binding fragment thereof.

2. The protein complex of **claim 1,** wherein the IL-15-binding fragment is selected in the group consisting of: soluble IL-15Ra of SEQ ID NO. 53, IL-15Ra sushi of SEQ ID NO. 51, and IL-15Ra sushi+ of SEQ ID NO. 52.

3. The protein complex of **claim 1 or 2,** wherein the cleavable peptide linker comprises a protease cleavage site.

4. The protein complex of any one of **claims 1 to 3,** wherein the protease cleavage site is cleaved by a matrix metalloproteinase or by uPA.

5. The protein complex of **claim 4,** wherein the matrix metalloproteinase is MMP-2, MMP-9.

6. The protein complex of any one of **claims 1 to 5,** wherein the cleavable peptide linker has a sequence selected from the group consisting of: GPLGIAGQ, GPLGLWAQ, GPLGMLSQ, PLGLAG, PVGLIG, SGRS, SGRSA, and PSSSRRRVN.

7. The protein complex of any one of **claims 1 to 6,** wherein the antibody or antigen-binding fragment thereof is selected from the group consisting of polyclonal antibodies, monoclonal antibodies, chimeric antibodies, humanised antibodies, scFv, single domain antibodies (e.g., shark and camelid antibodies), maxibodies, minibodies, intrabodies, diabodies, triabodies, tetrabodies, v-NAR and bis-scFv.

8. The protein complex of any one of **claims 1 to 7,** wherein:
(i) IL-15 or the functional fragment thereof is fused to the cleavable peptide linker, and
(ii) the cleavable peptide linker is used N-terminally or C-terminally to the antibody or antigen-binding fragment thereof.

9. The protein complex of any one of **claims 1 to 8,** wherein:
(i) IL-15 or the functional fragment thereof is fused to the cleavable peptide linker, and
(ii) the cleavable peptide linker is fused N-terminally or C-terminally to the heavy chain of the antibody or antigen-binding fragment thereof.

10. The protein complex of any one of **claims 1 to 9,** wherein the cofactor is bound covalently to the fusion protein.

11. The protein complex of **claim 10,** wherein the cofactor is linked to IL-15.

12. The protein complex of any one of **claims 1 to 9,** wherein the cofactor is bound non-covalently to the fusion protein.

13. The protein complex of any one of **claims 1 to 12** for use in stimulating an immune response in a subject.

14. The protein complex of **claim 13,** wherein the immune response involves expanding one or several lymphocyte populations, such as NK cells, NK-T cells, CD8⁺ T cells.

15. The protein complex of any one of **claims 1 to 12** for use in therapy.

16. The protein complex of any of **claims 1 to 12** for use in the treatment of cancer in a subject.

17. The fusion protein for use according to **claim 16,** wherein said use comprises activation of immune cell, preferably T-cells or monocytes, of said subject.

18. A pharmaceutical composition comprising at least one protein complex according to any one of **claims 1 to 12** and optionally a pharmaceutically acceptable excipient.
